# EUROPEAN PATENT APPLICATION

(11) **EP 2 325 638 A1**
(43) Date of publication of application: **25.05.2011**
(21) Application number: 10162359.3
(22) Date of filing: 07.05.2010
(51) Int. Cl.: G01N 33/50, G01N 33/68

(54) **Method for high recovery of serum low-abundance, low molecular-weight proteins and their analysis by matrix assisted laser desorption ionization mass spectrometry**

(30) Priority: 07.05.2009 EP 09159634
(71) Applicant: IRCCS San Raffaele Pisana- San Raffaele, 00166 Roma (IT)
(72) Inventor: Guadagni, Fiorella, 00163 Roma (IT); Di Girolamo, Francesco, 00163 Roma (IT)
(74) Representative: Capasso, Olga

(57) **Abstract**

It is described a method for the detection of low molecular weight protein profile from a serum sample comprising the step of:

a) precipitating high abundant serum proteins and collecting supernatant;
b) purifying said collected supernatant with magnetic beads with reverse phase functional surfaces;
c) crystallizing on MALDI mass spectrometer plate the products of step b), using a MALDI crystallization technique, in order to obtain four crystallized homogeneous spots;
d) analyzing LMW protein profiles of all crystallized spots by means of Matrix-Assisted Laser Desorption Ionization/Time-of-Flight mass spectrometer (MALDI/ToF ).

## Description

### BACKGROUND

Early detection of many diseases can greatly improve living conditions of a patient. Unfortunately, only few specific biomarkers for diagnosis of early-stage disease [1], prognosis or non-responsiveness to therapy are available today [2].

Most of these molecules, used as biomarkers, suffer of poor sensitivity, specificity and predictive value, especially when applied to rare diseases and to neurodegenerative, cardiovascular and oncologic diseases [3].

In addition, the proteins that can be utilized as potential biomarkers (1% of total serum proteins) are represented by low-abundance Low Molecular Weight proteins (LMW) (about 10,000 different proteins linked or not associated with high abundant carrier proteins with a mass range <15 kDa) [8b, 9b]. The low-abundance LMW serum proteome promises to contain a rich source of previously undiscovered biomarkers [8b, 10b] , as biological processes give rise to cascades of enzymatically generated and proteolytically clipped biomarker fragments. These LMW molecules have been until very recently not completely characterized, since many problems exist for their recovery by conventional techniques [11b, 12b].

In order to obtain highly sensitive and specific biomarkers, it is essential to:
1) work on biological samples that should be as representative as possible of patient's physiopathological condition;
2) employ analytical methodologies capable to identify and to rigorously validate molecular indicators ― biomarkers .

As regards point 1, both, plasma and serum, have been used for decades as a source of proteins and other agents indicating changes of the physiological state of an individual (molecular biomarkers) [4]. The currently known components of the serum proteome are only the tip of the iceberg: they potentially contain elements of all proteins produced in the body [5]. Human serum proteome consists of a large variety of proteins with different chemical and physical properties present in sera at different concentrations [6].

The complexity of serum makes it a very informative source for developing a proteomic pattern. Biomarkers are often low abundance LMW secreted into the bloodstream as a result of the disease process. [8].

Serum contains about 10,000 different proteins, the majority of which are LMW protein species [7] and a total of 60-80 mg/mL of proteins, with a concentration range spanning at least nine orders of magnitude. LMW proteins are present in the serum in two forms: free and linked to carrier proteins, such as albumin and immunoglobulins (IgG), 65-97% of all serum proteins.

As regards point 2, it is essential to use a highly specific and sensible analytical methodology to collect and identify serum LMW proteins as well as to obtain highly reproducible serum profiles to highlight the tiny differences between patients (independently from pathology) and control subjects and among specific diseases.

This idea has not yet materialized due to the many problems in standardizing the protocol at each analytical stage (pre-analytical, analytical and post-analytical) and to the extreme lability of the serum proteome [7a]. In addition, commercial kits developed so far by simply making a targeted depletion of the most abundant proteins (albumin and IgG) are not specific for the recovery of all LMW in serum.

Many potentially interesting biomarkers are linked to albumin and other highly abundant proteins in blood serum. For this reason Perkin Elmer developed PRO XPRESSION KIT to capture this carrier protein-bound LMW (the kit is now out of production). Many other potentially interesting LMW biomarkers are free (no-linked) and are extracted by means of profiling Kits (Invitrogen, Dynal Peptide Profiler and Bruker Daltonics Profiling kit).

Serum LMW protein profiling with high-throughput reverse-phase magnetic bead sample preparation followed by time of flight (TOF) mass spectrometry analysis is an emerging approach for biomarker discovery and for disease diagnostics and monitoring [13b-16]. It appears to be a highly sensitive analytical methodology to recover serum LMW proteins. It allows reproducible serum protein profiles that can distinguish differences between patients and control subjects.

Two commercial magnetic separation kits, Dynal Peptide Profiler RPC 18 (Invitrogen Corp. Carlsbad, CA USA) [15] and Profiling Kit MB-HIC-8 (Bruker Daltonics, Bremen, Germany) [13b, 16, 17] were developed to capture serum peptides and LMW proteins for MALDI mass spectrometry analysis. These commercial magnetic separation kits were presented as new approach to biomarker discovery and defined as robust, precise and rapid techniques for the investigation of complex blood samples [15, 16, 17, 18].

In light of these considerations and in order to obtain a better recovery and discrimination of Low Abundance, Low Molecular weight proteins in sera, a Novel Sample Pretreatment Procedure (NSPP) capable of improving the performance of the two commercial kits has been developed in the present invention. Finally, to evaluate whether a more sensitive procedure could be of potential use in the biomarker discovery field, two sets of sera (normal donors *versus* colorectal cancer patients) have been analyzed.

### DESCRIPTION OF THE INVENTION

In the present study, a new pre-treatment approach for reverse phase magnetic bead extraction of serum Low-Abundance, LMW protein, named "Novel Sample Pretreatment Procedure" (NSPP) also called New Analytical Approach (NAA) has been developed. This represents a novel, automated technology platform allowing a better LMW proteins recovery of three commercial magnetic separation kits (Pro Xpression Kit, Dynal Peptide Profiler RPC-18 and Profiling Kit MB-HIC-8) considered as the most precise and rapid techniques today available. Nevertheless, the application of the present procedure significantly improved LMW protein recovery (p<0.01). Finally, to evaluate whether a more sensitive procedure could be of potential use in the biomarker discovery field, two sets of sera (normal donors *versus* colorectal cancer patients) have been analyzed. The results demonstrated the recovery of a significant higher number of discriminatory peptides using NSPP than the kits alone (p<0.01), thus corroborating a potential value of this new approach for biomarker discovery.

The method of the present invention allow to obtain a highly specific selective and reproducible protein profiling spectra of both LMW proteins, free and linked to abundance carrier proteins in the serum. Authors considered the many requirements due to the extreme lability of the serum proteome and standardized the experimental conditions of each analytical step, from blood sampling, to serum preparation and mass spectrometry analysis [10-12].

The authors of the invention set up a new analytical approach, allowing to obtain:
1) A high recovery of LMW proteins from serum, by means of specific solvents, as acetonitrile and trifluoroacetic acid (TFA) and homogeneous crystallization of sera proteins on a MALDI (Matrix Assisted Laser Desorption Ionization ) plate;
2) A high specific and reproducible serum LMW protein profiling, preferably by means of MALDI-TOF mass spectrometry technique.

The method of the invention was compared with the method worked by the PRO XPRESSION KIT (PerkinElmer), Dynal peptide profiler (Invitrogen) and Profiling Kit (Bruker Daltonics) showing best reproducible results and best recovery of LMWs. MALDI/MS protein profiles as obtained by the two methods were divided in two classes and compared using the ClinProTools Software (Bruker Daltonics) for the statistic analysis of MALDI protein profiling [13]. Such analysis revealed that the method of the instant invention is able to recover a higher number of LMW proteins with respects to the other three kits.

Therefore, the specific object of the invention is a method for the detection of LMW protein profile from a serum sample comprising the step of:
a) precipitating high abundant serum proteins and collecting supernatant;
b) purifying said collected supernatant with magnetic beads with reverse phase functional surfaces;
c) crystallizing on MALDI mass spectrometer plate the products of step b), using a MALDI crystallization technique, in order to obtain four crystallized homogeneous spots;
d) analyzing LMW protein profiles of all crystallized spots by means of Matrix-Assisted Laser Desorption Ionization/Time-of-Flight mass spectrometer (MALDI/ToF ).

In a preferable embodiment the precipitation step of step a) is performed by means of acetonitrile/TFA precipitation.

In a preferable embodiment the purification step of step b) is performed by means of magnetic beads with C18 or C8 reverse phase functional surfaces, as i.e. RPC 18, Invitrogen or HIC-8, Bruker Daltonics.

It is also a specific object of the invention a kit to work the method as above disclosed comprising:
- Acetonitrile;
- Trifluoroacetic acid (TFA) ;
- Deionized water;
- α-cyano-4-hydroxycinnamic acid (CHCA) ;
- Magnetic beads with C18 or C8 reverse phase functional surfaces.

The invention will be now described by means of non limiting examples referring to the following figures.

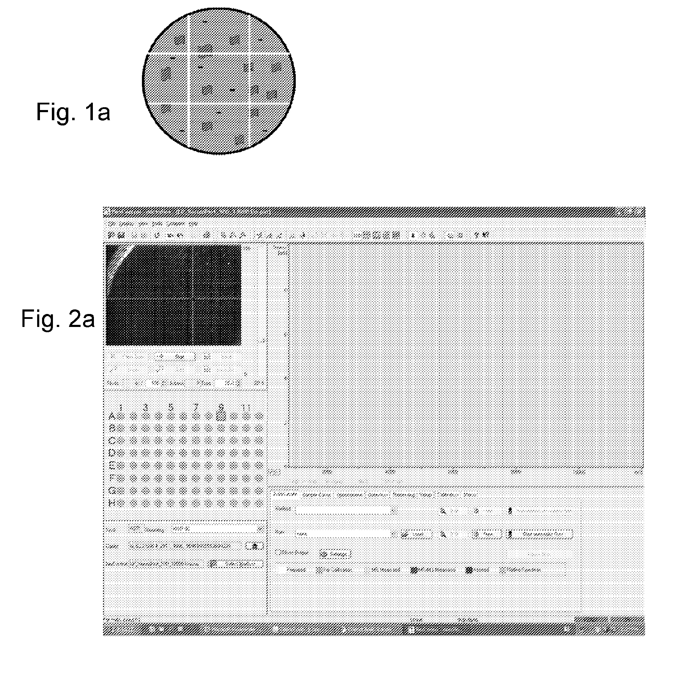

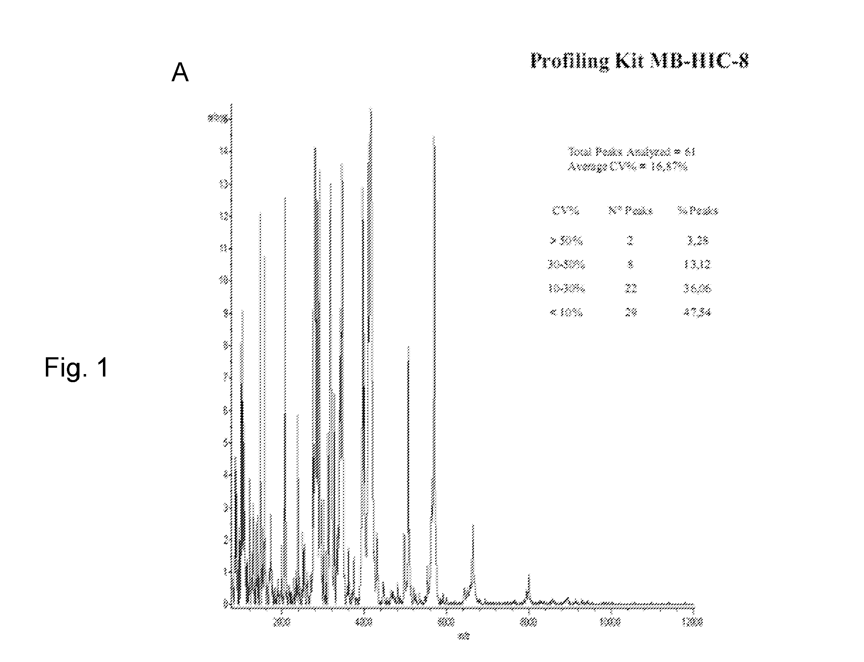

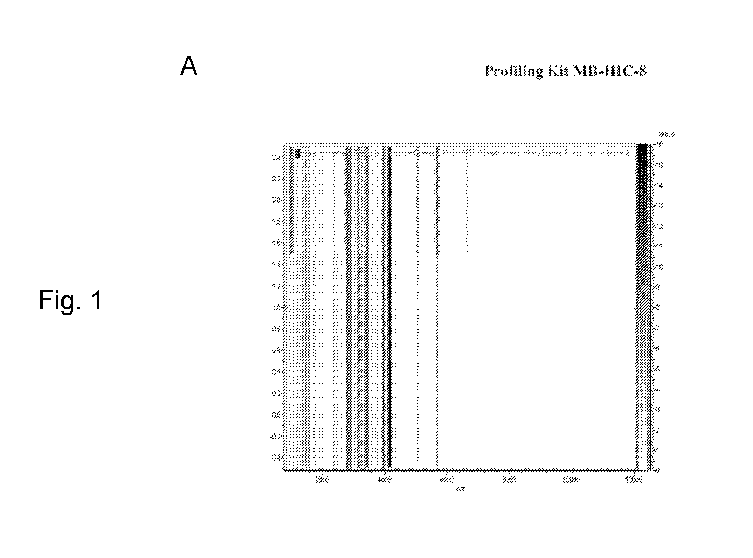

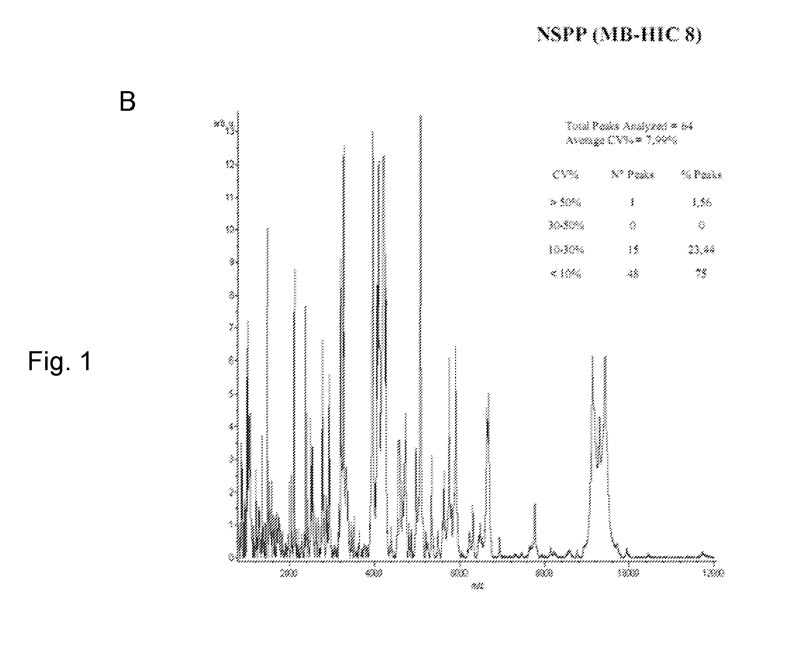

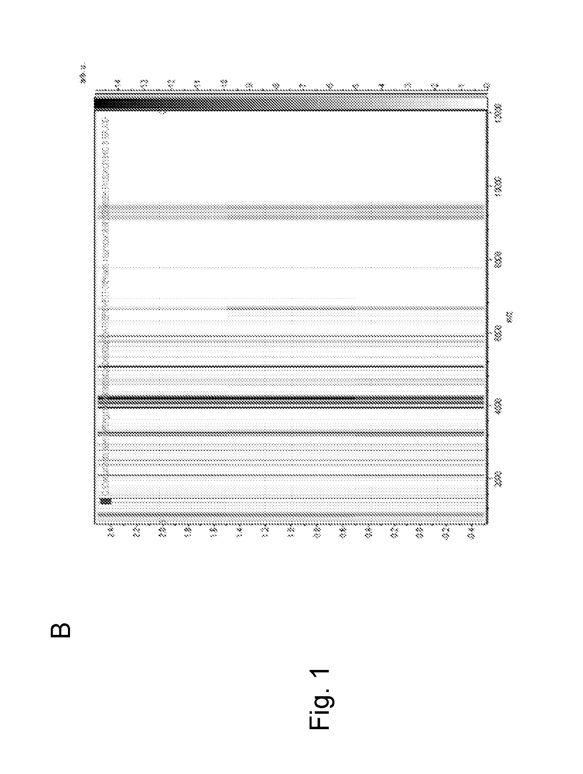

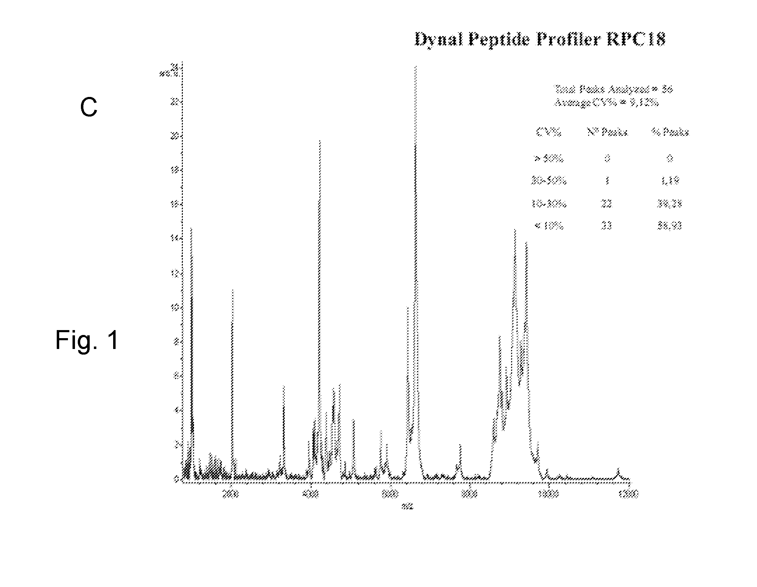

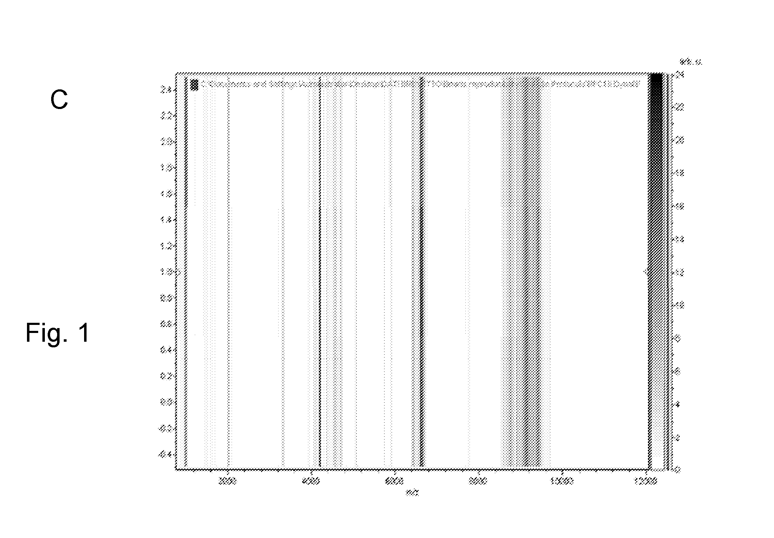

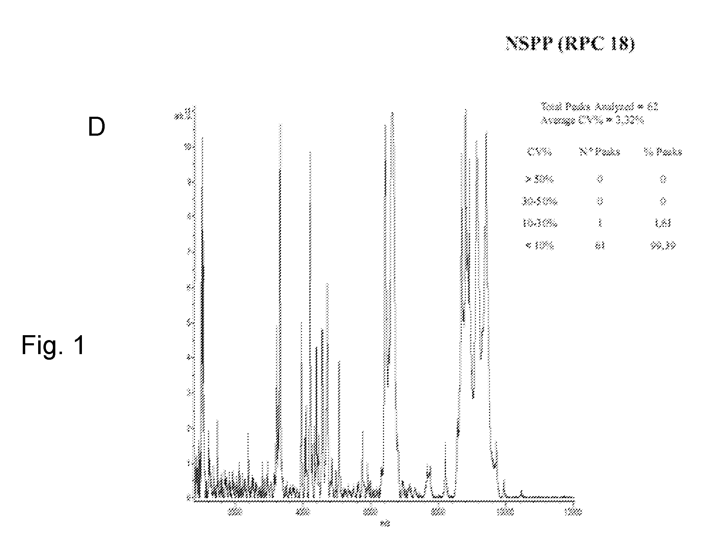

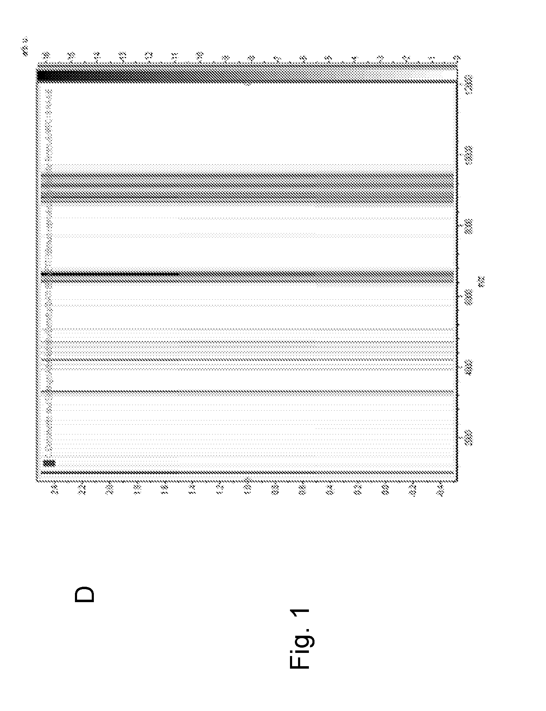

**Figure 1a**. MALDI target's spots have been divided into nine equal areas and were 2 acquisitions of 100 shots each on good crystals of each area were performed.

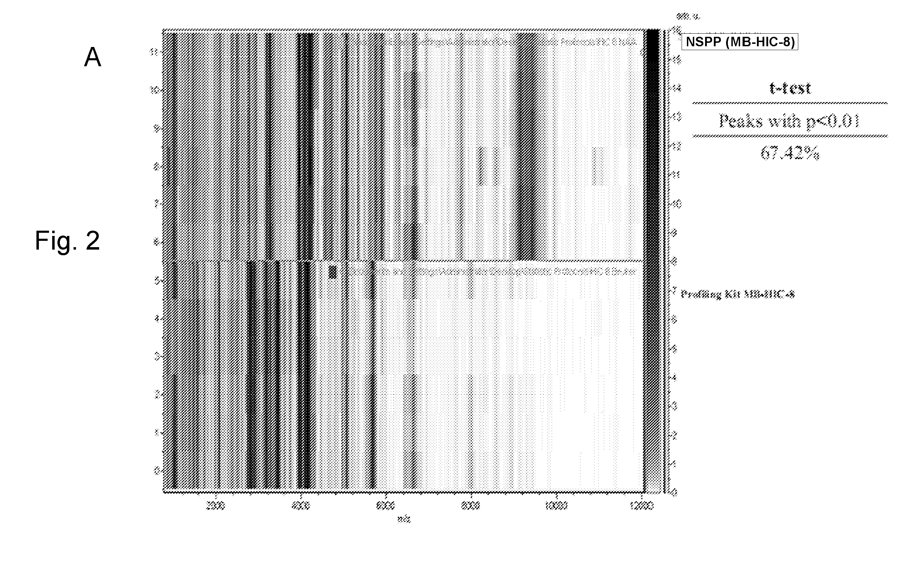

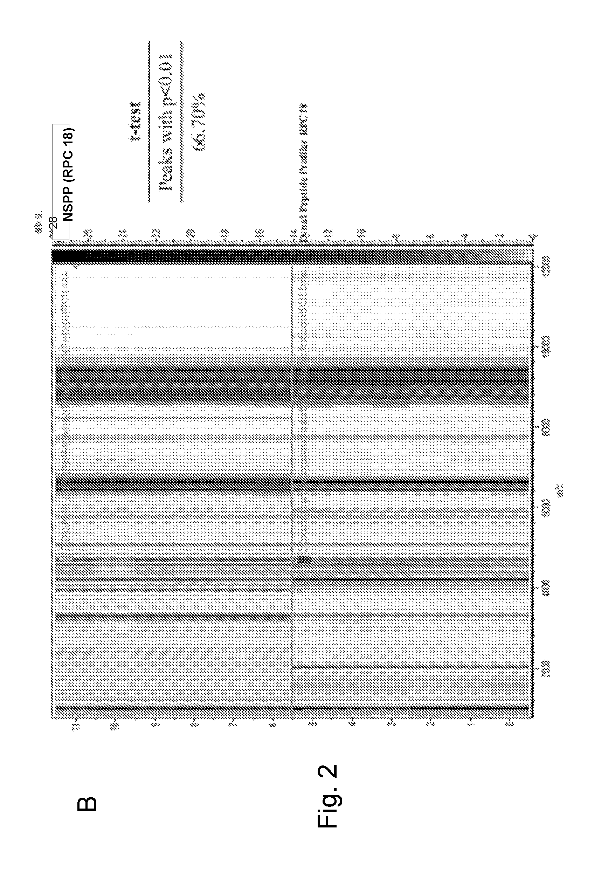

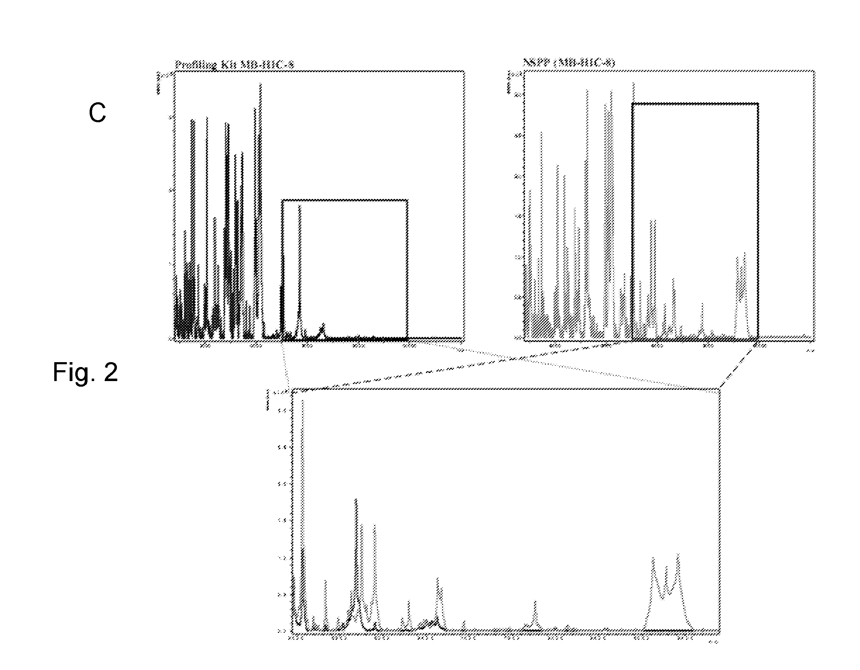

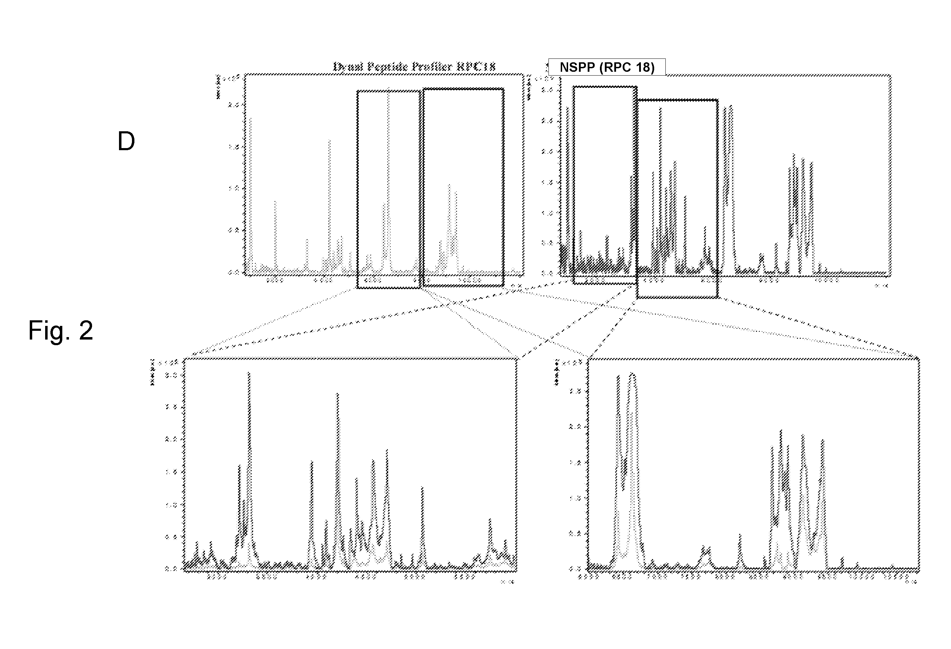

**Figure 2a**. Homogenous crystallization of serum sample over the spot MALDI (on the left of the picture): it allows to acquire very clean MS spectra and protein profiles easily comparable among them.

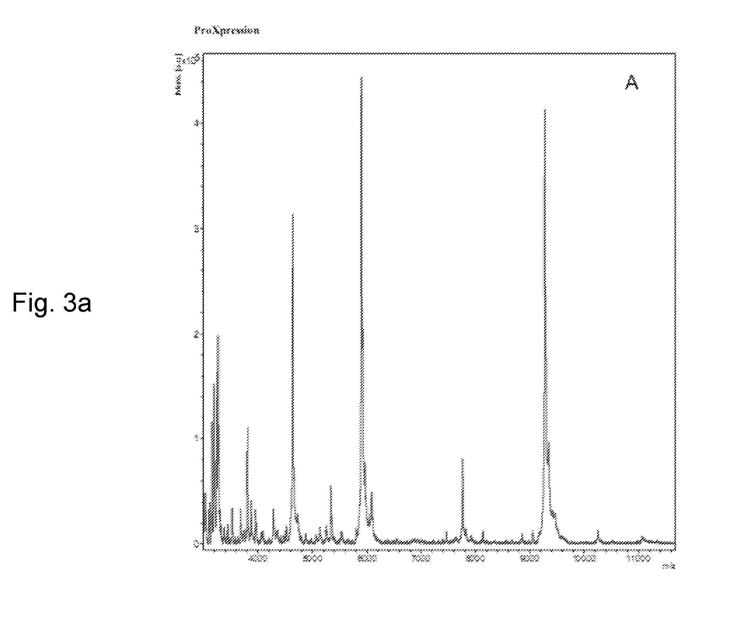

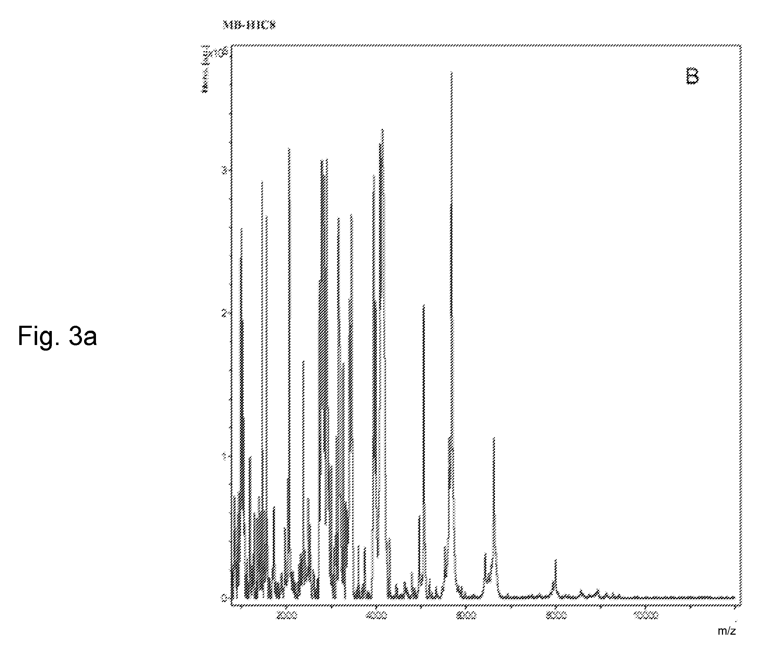

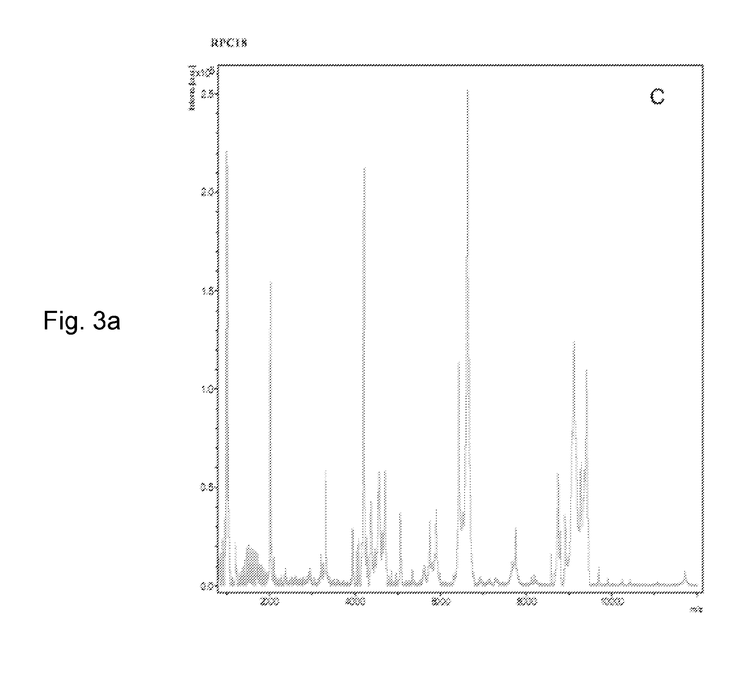

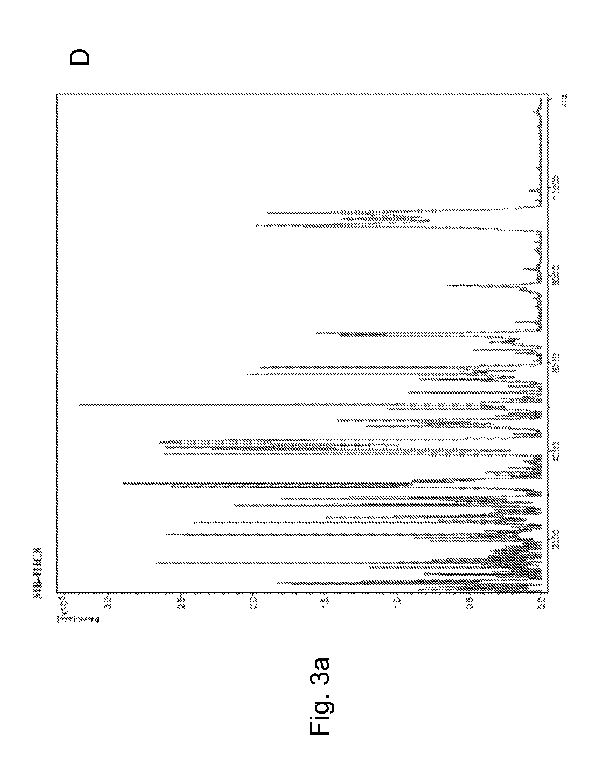

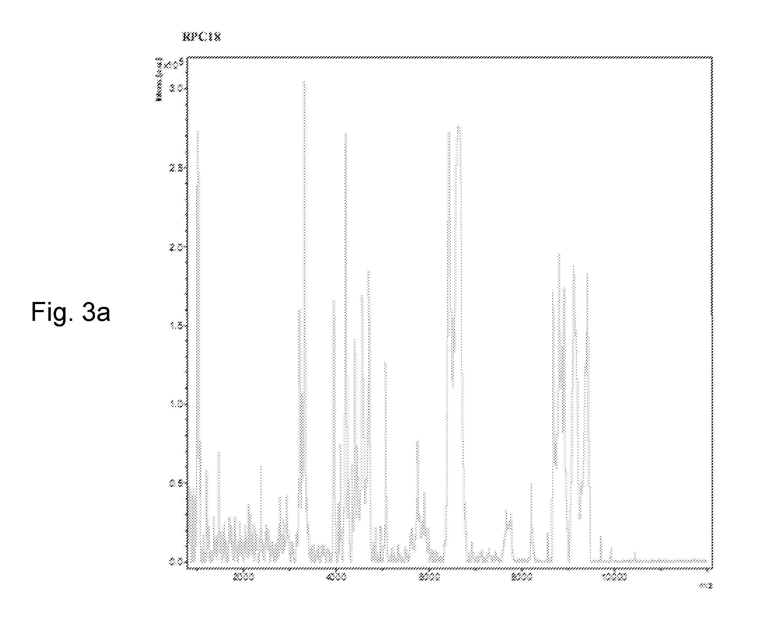

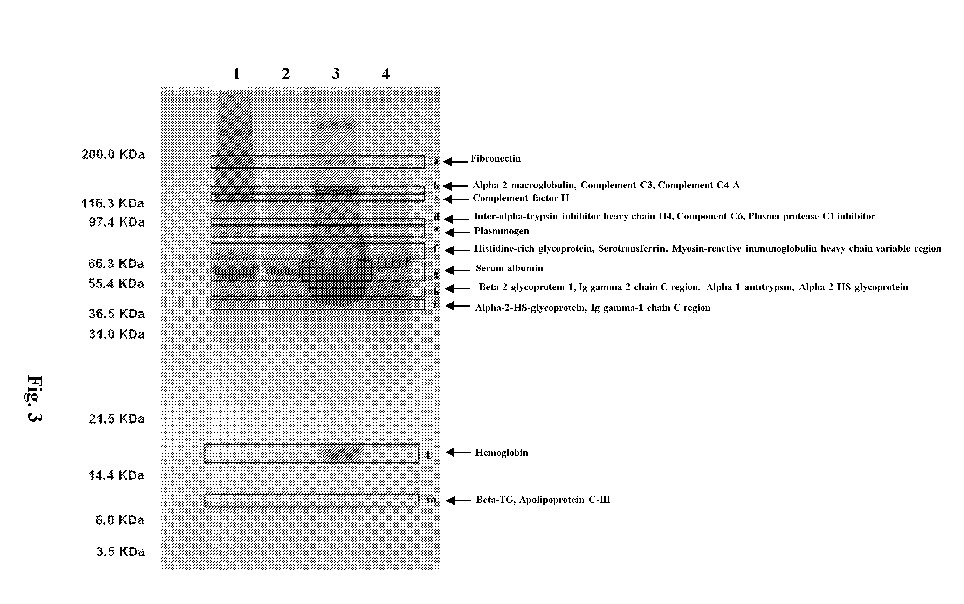

**Figure 3a**. Low-Molecular-Weight Serum profiling from: A) ProXpression Kit Perkin Elmer): low number of LMW recovery (carrier protein-bound LMW) B) Profiling Kit MB-HIC8 (Bruker Daltonics): high number of LMW recovery (no-linked to albumin and other highly abundant proteins) C) Dynal Peptide Profiler RPC18 (Invitrogen): high number of LMW recovery (no-linked to albumin and other highly abundant proteins) D) NAA (reverse phase magnetic beads MB-HIC8 and RPC18 purification): best number of LMW recovery.

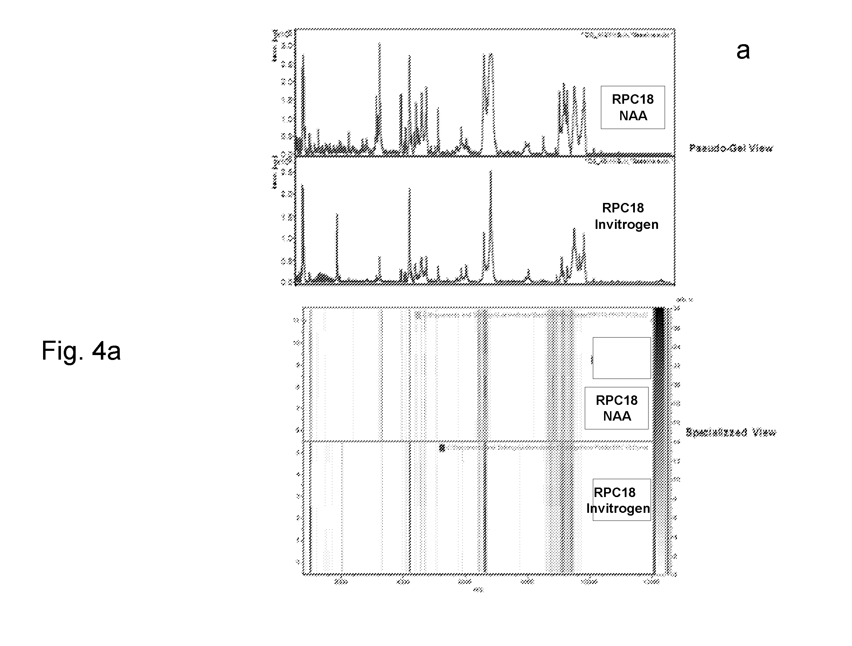

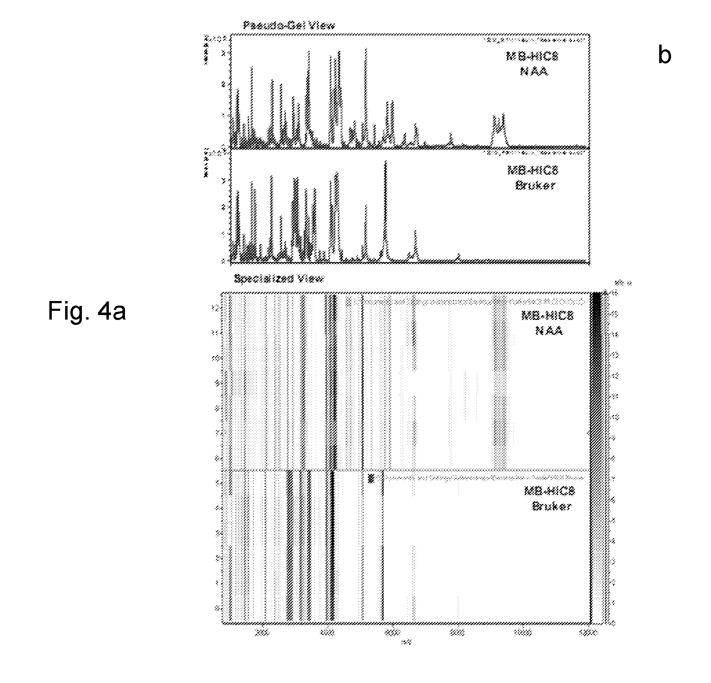

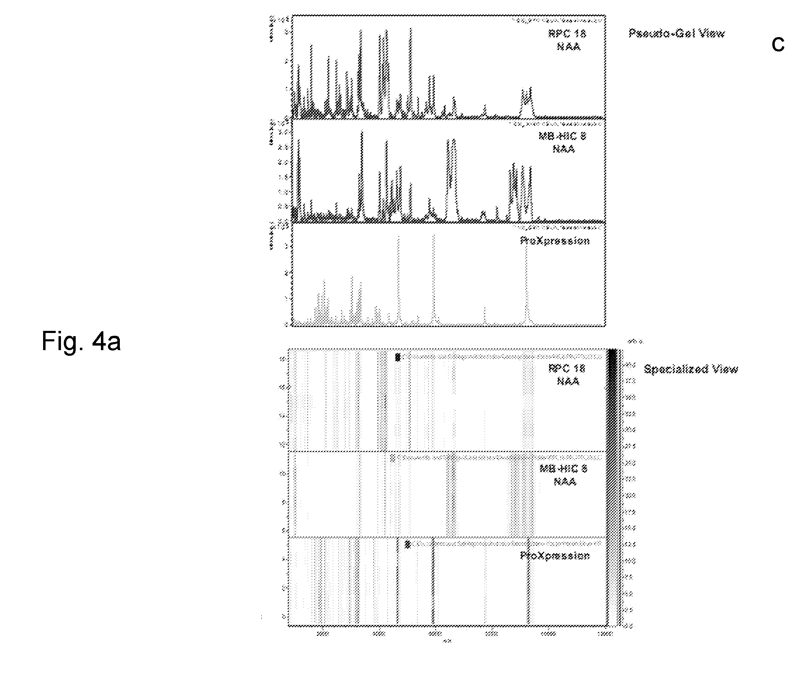

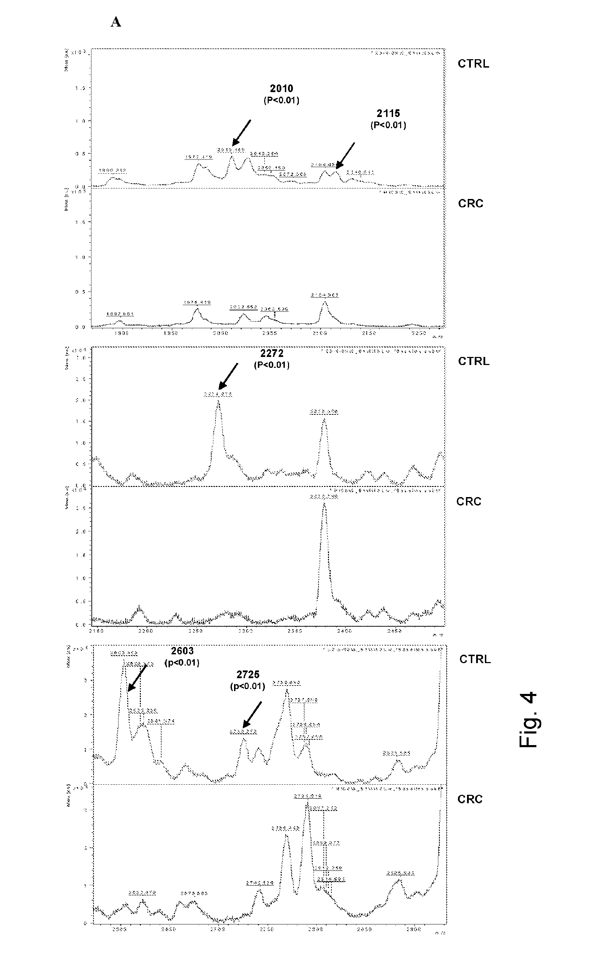

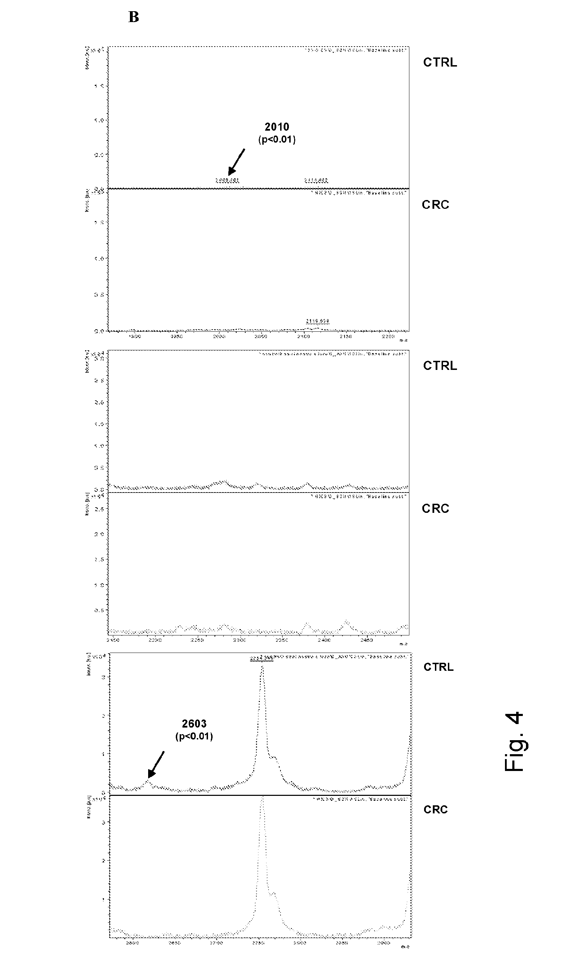

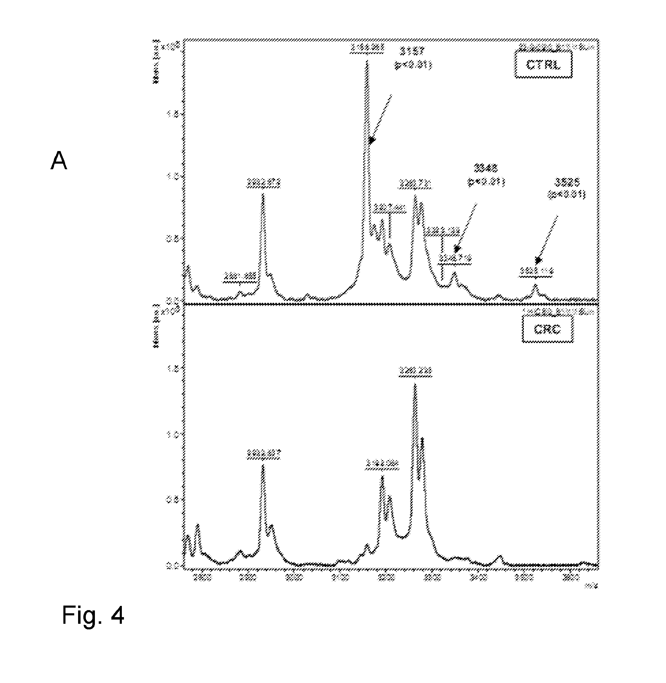

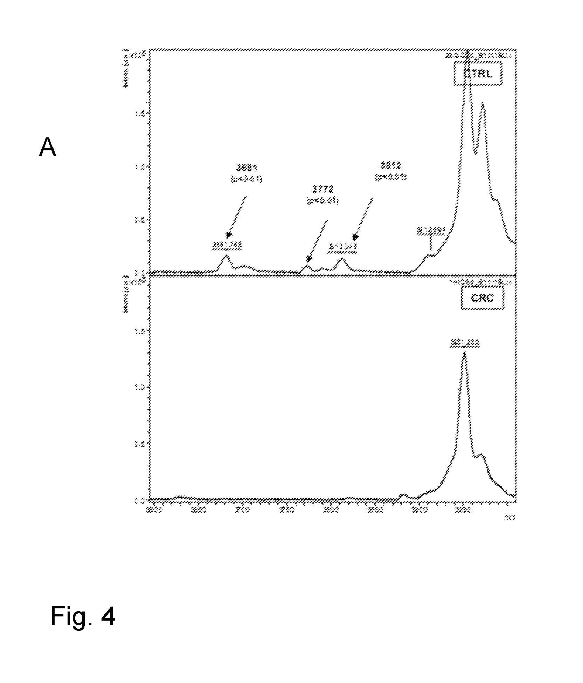

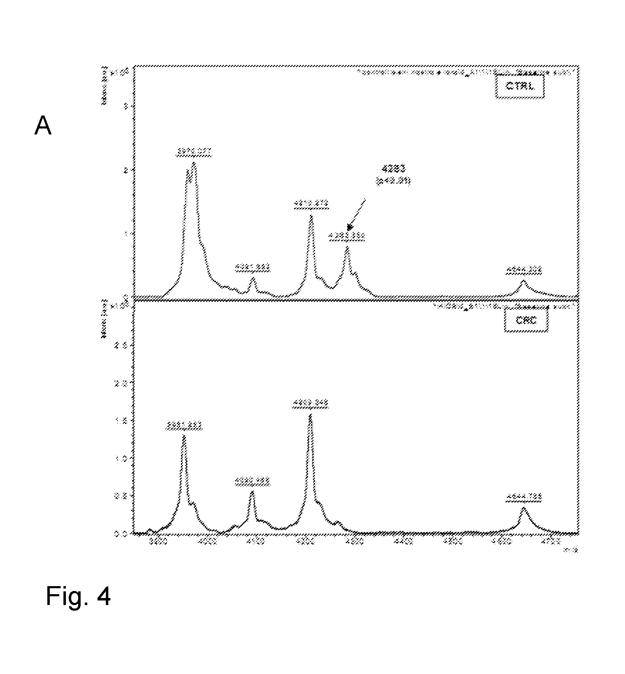

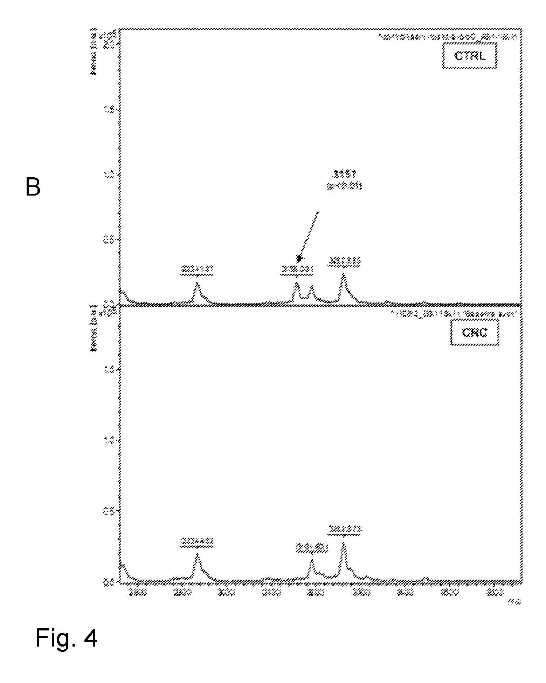

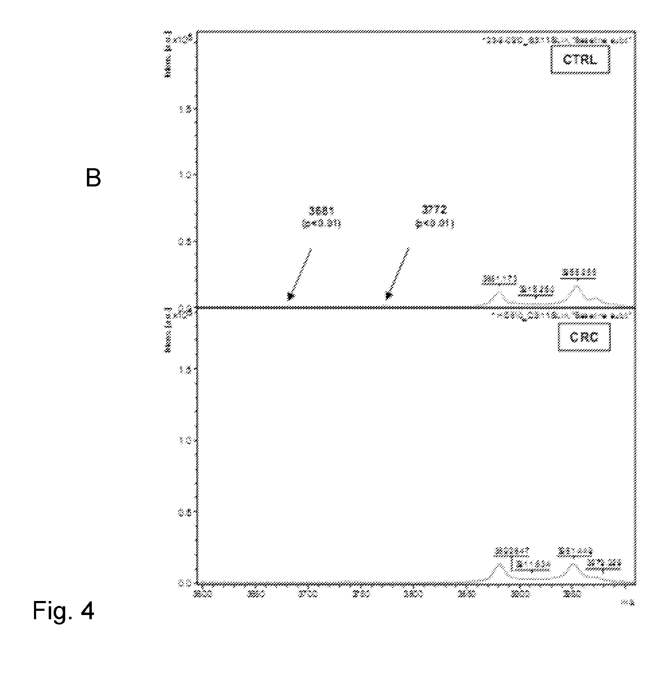

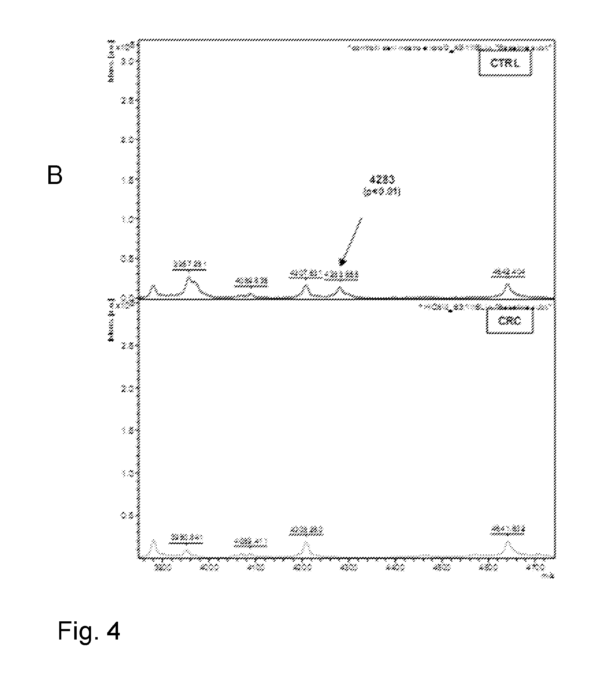

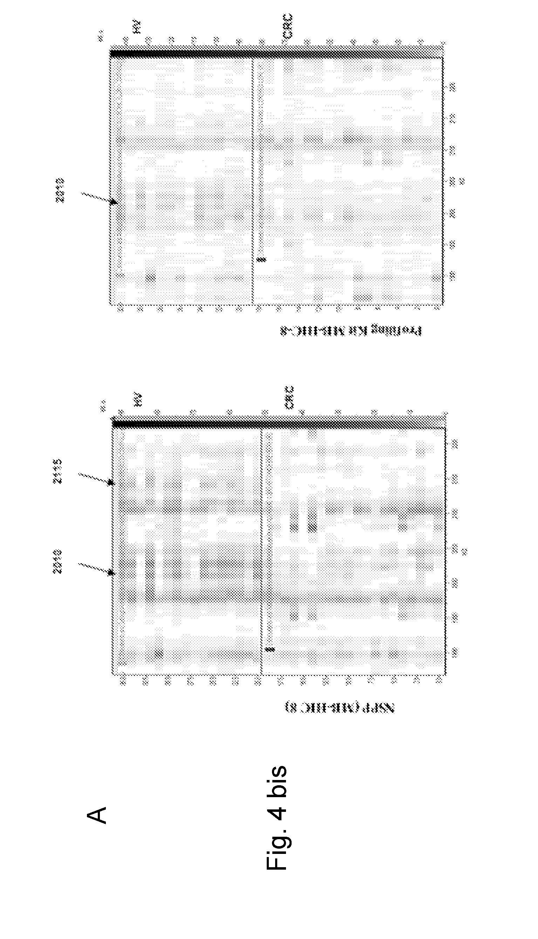

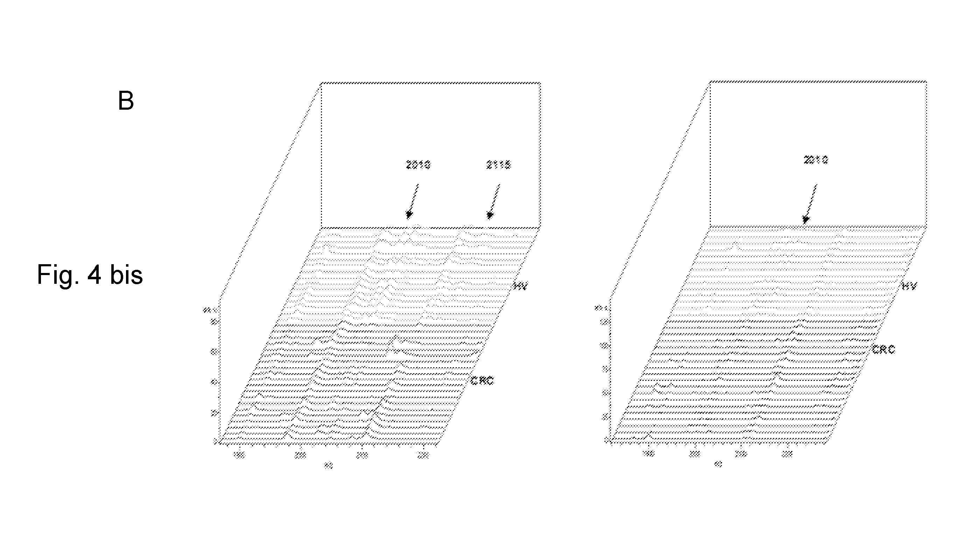

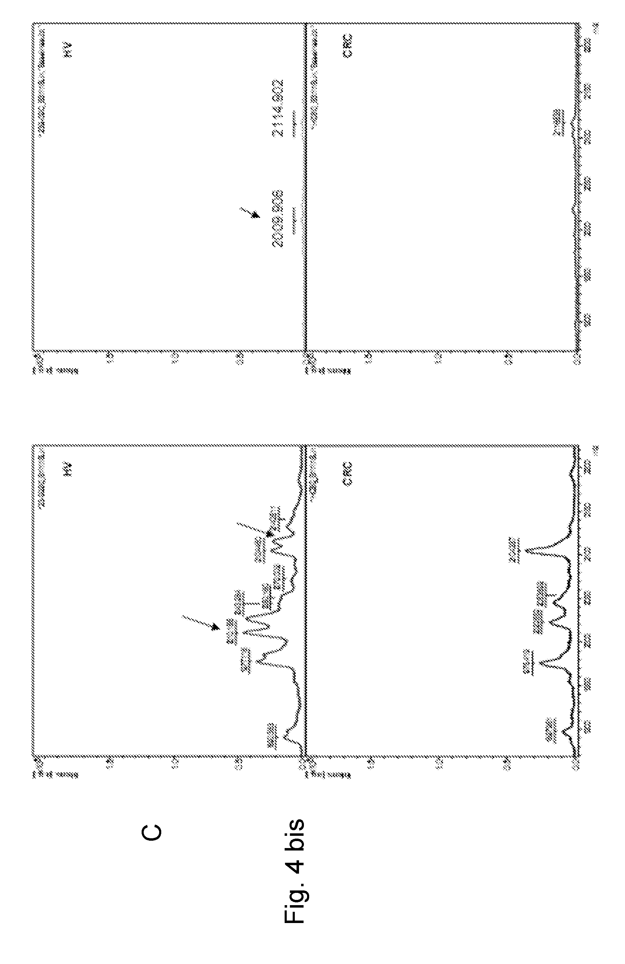

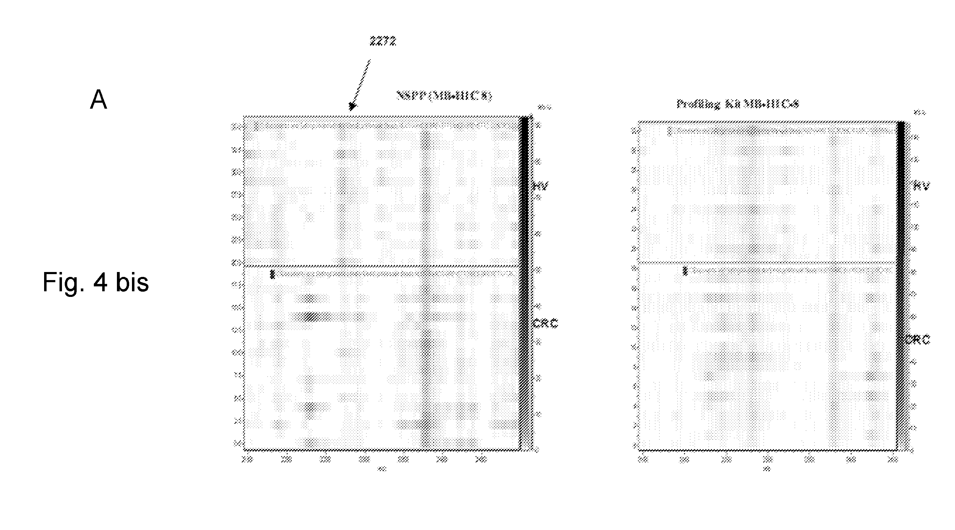

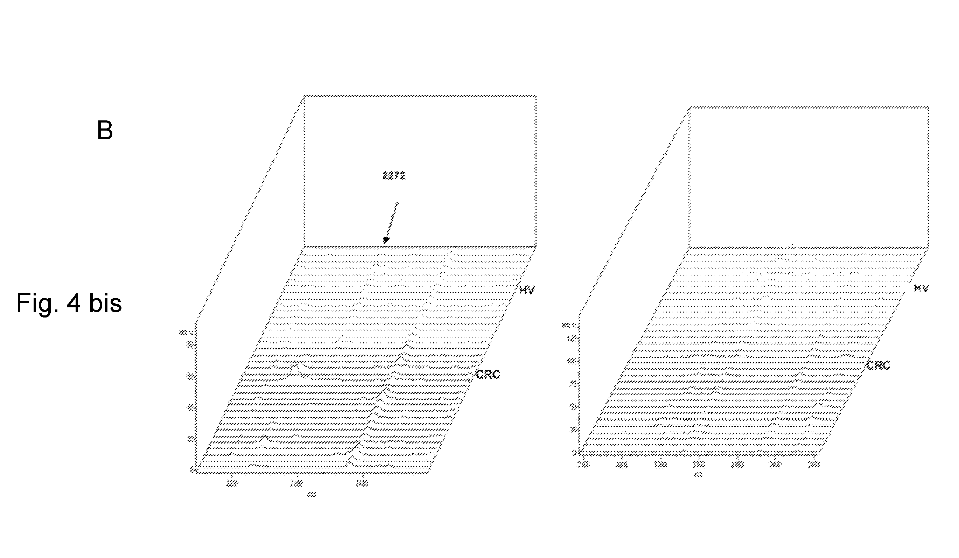

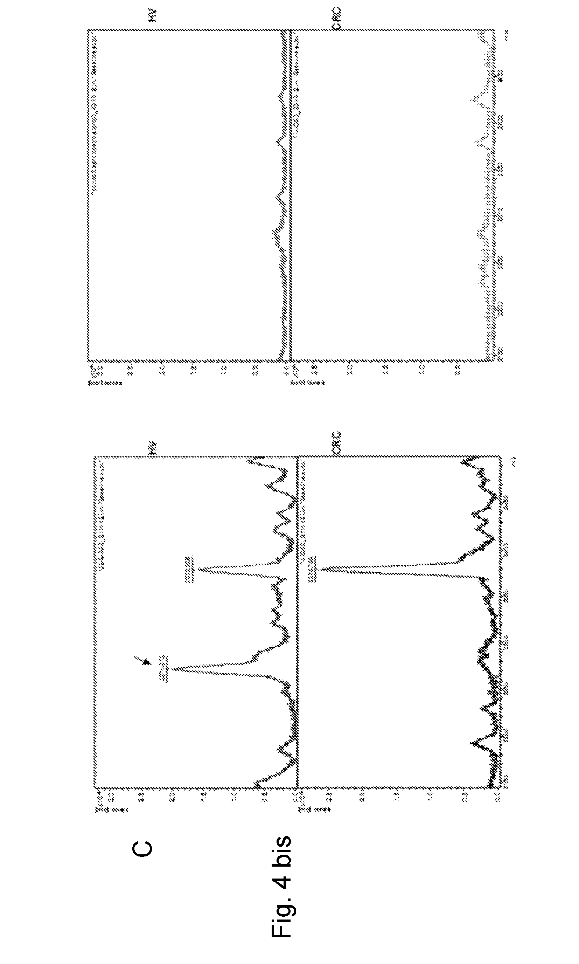

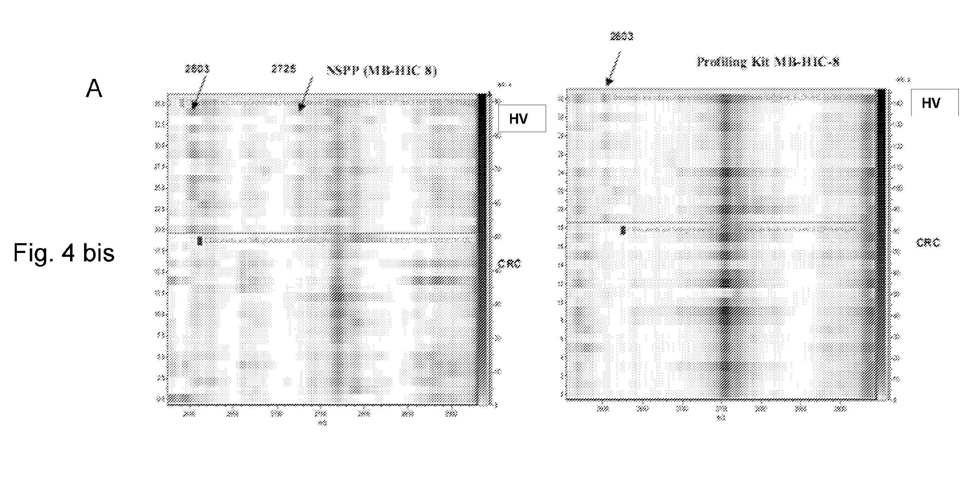

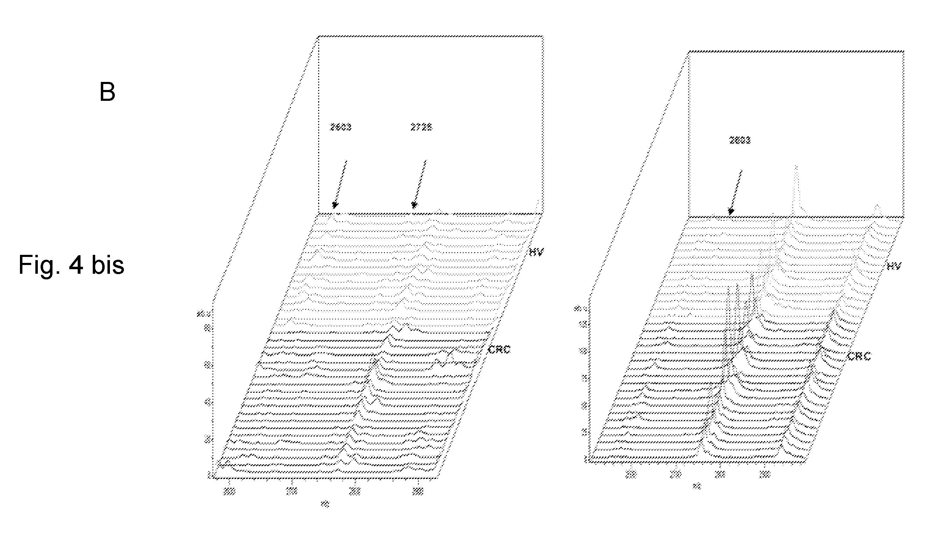

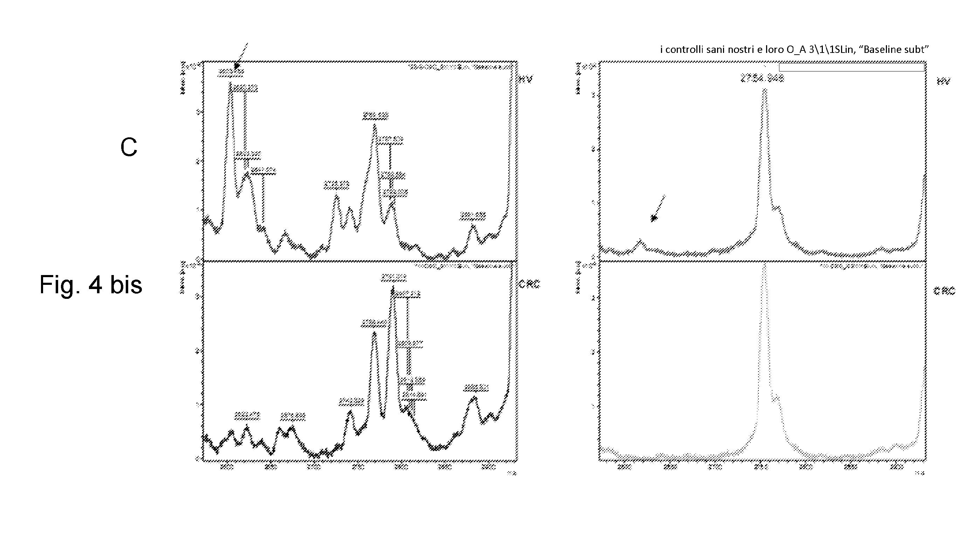

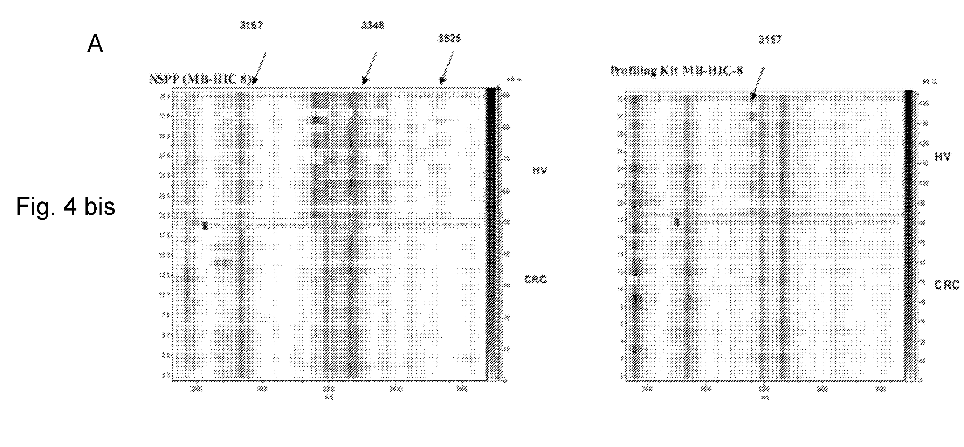

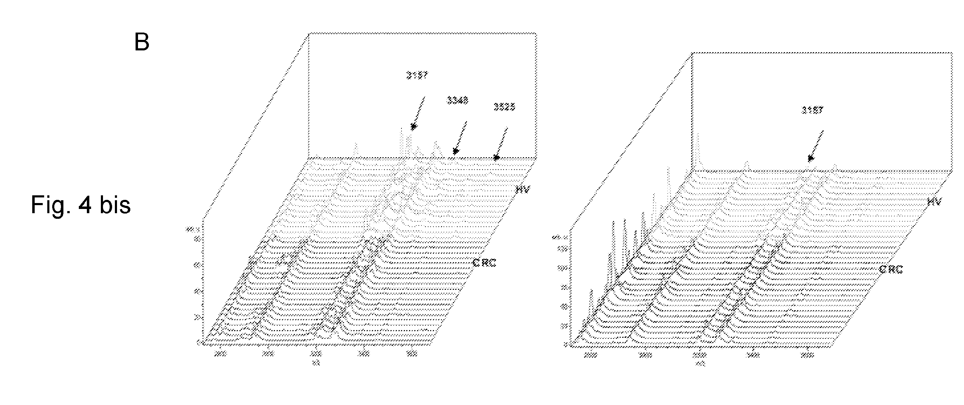

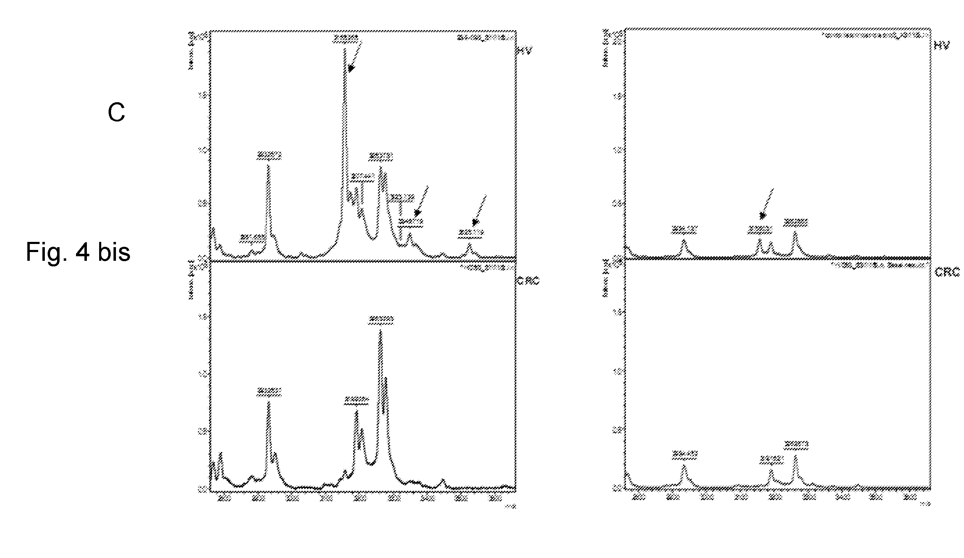

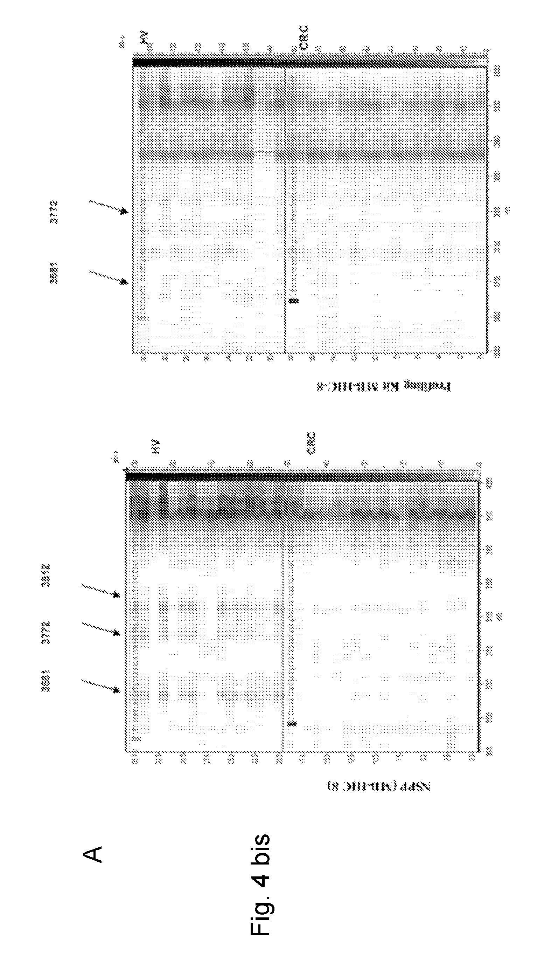

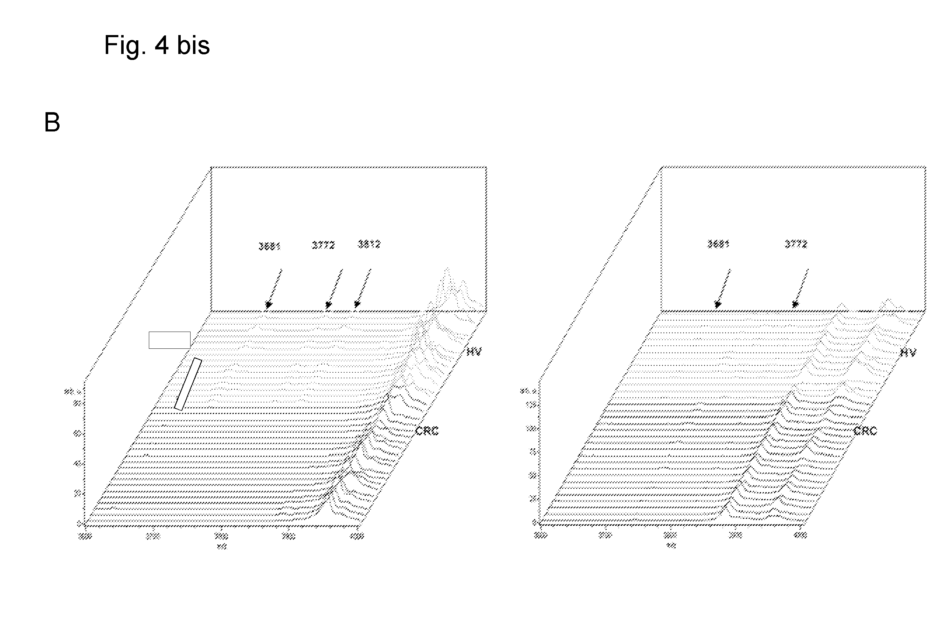

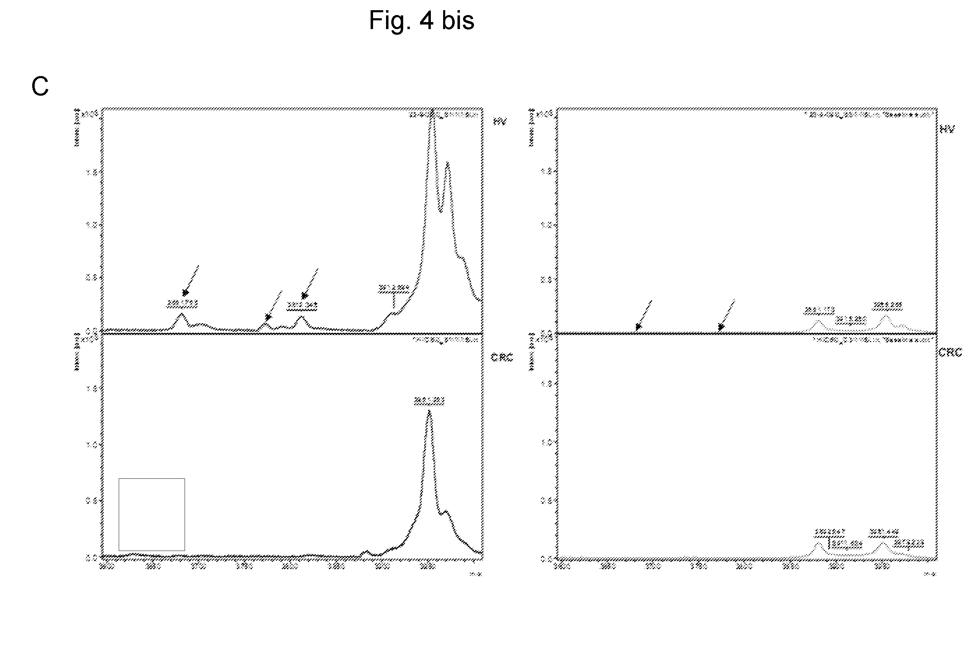

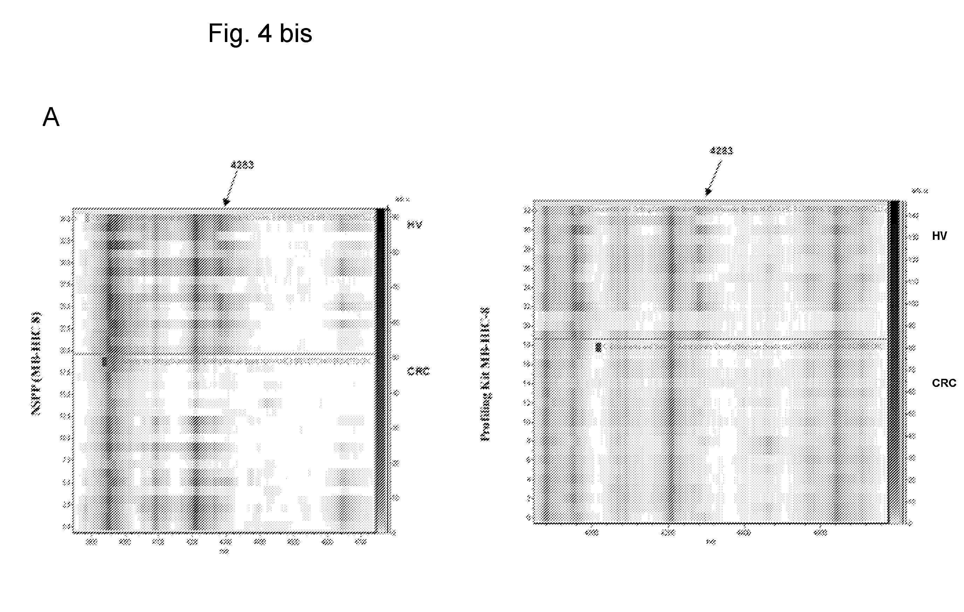

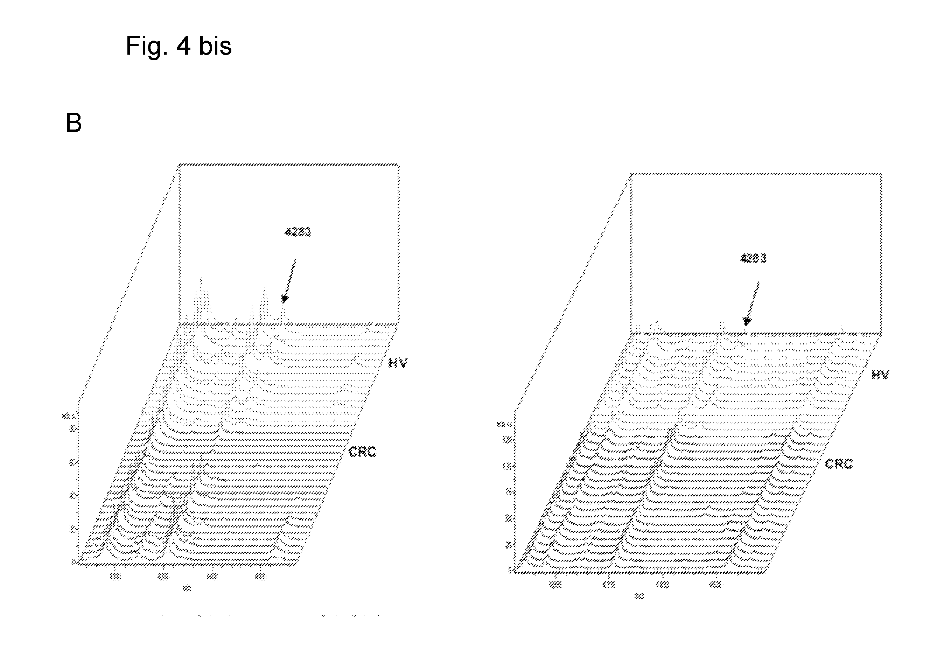

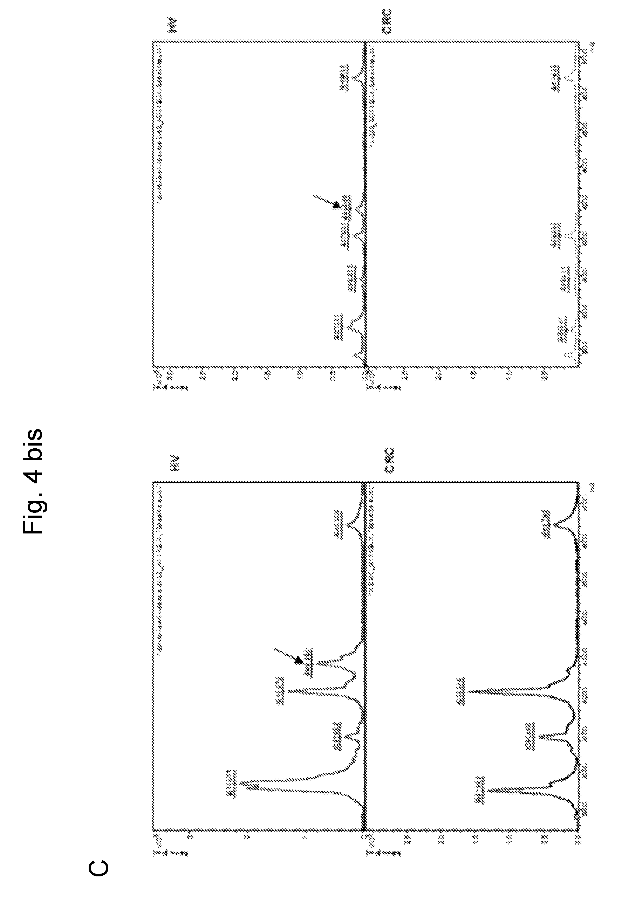

**Figure 4a**. Different viewer options in the ClinProTools bioinformatics package. The pseudo-gel view is the master navigation tool in a data set. All individual spectra are shown in a density scale. NAA MB-HIC8 contains 10 samples, NAA RPC 18 contains 10 samples, ProXpression Kit contains 10 samples: a) comparison between spectra acquired by Dynal Peptide Profiler RPC18 and NAA RPC 18 protocols, b) comparison between spectra acquired by Profiling Kit MB-HIC8 and NAA RPC 18 protocols, c) comparison between spectra acquired by ProXpression Kit and NAA RPC 18 and MB-HIC8 protocols. All spectra were acquired into the region of 0―12,000 Da with multiple differentially displayed signals.

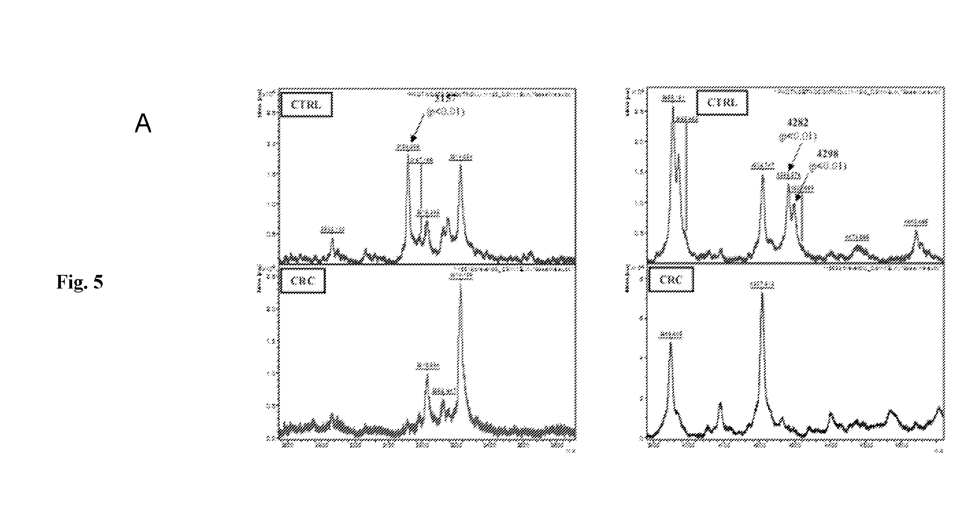

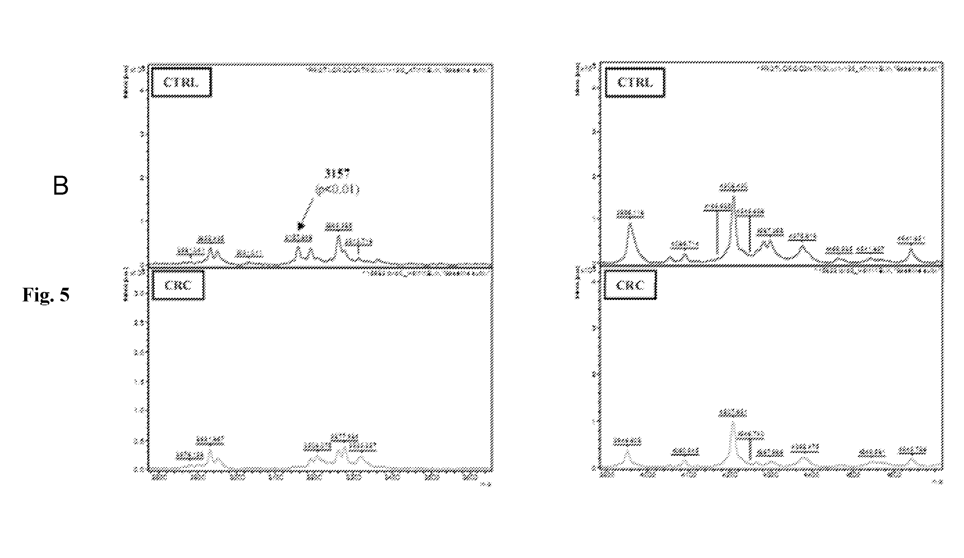

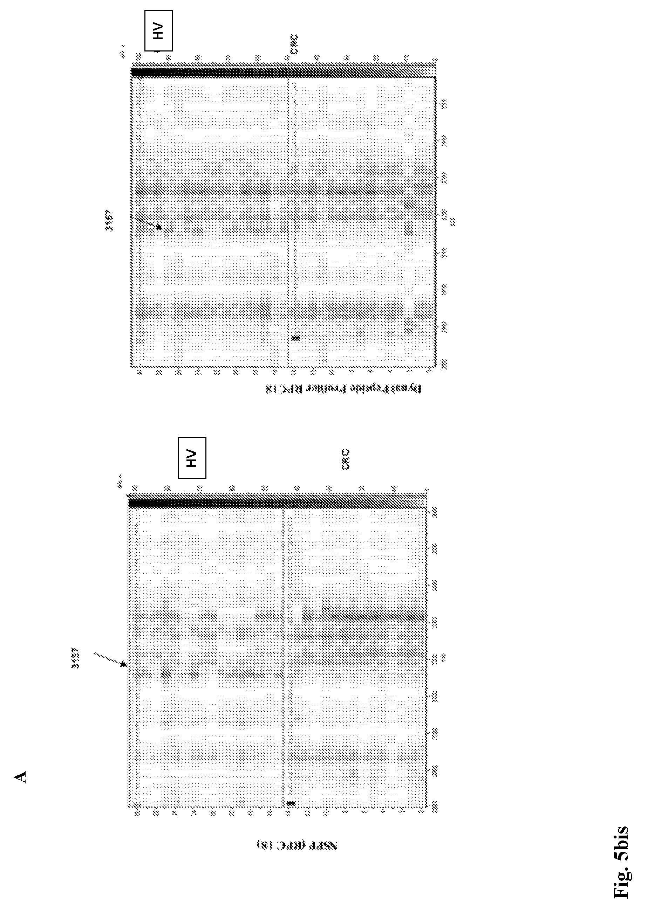

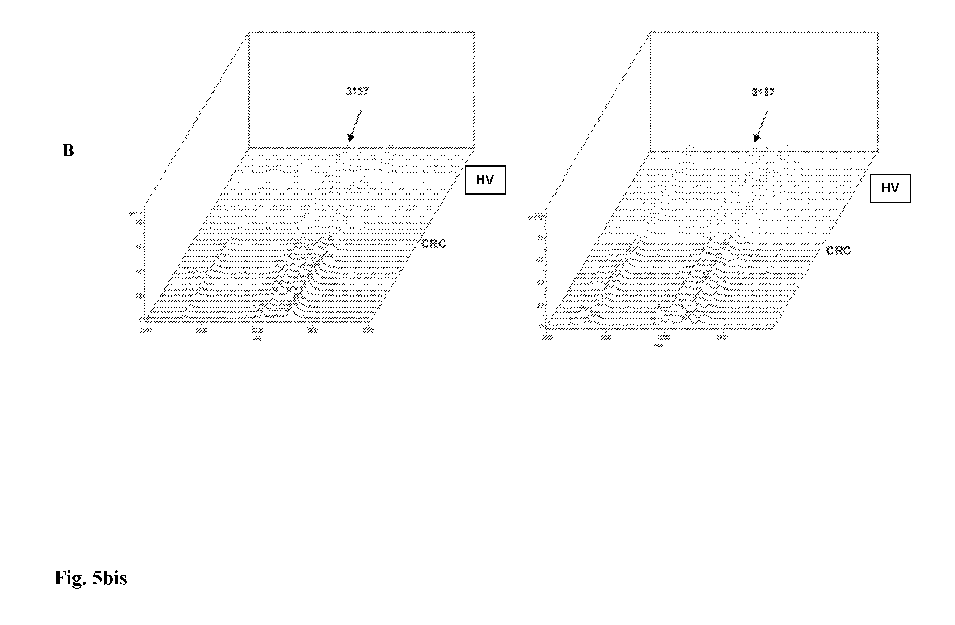

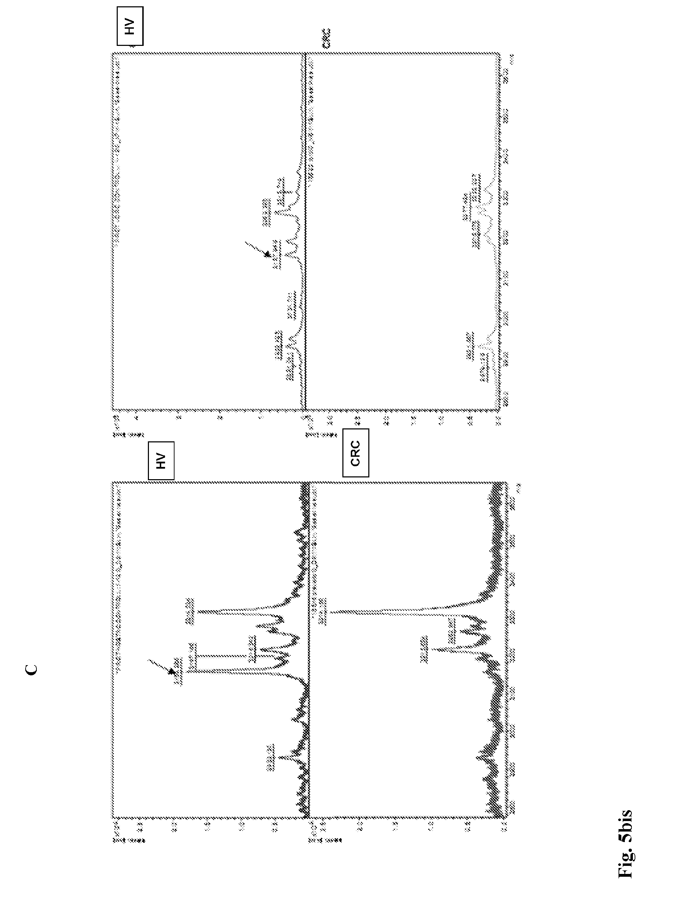

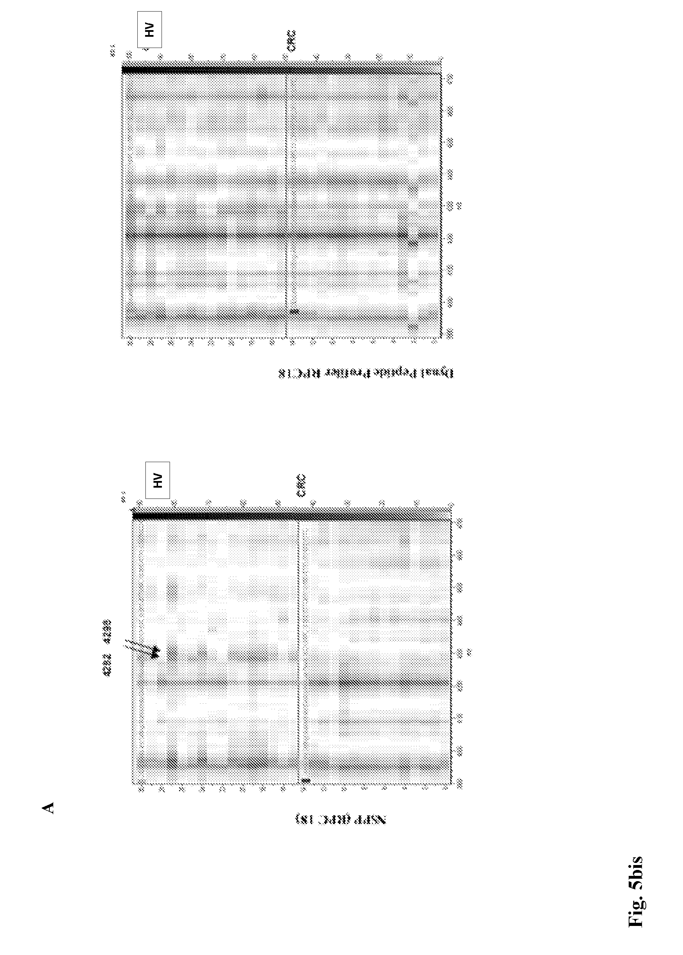

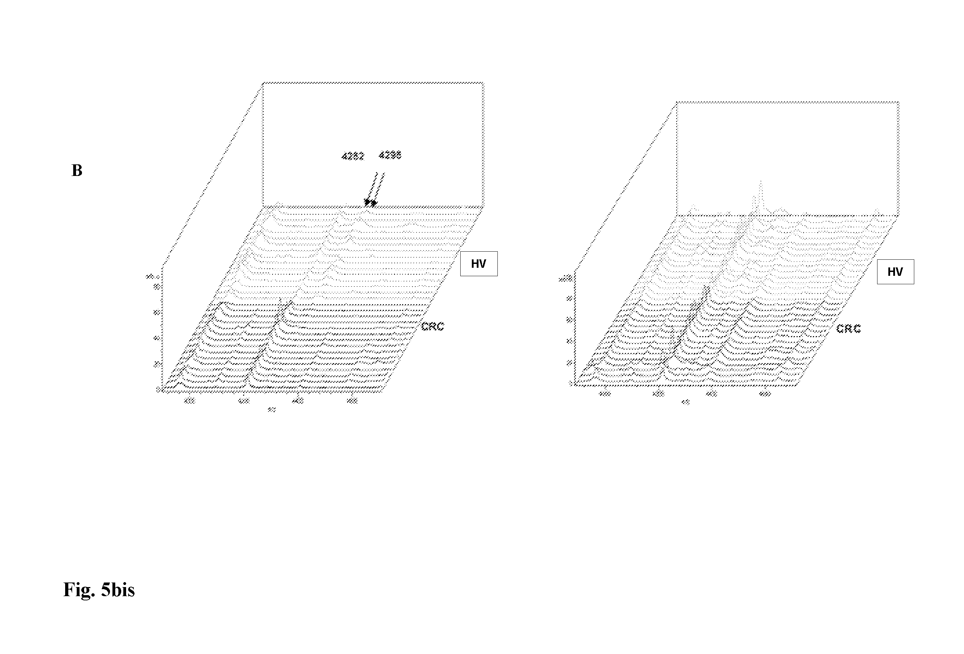

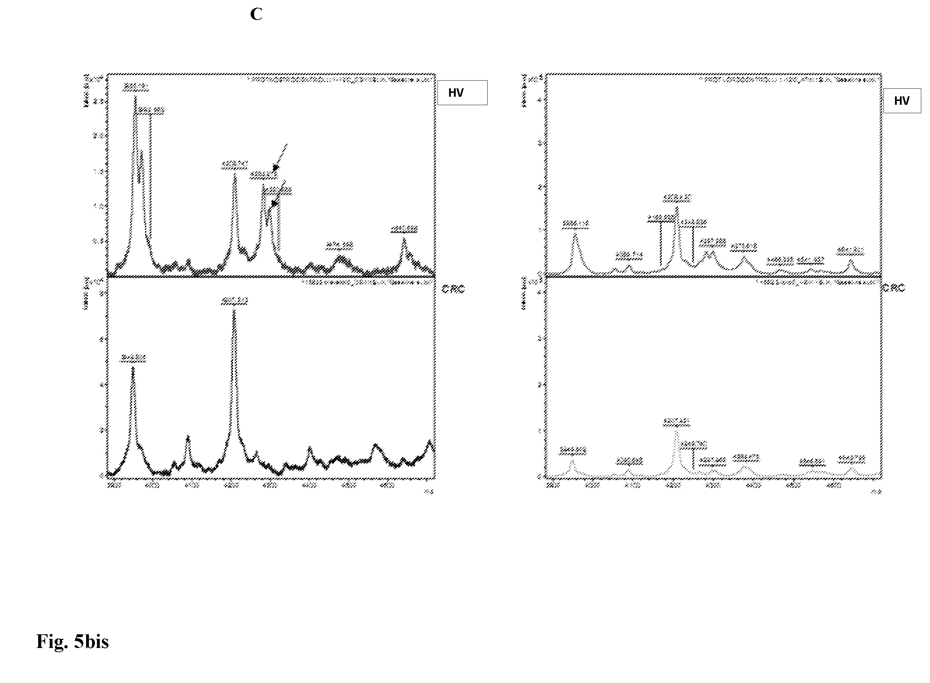

**Figure 5a**. **Feedback between visualization and peak statistics.** A,B,C, D and E (and relative tables 5A-E presented below) show all peaks statistics results calculated with the ClinProTools software. The quality of the peaks and the reproducible value of the analytical protocols can be directly evaluated in the visualization tools. NAA (MB-HIC 8: panel A and RPC 18: panel B) can identify more LMW proteins than Dynal Peptide Profiler RPC18 (panel C) Profiling Kit MB-HIC8 (panel D) and ProXpression Kit protocols (panel E). Moreover, the statistics of NAA is better than ProXpression Kit.
**Figure 1**. Comparison between two commercial kits [Profiling Kit MB-HIC8 (Bruker Daltonics) and Peptide Profiler RPC18 (Invitrogen)] and the new method NSPP was performed evaluating the reproducibility of proteome profiling and LMW proteins recovery. Panels A, B, C and D show the density plot of individual spectra to evaluate the reproducibility of serum proteome profiling and the improved LMW recovery of NSPP compared to Profiling Kit MB-HIC-8 and Dynal Peptide Profiler RPC18. The CV of each corresponding peaks and the average CV of all peaks, within the whole spectra set, are presented on the right.
**Figure 2****.** Panel A and B show the comparison of stacked spectra obtained from serum LMW proteomes profiles using NSPP approach, Profiling Kit MB-HIC-8 and Dynal Peptide Profiler RPC18; the p values of t-test from each corresponding comparison are presented on the right. In Panel C is depicted the spectra of serum LMW proteins obtained using NSPP approach (orange curve) and Profiling Kit MB-HIC-8 (blue curve). Lower panel shows the zoomed spectra into the region of approximate 4,500-10,000 Da to demonstrate variations of LMW proteins recovery.
The corresponding mass region labelled are with a frame in the upper panel. In Panel D is depicted the spectra of serum LMW obtained using NSPP approach (violet curve) and Dynal Peptide Profiler RPC18 (green curve). Lower panels show the zoomed spectra into the region of approximate 2,850-6,000 Da and into the region of approximate 6,000-10,700 to demonstrate variations of LMW protein recovery. The corresponding mass regions labelled are with a frame in the upper panel.
**Figure 3**. SDS-PAGE analysis of serum high molecular weight recovered from magnetic beads using: 1. Dynal Peptide Profiler RPC18 procedure; 2. NSPP (RPC 18); 3. Profiling Kit MB-HIC-8; 4. NSPP (MB-HIC-8). Using NSPP approach, serum samples were almost completely purified from high molecular weight proteins.
**Figure 4**. Impact of different purification approaches on LMW protein profiling obtained in CTRL versus CRC patients. In panel A a pattern of total 12 discriminatory peptides can distinguish CTRL from CRC serum samples using NSPP (MB-HIC 8) purification while, using Profiling Kit MB-HIC-8 (Panel B) the authors detected only 6 discriminatory peptides. The *p* values of t-test from each corresponding comparison are presented under each peak.
**Figure 4bis**. The impact of different purification approach (NSPP, Profiling Kit MB-HIC-8 and Dynal Peptide Profiler RPC18 ) on comparison of LMW recovered from healthy volunteers (HV) versus CRC patients. Different viewer options in the ClinProTools bioinformatics package were used. All individual spectra are shown in a density scale: panels A show the density plot of individual spectra to evaluate the different serum LMW recovery using diverse purification approach; Panel B and C shows the 3-D and 2-D view respectively.

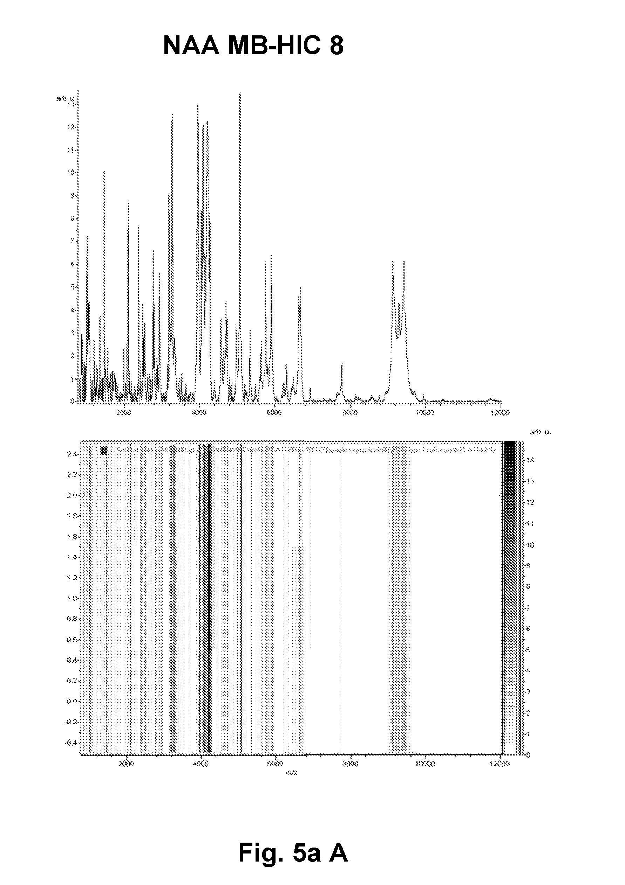

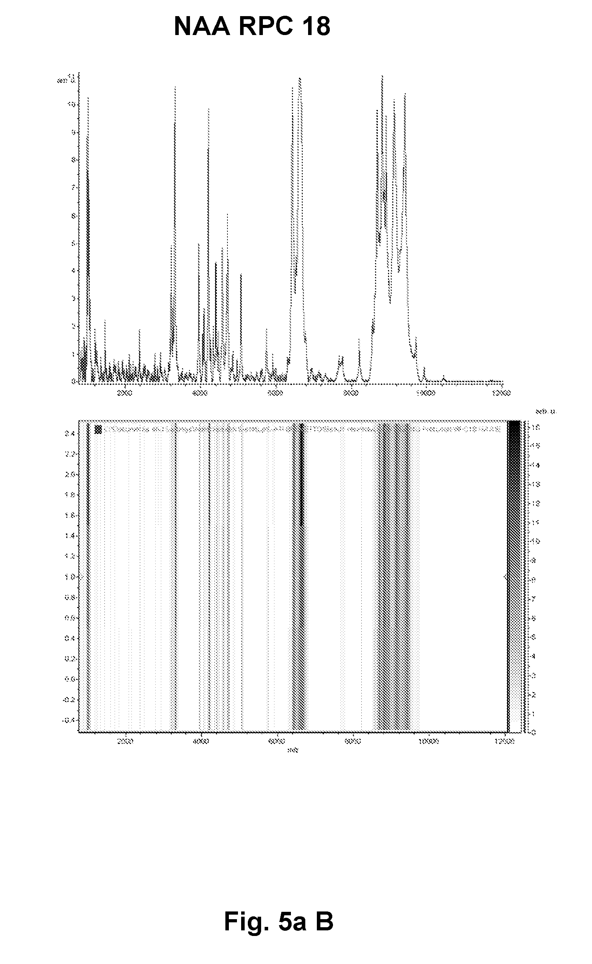

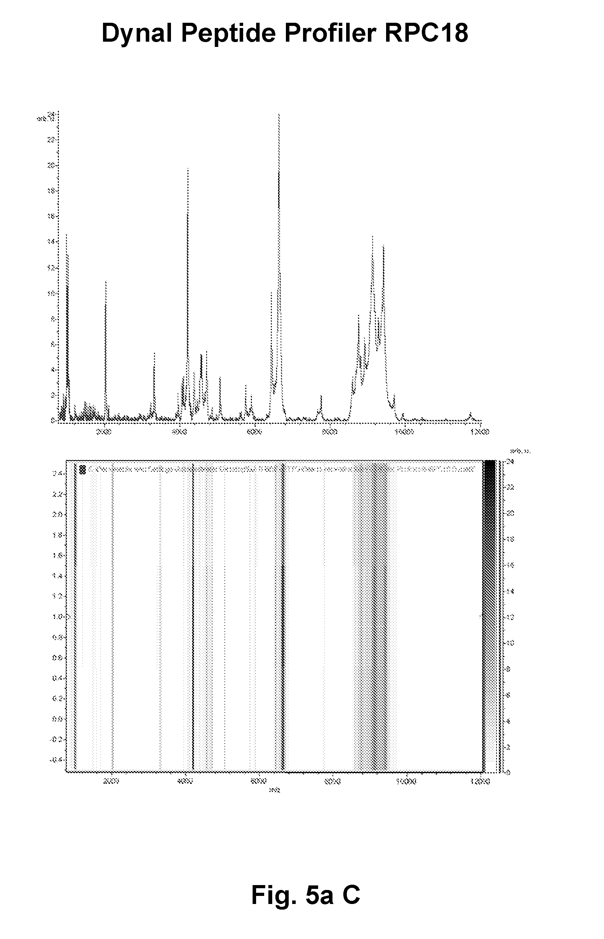

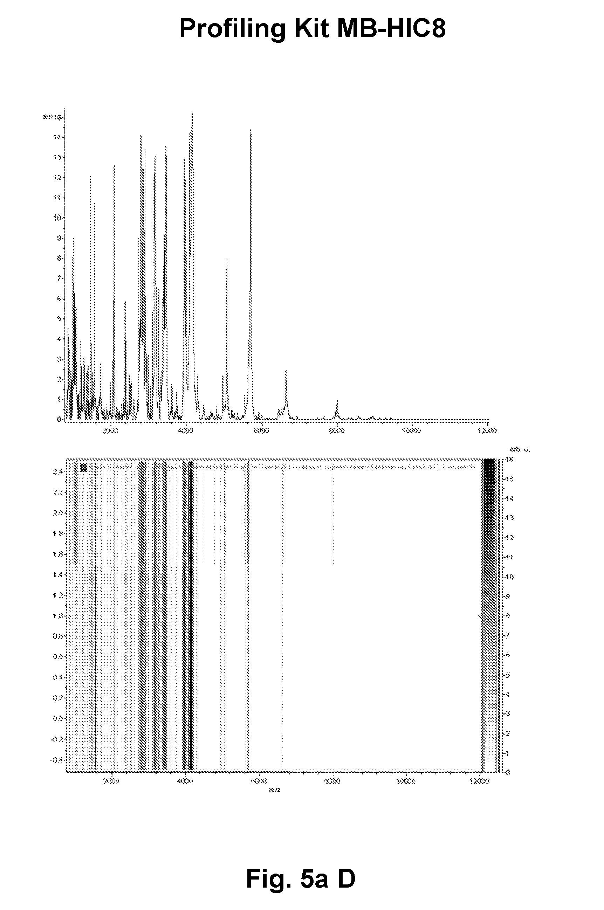

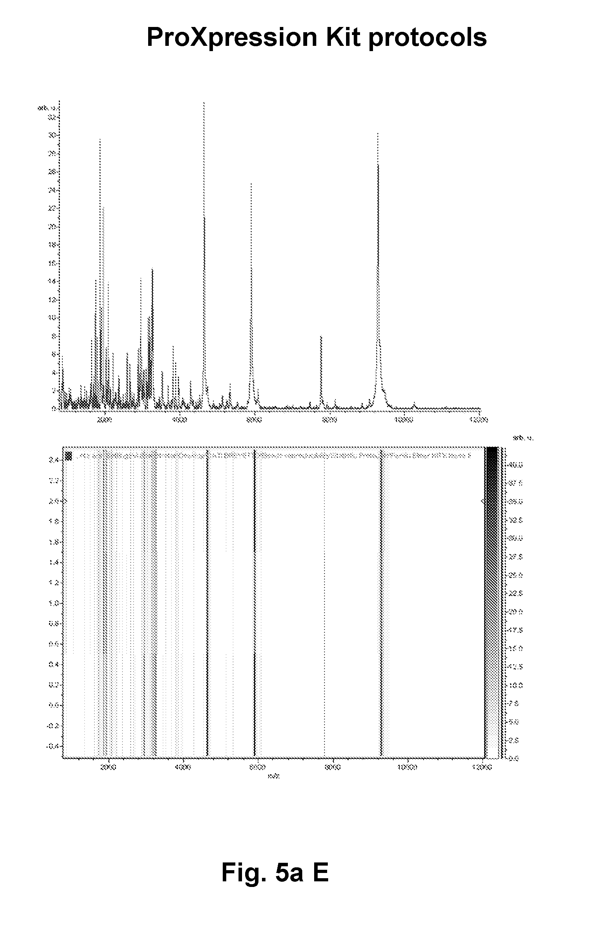

**Figure 5**. Impact of different purification approaches on LMW protein profiling obtained in CTRL versus CRC patients. In panel A a pattern of total 3 discriminatory peptides can distinguish CTRL from CRC serum samples using NSPP (RPC 18) purification while, using Dynal Peptide Profiler RPC18 (Panel B) the authors detected only 1 discriminatory peptide. P values from each corresponding comparison were conducted via the tailed t-test and were presented under each peak.
**Figure 5bis****.** The impact of different purification approach (NSPP, Profiling Kit MB-HIC-8 and Dynal Peptide Profiler RPC18 ) on comparison of LMW recovered from healthy volunteers (HV) versus CRC patients. Different viewer options in the ClinProTools bioinformatics package were used. All individual spectra are shown in a density scale: panels A show the density plot of individual spectra to evaluate the different serum LMW recovery using diverse purification approach; Panel B and C shows the 3-D and 2-D view respectively.

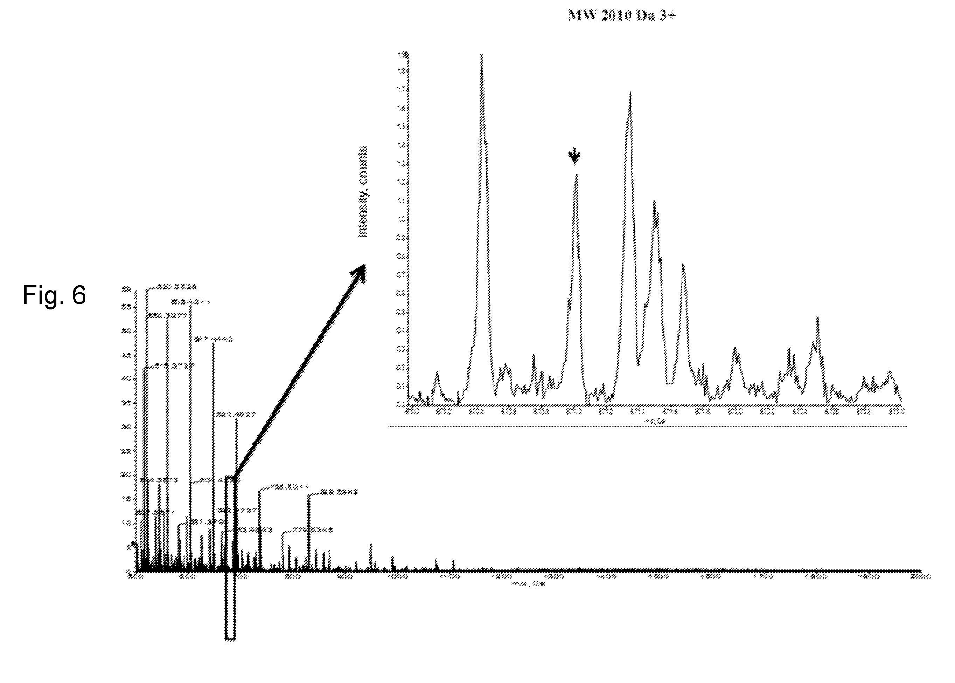

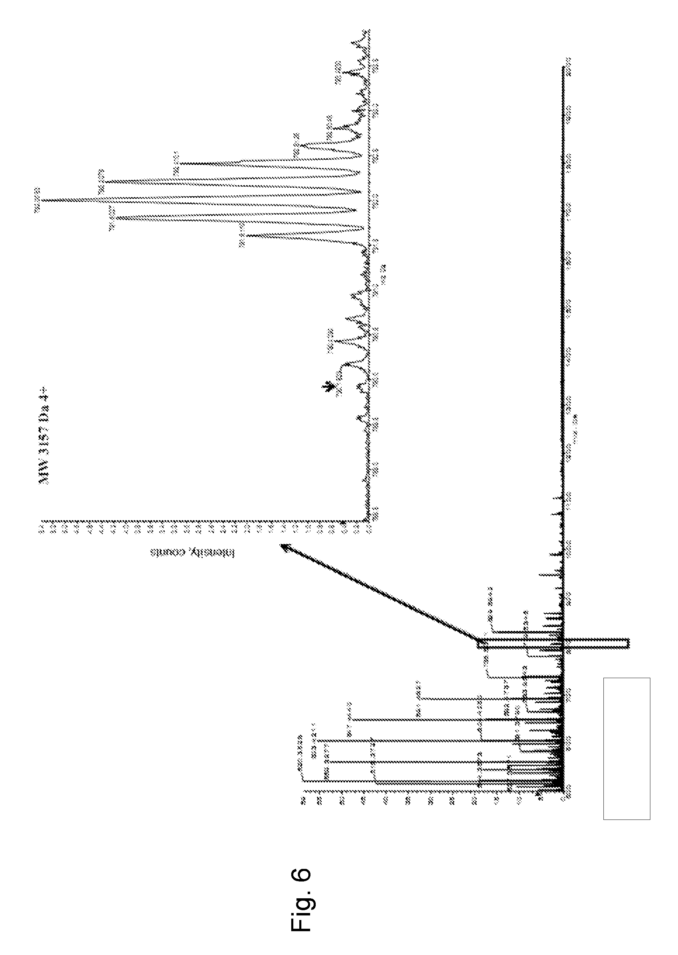

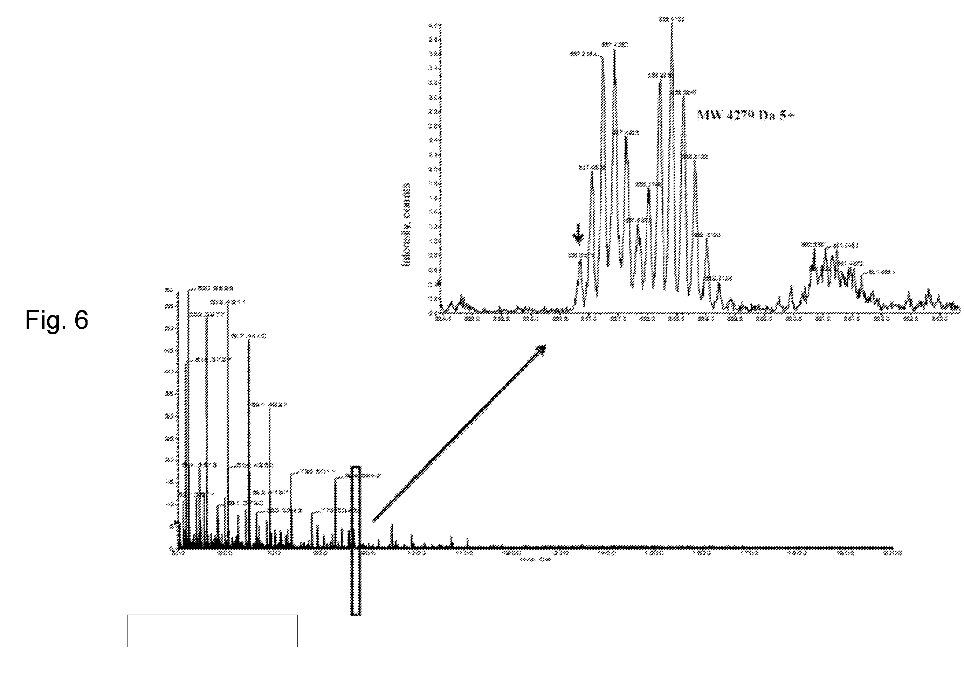

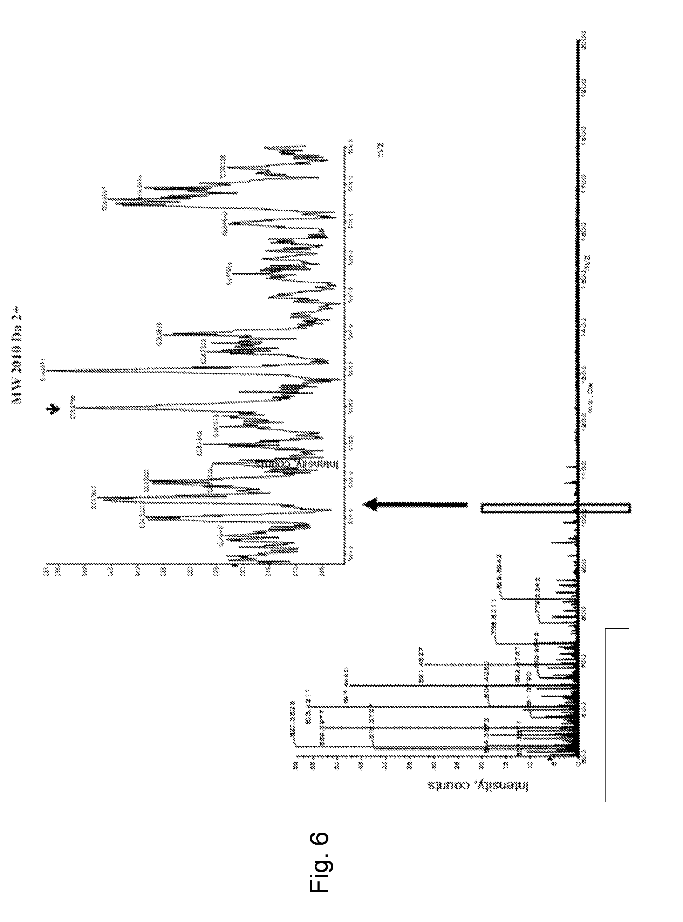

**Figure 6****.** The figure reports a MS spectrum of the discriminatory signals of CRC patients compared to healthy subjects demonstrating the proteic nature of these signals.

### MATERIALS AND METHODS

### Chemical

The α-cyano-4-hydroxycinnamic acid (CHCA), ProteoMass insulin MALDI/MS standard, bombesin protein standard, trifluoroacetic acid (TFA), Dulbecco's phosphate-buffered saline (D-PBS), 1.4-dithioerythritol (DTT) and RPMI 1640 were all purchased from Sigma Aldrich (St. Louis, MO, USA). SilverQuest Staining kit, SDS-PAGE Tricine gel and Dynal Peptide Profiler RPC 18 kit all from Invitrogen Corporation (Carlsbad, CA, USA). Profiling Kit MB-HIC-8 was obtained from Bruker Daltonics (Leipzig, Germany), and hyper grade for liquid chromatography (LC/MS) acetonitrile and deionized water were acquired from Merk (Darmstadt, Germany). Complete protease inhibitor cocktail tablets Roche (Mannheim Germany) and BCA protein assay kit Thermo Fisher Scientific (Rockford, IL USA). Ammonium bicarbonate (NH₄HCO₃) was obtained from Inc Biochemicals (Milwaukee, WI, USA). Iodoacetamide (IAA) from Fluka (Milwaukee, WI), trypsin (TRY) was obtained from Promega (Madison, WI, USA).

### Sample collection and preparation

Blood samples were obtained from 15 consenting healthy donors (CTRL) (aged 40 to 60 years, 8 males and 7 females) and 15 consenting colorectal cancer patients (CRC) (aged 40 to 60 years, 8 males and 7 females, all stage D) prior to any treatment. Cases were age and sex paired. Blood was collected by standard transcutaneous venipuncture into a 4-ml Vacutainer CAT tubes with clot activator (Becton, Dickinson and Company, Plymouth, UK).

The first milliliter of blood was discarded to avoid cellular activation and erythrocyte lyses. The drawn blood was allowed to clot for 1 hour at room temperature, without shaking to avoid haemolysis. Blood samples were centrifuged at 2000g for 5 min at 4°C to separate serum from clot and then centrifuged again at 2400g for 15 min at 4°C to eliminate microparticles. Sera were routinely analyzed by a high sensitivity Hemoglobin Ittero Lipemia test on an Architect c8000 system (Abbott, Irving TX USA) to confirm the absence of haemolysis.

Serum samples were treated with protease inhibitor, aliquoted and immediately stored at -80°C [19].

### NSPP serum low-abundance LMW proteins purification and MALDI target spotting

Serum from CTRL were pooled as a single sample; 80 µl of this serum (kept at 4°C) were treated with 160 µl acetonitrile/TFA 0.1% solution, vigorously agitated and placed at 4°C for 30 minutes. Precipitated serum samples were centrifuged (10000g for 5 min at 4°C), the pellet discarded and the supernatant concentrated to 80 µl in a Savant SpeedVac Systems (Thermo Fisher Scientific Inc. Waltham, MA USA). Protein concentrations were determined using the MicroBCA assay using BSA as a standard (Pierce, Rockford, IL), according to the manufacturer's instructions. The sample (5 µl, 2.5 µg of LMW proteins), was purified 2 times using magnetic beads with reverse phase functional surfaces RPC 18 (Invitrogen) and twice using magnetic beads with reverse phase functional surfaces MB-HIC-8 (Bruker Daltonics), to generate LMW protein profiles. The preparation was performed according to the manufacturer's instructions. The automated purification was performed using TECAN FreedomEVO 150 work surface (Tecan Group Ltd, Männedorf, Switzerland). In order to perform the RPC 18 automated purification, the sample was loaded into tube strip racks on the FreedomEVO 150 work surface. Briefly, 5 µl of serum were robotically mixed with 20 µl magnetic beads with C18-hydrophobic interaction chromatography resin (C18) in a polypropylene plate placed on the magnet (Dynal MPC™ magnet) for 1-2 minutes and supernatant was then removed by aspiration (each sample was purified two times). After removal of the plate from the magnet, the bead/peptides complexes were washed with 200 µl trifluoroacetic acid 0.1 % (TFA) by placing the plate on the magnet between wash steps to aspirate off the solution. Beads were resuspended in 6 µl 50 % acetonitrile and incubated for 2 minutes at RT. The plate was placed again on the magnet and 1 µl of the eluate was robotically removed and loaded on a stainless steel MALDI target plate and allowed to air dry from 3 to 10 minutes at RT. One microliter of the MALDI matrix α-cyano-4-hydroxy-cinnamic acid (3 mg/ml in 50% ACN/2% TFA) was added to each dried serum LMW sample and dried again. In order to perform the MB-HIC-8 purification, 5 µl of serum samples were loaded into tube strip racks on the FreedomEVO 150 work surface and were robotically diluted with 10 µl of a binding solution (each sample was purified two times). The sample was added to the bead slurry (5 µl) in a 0.2-ml polypropylene plate, mixed thoroughly, and incubated for 1 min at RT. Subsequently the polypropylene plate was placed on the magnet (Dynal MPC™ magnet) for 2 minutes and supernatant was then removed by aspiration. After magnetic bead separation and 3 washings, the LMW proteins fraction was eluted from the magnetic beads with 5 µl of an acetonitrile-water mixture (1:1 by volume). One µl of the elute was robotically removed and loaded on a stainless steel MALDI target plate and allowed to air dry from 3 to 10 minutes at RT (loaded in duplicate). One microliter of the MALDI matrix α-cyano-4-hydroxy-cinnamic acid (3 mg/ml in 50% ACN/2% TFA) was added to each dried serum LMW sample and dried again.

The authors obtained four MALDI target's spots from two bead purifications (loaded in duplicate) of serum sample treated with MB-HIC8 and four MALDI target's spots from two bead purifications (loaded in duplicate) of serum sample treated with RPC 18.

### Automated Dynal Peptide Profiler RPC 18 (Invitrogen), automated Profiling Kit MB-HIC-8 (Bruker Daltonics) and ProXpression kit (Perkin Elmer) serum low-abundance Low proteins purification and MALDI target spotting

Serum samples, were also automatically fractionated using functionalized magnetic bead-based kit Dynal Peptide Profiler ^{®} RPC 18, (Invitrogen Corporation) and magnetic bead Profiling Kit MB-HIC-8 (Bruker Daltonics) for the capture of LMW proteins according to the automated manufacturer's protocols. To this purpose, the authors obtained four MALDI target's spots from two beads purification of serum sample (loaded in duplicate) from each of the commercial kits. The same serum sample (the same amount of instant purification), was also treated with Pro Xpression Kit (PerkinElmer, [16b]) according to the manufacturer's instructions.

### MALDI Mass Spectrometry

Mass spectrometry was performed using the ClinProt system, equipped with a Microflex™ *linear* TOF instrument (Bruker Daltonics, Bremen, Germany). Ionization was achieved by irradiation with a nitrogen laser (λ = 337 nm) operating at 50 Hz. For matrix suppression, the authors used a high gating factor with signal suppression up to 500 Da. Mass spectra were detected in linear positive mode. Spectra were collected automatically over a mass range of 500-12000 m/z using the FlexControl™ software (Bruker Daltonics) for fuzzy-controlled adjustment of critical instrument settings to generate raw data of optimized quality. Mass calibration was externally performed using insulin (MW= 5729,61 Da) and bombesin (MW= 1619.85 Da) peaks.

Each MALDI target's spot were divided into nine equal areas (Fig. 1) and 2 acquisitions of 100 shots on good crystals of each area were performed, for a total of 7,200 shots from each serum sample. Finally the authors obtained from four target's spots from each beads purification. To increase the detection sensitivity, the authors removed excess matrix with 6 shots at a laser power of 35% before data acquisition at 28%. The resulting mass spectra showed a mass accuracy of 100 p.p.m. on average with mass resolution between 300-1100 FWHM. All signals with a signal-to-noise (S/N) ratio > 3 in a mass range of 500-12,000 Da were recorded by means of the AutoXecute tool of the flexControl acquisition software (Ver. 2.0; Bruker Daltonics), which uses a peak detection algorithm centroid based on the first and second derivative. For the peak position a value of 80% of Percent Height was specified. This value determines the upper part of the peak that is used to locate the peak position on the x-axis, i.e. m/z. If the separated peak area is symmetric the peak position is considered centric, otherwise the maximum is shifted. Using this peak detection algorithm centroid, the baseline is automatically subtracted, whereas Peak Width parameter was set to 1 m/z. Accordingly, only peaks having this width were incorporated in mass spectra.

### Statistical data analysis of LMW protein MALDI profiles

Image analysis on samples spectra was performed with FlexAnalysis™ 3.0 software (Bruker Daltonics) for image/signals overlapping, and with ClinProTools™ 2.0 software (Bruker Daltonics) for proteomic fingerprinting [14, 20]. Briefly, with ClinProTools each spectrum was processed for baseline subtraction, normalization, recalibration, total spectrum averaging, peak area detection (on total average spectrum), calculation and final normalization. Particularly, baseline subtraction was performed on each spectrum for removing unneeded/interfering broad structures and allowing recalibration and quality checks, while baseline correction was done to remove such structures/artefacts arising from noise averaging in the individual spectra. A Top Hat Baseline algorithm was employed to optimize the baseline correction process [15].

All spectra were normalized with respect to their own total ion count, and each single spectrum was recalibrated using a list of reference masses. Such a list was obtained from the line spectra derived from the original data using peak picking. Only those masses, which occurred in at least 30% of the spectra, were used as reference masses. Reference masses in the peak list of each spectrum were identified by a recalibration algorithm and their number depended on the application used. For measurements within the mass range 500 ― 12,000 Da, approximately 45 to 85 reference masses can be expected. Typically 50 % and more of these peaks are used for the recalibration of individual spectra. The latter, in turn, are used to calculate a total average spectrum, which has been shown to be more advantageous than peak lists obtained from individual spectra [16]. Following peak area detection on the total average spectrum, the area of each peak was calculated by integrating the intensities over the region of the peak. Finally the peak areas were normalized to make different peaks comparable to each other.

Statistical analysis was performed on the spectra with ClinProTools. The average peak intensity, SD and CV (%) for each corresponding peak were calculated. The degree of variation on the basis of the whole spectrum was thus determined by calculating the CV values for all of the peaks of the tested spectra. Besides, the authors defined average CV (%) values for all of the peaks of the tested spectra set. To compare paired sample sources (such as NSPP, Peptide Profiler RPC18 and Profiling Kit MB-HIC8 approach), the authors used the two tailed *t*-test calculating the *p* value for each defined corresponding peak between the two compared groups, and assessing statistical difference for *p* < 0.01.

To evaluate the analytical recovery of LMW proteins and the reproducibility on LMW profiles, the authors compared 30 average spectra of LMW proteins from NSPP purification, 30 from Dynal Peptide Profiler RPC 18 approach and 30 from Profiling Kit MB-HIC-8 approach of the same pooled serum.

### In-gel tryptic digestion

Ten µg of LMW proteins from each purification, NSPP (MB-HIC 8) Profiling Kit MB-HIC-8 NSPP (RPC 18) and Dynal Peptide Profiler RPC18, were lyophilized, separated by SDS-PAGE using a 10-20% Tricine gel (Invitrogen, Carlsbad, CA, USA) and the gel was stained with SilverQuest Staining kit (Invitrogen). After visualization, each sample slice was cut, destained and covered with acetonitrile until gel pieces shrunk. Acetonitrile was removed and gel particles dried by centrifugation under vacuum. Proteins were reduced (10 mM DTT, 25 mM ammonium carbonate) for 30 min at 56°C, cooled to room temperature and alkylated (55 mM IAA, 25 mM ammonium carbonate) for 30 min in the dark at room temperature. Gel pieces were washed in 50 mM ammonium hydrogencarbonate/acetonitrile 1:1 for 15 min and covered by acetonitrile until gel pieces shrunk. Acetonitrile was removed and gel particles dried by centrifugation under vacuum. In-gel digestion was performed by adding 12.5 ng/µl of trypsin in 25 mM ammonium carbonate at 37°C overnight under stirring.

### LC-ESI-MS/MS analysis of the gel slices

Samples (5 µl) were mixed with equal volume of 3% acetonitrile, 2% formic acid and analysed in micro LC-ESI-MS/MS. Chromatographic separations were carried out using a 300 µm ID x 15 cm long fused silica capillary column (Vydac, 300 A). Micro-RPLC was performed using an Agilent 1100 system (Agilent Technologies). After injection of 5 µl of the LMW sample, the column was washed for 5 min with 95 % mobile phase A (95 % H₂O, 5% CH₃CN, 0.1 % HCOOH) and proteins/peptides eluted using a linear gradient from 5 % to 95 % mobile phase B (95 % CH3CN, 5% H₂O, 0.1 % HCOOH) over 75 min with a constant flow rate of 5 µl/min. The column was washed for 10 min with 95 % of mobile phase B and re-equilibrated with 5 % of mobile phase B for 20 min prior to sample loading. Micro-RPLC column was coupled online to an Ion Trap MS/MS (HCT plus Bruker Daltonics Bremen, Germany) using a micro-electrospray source with an applied electrospray potential of 1800 V, m/z range was 350-1400 for MS and 100-1800 for MS/MS. Spectra were recorded using external calibration. Raw MS/MS data were analysed using Data Analysis control provided by the manufacturer and reported as monoisotopic masses.

### Application of LMW proteins purification strategies on CRC patients sera

Using NSPP (MB-HIC 8), Profiling Kit MB-HIC-8, NSPP (RPC 18) and Dynal Peptide Profiler RPC18 purification protocols (on the same serum aliquot from each subject), the authors analyzed 15 CRC patients and 15 consenting CTRL and finally the authors compared profiles MALDI mass spectra of them. To test the characterization of some significant MS peaks, the authors acidified by formic acid to final concentration of 0.1% and injected the samples into nano ESI qQToF system (QSTAR Applied Biosystem, Toronto, Canada).

### RESULTS

### Analysis of haemolysis on serum samples

Haemolysis often results in the identification of additional peaks, particularly with molecular masses lower than 7000 Da [21]. All tested serum samples were haemolysis-free (data not shown).

### Initial results

The instant method (NAA or NSPP) was developed and optimized to high recovery of LMW protein from serum and to obtain highly specific and reproducible serum LMW protein profiling through MALDI-TOF mass spectrometry technique.

The instant method (NAA or NSPP) uses acetonitrile and trifluoroacetic acid precipitation, which has been shown to effectively precipitate large abundant proteins (albumin and immunoglobulins) out of the serum, to show up low abundance LMW peptides and proteins. Acetonitrile denatures high carrier proteins allowing carriers removal and causing the small, low-abundance proteins and peptides normally bound to these carrier proteins to dissociate. Funtionalized reverse phase magnetic beads, allow to recover these LMW thus making them available for detection by mass spectrometry [10, 11]. Profiling Kit MB-HIC8 and Dynal Peptide Profiler RPC18 are mixed with raw serum samples and allow to recovery only free LMW (no-linked to albumin and high abundant carrier proteins). The peptides and proteins extracted are subsequently eluted and analysed by mass spectrometry. The ProXpression Kit selectively captures abundant carrier proteins, followed by eluting and concentrating the biomarkers associated with them. This kit uses the Vivapure 96 Blue membranes that utilize the membrane chromatography technology of Viva science AG combined with PerkinElmer's buffer and elution chemistries to quickly and reproducibly elute the peptide biomarkers bound to albumin. Both NAA sample, Profiling Kit MB-HIC8 sample, Dynal Peptide Profiler RPC18 sample and ProXpression Kit sample, were analyzed through mass spectrometry MALDI-TOF technique, therefore sample crystallization is crucial to obtain very clean spectra. Using sample crystallization technique for both experimental procedures above described, the authors obtained a very high sample crystallization homogeneous on MALDI target's spot (fig. 2a) and very clean spectra easily comparable to each other. While ProXpression Kit allowed to recover only the LMW linked to high carrier protein, Profiling Kit MB-HIC8 and Dynal Peptide Profiler RPC18 both allowed to recover only the free LMW (no-linked with high carrier protein) in raw serum, the instant method retrieved both LMW-related carrier proteins and free LMW. Figure 3a shows the best number of LMW recovery of the instant method compared with the low number of LMW recovery of the Profiling Kit MB-HIC8, Dynal Peptide Profiler RPC18 and ProXpression Kit. Subsequently, ClinProTools software (Bruker Daltonik) was used for statistical analysis, data interpretation and comparison of MALDI-TOF spectra derived from the instant method, Profiling Kit MB-HIC8, Dynal Peptide Profiler RPC18 and Pro Xpression Kit LMW samples purification. ClinProTools offers a variety of viewer options for the analysis of clinical profiling data (Figure 4a). A virtual gel view gives an overview representation of data sets derived from large sample cohorts. This viewer is the master navigation tool for the large data set. A representation of the signal intensities in a grey scale allows for detection of faint differences between the increasing peak height (Figure 4a).

Finally, ten sample of each purification protocol (NAA HIC8: Fig. 5aA and Table 5A, NAA RPC18: Fig. 5aB and Table 5B, Dynal Peptide Profiler RPC18: Fig. 5aC and Table 5C, Profiling Kit MB-HIC8: Fig. 5aD and Table 5D, ProXpression Kit: Fig. 5aE and Table 5E), were divided in two classes of five samples. These new classes were analyzed through ClinProTools software that provides a list of peaks sorted according to the statistical significance to differentiate between both classes. A list of all peaks sorted according to the statistical separation strength is created. Tables 5A-E show all peaks picked in peak calculation along with several values. The following data are displayed for each peak:

S, Inclusion/exclusion state of the peak: 'X' used for model generation (= included); '-' not used for model generation (= excluded); *Index:* Peak index; *Mass:* m/z value; *Dave:* Difference between the maximal and the minimal average peak area of all classes; *PTTA:* P-value of t-test (2 classes) or ANOVA test (> 2 classes), range 0..1; 0: good, 1: bad. Preferable for normal distributed data; *PWKW:* P-value of Wilcoxon (2 classes) or Kruskal-Wallis test (> 2 classes), range 0..1; 0: good, 1: bad. Preferable for not normal distributed data; *PAD:* P-value of Anderson-Darling test; gives information about normal distribution; range 0..1; 0: normal distributed, 1: not normal distributed; *AveN:* Peak area average of class N; *StdDevN:* Standard deviation of the peak area average of class N.

**Table 5A**

| | | | | | | | | | | |
|---|---|---|---|---|---|---|---|---|---|---|
| **ClinProt Peak Statistic** | | | | | | | | | | |
| ClinProTools Version 20 build 365 | | | | | | | | | | |
| Number of peaks 64 | | | | | | | | | | |
| Sort Mode p value tta | | | | | | | | | | |

| S | Index | Mass | DAve | PTTA | PWKW | PAD | Ave1 | Ave2 | StdDev1 | StdDev2 |
|---|---|---|---|---|---|---|---|---|---|---|
| X | 33 | 4209 42 00 | 151 78 | 0,488888889 | 0 08 | 1 | 885 15 00 | 1036 93 | 92 89 | 83 44 00 |
| X | 34 | 4267 39 00 | 59 83 | 0,488888889 | 0 08 | 1 | 184 58 00 | 244 41 00 | 1643 | 39 04 00 |
| X | 31 | 409085 | 36 11 00 | 0,488888889 | 008 | 1 | 3064200 | 3425200 | 15 31 | 1237 |
| X | 60 | 9284 19 00 | 30 23 00 | 0,488888889 | 0 08 | 1 | 250 31 00 | 220 09 00 | 18 53 | 20 03 |
| X | 30 | 40544700 | 2055 | 0,488888889 | 008 | 1 | 1700800 | 191 35 00 | 0,422916667 | 1435 |
| X | 32 | 4118 43 00 | 17 56 | 0,488888889 | 008 | 1 | 144 31 00 | 161 87 | 753 | 1227 |
| X | 29 | 4015 42 00 | 0,679861111 | 0,488888889 | 0 08 | 1 | 30 22 00 | 46 01 00 | 0,188888889 | 5 03 |
| X | 4 | 105979 | 8 02 | 0,488888889 | 0 08 | 1 | 2598 | 34 17 00 | 0,225694444 | 3 01 |
| X | 62 | 9707 46 00 | 0,258333333 | 0,488888889 | 008 | 1 | 292500 | 2353 | 1 05 | 0.14375 |
| X | 63 | 99320700 | 0,145138889 | 0,488888889 | 008 | 1 | 0,564583333 | 1003 | 0,089583333 | 0.04375 |
| X | 52 | 7670 21 00 | 209 | 0,488888889 | 008 | 1 | 0,517361111 | 0,427777778 | 1 05 | 1 53 |
| X | 51 | 7646 31 00 | 1 13 | 0,488888889 | 008 | 1 | 0.50625 | 1056 | 006 | 054 |
| X | 59 | 913071 | 13076 | 0,547222222 | 008 | 1 | 7202800 | 589 51 00 | 8496 | 994400 |
| X | 61 | 9419 09 00 | 72 87 | 0,59375 | 008 | 1 | 53298 | 460 11 00 | 51 06 00 | 72 97 |
| X | 35 | 457072 | 27 45 00 | 0,59375 | 008 | 1 | 189 04 00 | 161 59 00 | 0,761111111 | 25 96 |
| X | 37 | 47100200 | 0.96875 | 0,59375 | 008 | 1 | 205 44 00 | 18269 | 17 46 | 2231 |
| X | 28 | 3951 47 00 | 0.811805556 | 0,59375 | 0,658333333 | 1 | 3680600 | 38695 | 18 01 | 0,54375 |
| X | 38 | 4963 97 | 5 51 | 0.59375 | 0,658333333 | 1 | 67 06 00 | 61 55 00 | 5 03 | 4 08 |
| X | 49 | 6677 01 00 | 4294 | 0,615972222 | 1 | 1 | 201 07 00 | 158 13 00 | 81 | 520800 |
| X | 55 | 821277 | 2026 | 0,615972222 | 1 | 1 | 29 82 | 957 | 362200 | 055 |
| X | 3 | 10370600 | 0,525 | 0,615972222 | 0,658333333 | 1 | 92 17 00 | 1041400 | 15 36 | 0.595138889 |
| X | 1 | 98769 | 0,472916667 | 0,615972222 | 1 | 1 | 824300 | 932500 | 0.9 | 0.545138889 |
| X | 25 | 3314 21 00 | 0,427777778 | 0,615972222 | 1 | 1 | 77 05 00 | 672900 | 264900 | 0,651388889 |
| X | 2 | 1013 43 00 | 939 | 0,615972222 | 0,658333333 | 1 | 118 35 00 | 12774 | 0,793055556 | 1604 |
| X | 24 | 327886 | 725 | 0,615972222 | 0,658333333 | 1 | 99 14 00 | 91 89 | 8 38 | 8 38 |
| X | 23 | 3263 36 00 | 7 05 | 0,615972222 | 1 | 1 | 20284 | 20989 | 19 01 | 8 05 |
| X | 26 | 3337 01 00 | 0,314583333 | 0,615972222 | 1 | 1 | 43 99 | 37 06 00 | 9 52 | 11 02 |
| X | 40 | 506579 | 642 | 0,615972222 | 1 | 1 | 297 59 00 | 304 01 00 | 1258 | 3 33 |
| X | 53 | 7764 01 00 | 0.275694444 | 0,615972222 | 0,658333333 | 1 | 64 01 00 | 58 04 00 | 7 41 | 1 04 |
| X | 47 | 6301 94 | 0,227083333 | 0,615972222 | 0,658333333 | 1 | 41 0900 | 45 96 | 0,265972222 | 0.140277778 |
| X | 21 | 3209 16 00 | 0,227083333 | 0,615972222 | 0,658333333 | 1 | 64 52 00 | 5965 | 7 59 | 4 46 |
| X | 43 | 57525500 | 453 | 0,615972222 | 1 | 1 | 15673 | 1520200 | 13 23 | 501 |
| X | 36 | 4643 36 00 | 4 23 | 0,615972222 | 0.658333333 | 1 | 6561 | 61 39 00 | 6 24 | 0.085416667 |
| X | 27 | 33640900 | 0,16875 | 0,615972222 | 0,658333333 | 1 | 4384 | 40 21 00 | 225 | 4 32 |
| X | 56 | 8563 72 | 349 | 0,615972222 | 1 | 1 | 0.525 | 847 | 835 | 057 |
| X | 22 | 3241 03 00 | 0,148611111 | 0,615972222 | 0,658333333 | 1 | 41 69 | 38 75 | 4 07 | 0.141666667 |
| X | 54 | 81400300 | 247 | 0,615972222 | 1 | 1 | 14 04 | 11 57 | 354 | 041 |
| X | 58 | 89681500 | 2 18 | 0,615972222 | 1 | 1 | 11 59 | 941 | 0,138194444 | 0,149305556 |
| X | 41 | 5337 13 00 | 2 18 | 0,615972222 | 1 | 1 | 604600 | 58 28 00 | 0,211111111 | 3 11 |
| X | 14 | 27905500 | 2 15 | 0,615972222 | 1 | 1 | 590500 | 573500 | 0.261111111 | 0,174305556 |
| X | 17 | 29320900 | 0.09375 | 0,615972222 | 1 | 1 | 700400 | 682900 | 0,224305556 | 229 |
| X | 16 | 2876 21 00 | 144 | 0,615972222 | 1 | 1 | 332800 | 31 84 | 302 | 1 19 |
| X | 12 | 253263 | 1 36 | 0,615972222 | 1 | 1 | 495400 | 500900 | 3 36 | 1 51 |
| X | 15 | 2861 04 00 | 1 34 | 0.615972222 | 1 | 1 | 0,836111111 | 18 03 | 2 17 | 1 09 |
| X | 50 | 6933 59 00 | 0,066666667 | 0,615972222 | 1 | 1 | 1604 | 17 36 | 2 52 | 0,098611111 |
| X | 48 | 66324500 | 390500 | 0,619444444 | 1 | 1 | 194 58 00 | 155 53 00 | 134 71 | 642300 |
| X | 13 | 276961 | 259 | 0,619444444 | 1 | 1 | 8667 | 840800 | 0,351388889 | 0.234722222 |
| X | 44 | 580674 | 0,065972222 | 0,619444444 | 1 | 1 | 411600 | 42 11 00 | 209 | 1 43 |
| X | 7 | 1975 26 00 | 0,047222222 | 0,619444444 | 1 | 1 | 23 19 | 1,01875 | 2 34 | 1 11 |
| X | 6 | 1465 34 00 | 0,18125 | 0,6625 | 1 | 1 | 1072800 | 111 09 00 | 1601 | 1004 |
| X | 11 | 24954700 | 1 15 | 0.6625 | 1 | 1 | 49 | 4785 | 0,225 | 0.171527778 |
| X | 19 | 3159 09 00 | 039 | 0.663194444 | 1 | 1 | 0,926388889 | 21 35 | 1 08 | 1 26 |
| X | 46 | 5904 32 00 | 258 | 0,68125 | 1 | 1 | 221 53 00 | 218 94 | 15 11 | 805 |
| X | 42 | 563366 | 0,063194444 | 0,688888889 | 1 | 1 | 620500 | 61 59 00 | 729 | 403 |
| X | 57 | 8929 08 00 | 0,049305556 | 0.688888889 | 1 | 1 | 11 03 | 10 06 | 0,235416667 | 0,141666667 |
| X | 20 | 3192 41 00 | 0 07 | 0,688888889 | 1 | 1 | 119 46 00 | 118 75 | 4 09 | 0.309027778 |
| X | 18 | 2951 39 00 | 0 39 | 0,688888889 | 1 | 1 | 44 07 00 | 44 32 00 | 3 59 | 1 16 |
| X | 39 | 502475 | 023 | 0,688888889 | 1 | 1 | 31 07 00 | 31 29 00 | 0,129861111 | 0,054166667 |
| X | 9 | 2104 46 00 | 046 | 0,69375 | 1 | 1 | 10286 | 1020400 | 9 36 | 734 |
| X | 10 | 2379 16 00 | 039 | 0,69375 | 1 | 1 | 8497 | 853600 | 5 42 | 0,257638889 |
| X | 8 | 204486 | 0 08 | 0.69375 | 1 | 1 | 2326 | 23 18 | 2 39 | 2 23 |
| X | 64 | 1172447 | 005 | 0,69375 | 1 | 1 | 0,355555556 | 0,359027778 | 0,0875 | 0,105555556 |
| X | 45 | 5868 96 | 0 05 | 0,69375 | 1 | 1 | 4977 | 4973 | 3 09 | 1 54 |
| X | 5 | 13504300 | 0 | 0,69375 | 1 | 1 | 30 | 30 | 2 19 | 0,227777778 |

**Table 5B**

| | | | | | | | | | | |
|---|---|---|---|---|---|---|---|---|---|---|
| **ClinProt Peak Statistic** | | | | | | | | | | |
| ClinProTools Version 2 0 build 365 | | | | | | | | | | |
| Number of peaks 62 | | | | | | | | | | |
| Sort Mode value tta | | | | | | | | | | |

| S | Index | Mass | DAve | PTTA | PWKW | PAD | Ave1 | Ave2 | StdDev1 | StdDev2 |
|---|---|---|---|---|---|---|---|---|---|---|
| X | 40 | 6629 51 00 | 935900 | 0.688194444 | 1 | 1 | 1619 58 00 | 1713 17 00 | 165 53 00 | 184 88 |
| X | 38 | 6430 88 | 28 27 00 | 0.688194444 | 1 | 1 | 599 06 00 | 627 33 00 | 34 08 00 | 50 78 |
| X | 25 | 3314 52 00 | 19 48 | 0.688194444 | 1 | 1 | 281 31 00 | 261 83 | 0.641666667 | 4695 |
| X | 32 | 470799 | 12 15 | 0.688194444 | 1 | 1 | 231 92 | 219 77 | 0.793055556 | 480200 |
| X | 39 | 652665 | 11 48 | 0.688194444 | 1 | 1 | 142 15 00 | 153 63 | 24 16 00 | 0.759722222 |
| X | 47 | 8680 47 00 | 11 26 | 0.688194444 | 1 | 1 | 445 82 | 434 56 00 | 29 94 | 17 27 |
| X | 22 | 3215 48 00 | 0,436111111 | 0.688194444 | 1 | 1 | 98 67 | 88 79 | 0,420833333 | 0,716666667 |
| X | 54 | 9129 12 00 | 849 | 0,688194444 | 1 | 1 | 1251 14 00 | 124265 | 503 | 4299 |
| X | 30 | 44024500 | 7 58 | 0,688194444 | 1 | 1 | 74 15 00 | 66 57 00 | 7 38 | 1,017361111 |
| X | 58 | 9418 02 00 | 0.30625 | 0,688194444 | 1 | 1 | 8454500 | 83864 | 13 46 | 49 31 00 |
| X | 53 | 8913 25 00 | 632 | 0,688194444 | 1 | 1 | 355 23 00 | 348 91 | 0,629861111 | 14 01 |
| X | 28 | 40902500 | 602 | 0.688194444 | 1 | 1 | 45 08 00 | 39 06 00 | 0.065972222 | 604 |
| X | 45 | 8560 83 | 601 | 0.688194444 | 1 | 1 | 54 49 00 | 48 39 00 | 804 | 603 |
| X | 31 | 4568 57 00 | 0,261111111 | 0.688194444 | 1 | 1 | 161 68 | 155 91 | 15 27 | 31 34 00 |
| X | 57 | 9379 04 00 | 524 | 0.688194444 | 1 | 1 | 429 26 00 | 424 02 00 | 17 01 | 29 81 |
| X | 23 | 3262 91 | 0,23125 | 0.688194444 | 1 | 1 | 45 84 | 40 91 | 0.107638889 | 7 54 |
| X | 8 | 1014 02 00 | 0.224305556 | 0.688194444 | 1 | 1 | 179 08 00 | 174 97 | 26 01 00 | 403400 |
| X | 33 | 506499 | 0,191666667 | 0.688194444 | 1 | 1 | 9378 | 9774 | 0,44375 | 1042 |
| X | 29 | 420889 | 0,186111111 | 0.688194444 | 1 | 1 | 233 77 | 237 64 | 1,004166667 | 3 51 |
| X | 55 | 92850300 | 3 51 | 0,688194444 | 1 | 1 | 185 58 00 | 1820700 | 0,510416667 | 15 09 |
| X | 46 | 8577 65 | 304 | 0,688194444 | 1 | 1 | 37 63 | 34 22 00 | 5 46 | 303 |
| X | 24 | 3279 68 | 0,1375 | 0,688194444 | 1 | 1 | 1,020138889 | 21 11 | 1 25 | 0,136805556 |
| X | 48 | 87203400 | 0,1375 | 0,688194444 | 1 | 1 | 904500 | 87 67 | 0,470138889 | 0,170138889 |
| X | 56 | 9306 73 | 247 | 0,688194444 | 1 | 1 | 1000900 | 98 43 00 | 0,29375 | 7 08 |
| X | 34 | 575083 | 236 | 0,688194444 | 1 | 1 | 490400 | 51 39 00 | 0,142361111 | 1 36 |
| X | 12 | 1204 54 00 | 2 19 | 0,688194444 | 1 | 1 | 0,960416667 | 2043 | 0.056944444 | 0.149305556 |
| X | 49 | 8735 82 | 2 01 | 0,688194444 | 1 | 1 | 10688 | 104 78 | 12 46 | 0,23125 |
| X | 21 | 3191 94 | 0.107638889 | 0.688194444 | 1 | 1 | 2316 | 21 21 | 1 32 | 0,127083333 |
| X | 37 | 6342 05 00 | 0,102777778 | 0,688194444 | 1 | 1 | 27 68 | 29 56 00 | 12 24 | 5 41 |
| X | 27 | 3950 09 00 | 0,093055556 | 0,688194444 | 1 | 1 | 80 56 00 | 82 03 00 | 0,175694444 | 218 |
| X | 41 | 6777 04 00 | 0,088194444 | 0,688194444 | 1 | 1 | 85 96 | 84 29 00 | 804 | 7 46 |
| X | 26 | 3363 92 | 1 45 | 0,688194444 | 1 | 1 | 19 11 | 0,754861111 1 | 1 52 | 306 |
| X | | 103967 | 1 04 | 0,688194444 | 1 | 1 | 12865 | 1272500 | 0,584722222 | 340700 |
| X | 7 | 98792 | 1 38 | 0,688194444 | 1 | 1 | 128 31 00 | 12692 | 1609 | 395500 |
| X | 59 | 96660900 | 1 19 | 0,688194444 | 1 | 1 | 28 22 00 | 27 02 00 | 0,061111111 | 4 53 |
| X | 44 | 820296 | 1 12 | 0,688194444 | 1 | 1 | 444200 | 430300 | 508 | 4 18 |
| X | 17 | 2103 89 | 0,067361111 | 0,688194444 | 1 | 1 | 949 | 8 52 | 042 | 2 08 |
| X | 3 | 8523200 | 0,067361111 | 0,688194444 | 1 | 1 | 14 33 | 13 36 | 0,064583333 | 1 47 |
| X | 6 | 960 16 00 | 0,065972222 | 0,688194444 | 1 | 1 | 0,588194444 | 0,626388889 | 2 27 | 553 |
| X | 50 | 8806 94 | 0,064583333 | 0.688194444 | 1 | 1 | 559 28 00 | 560 22 00 | 7 12 2 | 7 33 |
| X | 43 | 7762 27 00 | 009 | 0,688194444 | 1 | 1 | 24 31 00 | 25 21 00 | 0.085416667 | 1 04 |
| X | 20 | 2932 68 | 009 | 0,688194444 | 1 | 1 | 0,515972222 | 0,480555556 | 007 | 1 39 |
| X | 5 | 920 09 00 | 0,059027778 | 0.688194444 | 1 | 1 | 0,429861111 | 0.398611111 | 031 | 007 |
| X | 4 | 90291 | 0,058333333 | 0,688194444 | 1 | 1 | 1605 | 15 21 | 0.064583333 | 0.045833333 |
| X | 1 | 8092500 | 0,045138889 | 0.688194444 | 1 | 1 | 7 19 | 0,35 | 0 34 | 2 51 |
| X | 51 | 8848 07 00 | 0,042361111 | 0,688194444 | 1 | 1 | 10692 | 10631 00 | 0,354861111 | 0.110416667 |
| X | 15 | 1464 86 | 056 | 0,688194444 | 1 | 1 | 2226 | 21 07 | 0.105555556 | 4 33 |
| X | 62 | 10441 65 | 055 | 0,688194444 | 1 | 1 | 0,342361111 | 7 18 | 027 | 1 34 |
| X | 36 | 630086 | 0 52 | 0,688194444 | 1 | 1 | 23 13 | 1,003472222 | 12 04 | 747 |
| X | 11 | 108595 | 051 | 0,688194444 | 1 | 1 | 0,605555556 | 13 41 | 0,096527778 | 0,107638889 |
| X | 19 | 2791 01 00 | 051 | 0,688194444 | 1 | 1 | 11 03 | 11 08 | 1 04 | 1 14 |
| X | 14 | 125274 | 049 | 0,688194444 | 1 | 1 | 12 04 | 12 53 | 032 | 1 01 |
| X | 10 | 1062 13 00 | 044 | 0,688194444 | 1 | 1 | 54 68 | 55 12 00 | 655 | 0.477083333 |
| X | 61 | 9931 04 00 | 0 42 | 0,688194444 | 1 | 1 | 0.69375 | 16 04 | 0 58 | 1 06 |
| X | 42 | 76680900 | 037 | 0,688194444 | 1 | 1 | 38 86 | 39 23 00 | 0,139583333 | 2 29 |
| X | 35 | 590275 | 031 | 0,688194444 | 1 | 1 | 17 36 | 17 05 | 328 | 0,099305556 |
| X | 16 | 1473 88 | 028 | 0,688194444 | 1 | 1 | 821 | 848 | 041 | 1 19 |
| X | 18 | 23785900 | 025 | 0,688194444 | 1 | 1 | 20 36 | 0,875694444 | 027 | 0,085416667 |
| X | 13 | 12272600 | 023 | 0,688194444 | 1 | 1 | 843 | 821 | 047 | 206 |
| X | 2 | 824 86 | 002 | 0,688194444 | 1 | 1 | 8 32 | 8 12 | 037 | 0,105555556 |
| X | 52 | 8864 52 00 | 019 | 0.69375 | 1 | 1 | 232 94 | 232 75 | 15 52 | 0,252083333 |
| X | 60 | 9707 43 00 | 001 | 0,69375 | 1 | 1 | 740600 | 740500 | 1 19 9 | 0,472916667 |

**Table 5C**

| | | | | | | | | | | |
|---|---|---|---|---|---|---|---|---|---|---|
| **ClinProt Peak Statistic** | | | | | | | | | | |
| ClinProTools Version 20 build 365 | | | | | | | | | | |
| Number of peaks 56 | | | | | | | | | | |
| Sort Mode p value tta | | | | | | | | | | |

| S | Index | Mass | DAve | PTTA | PWKW | PAD | Ave1 1 | Ave2 | StdDev1 | StdDev2 |
|---|---|---|---|---|---|---|---|---|---|---|
| X | 48 | 908694 | 9998 | 0,228472222 | 035 | 1 | 63371 | 73368 | 13 49 | 5293 |
| X | 44 | 8751 33 00 | 81 5600 | 0,228472222 | 0 35 | 1 | 448 25 00 | 529 81 | 28 07 00 | 33 72 |
| X | 8 | 1013 97 | 77 08 00 | 0,228472222 | 035 | 1 | 26299 | 185 19 00 | 9 | 45 01 00 |
| X | 7 | 9882100 | 7666 | 0,228472222 | 035 | 1 | 2640400 | 187 74 | 16 15 | 3567 |
| X | 42 | 859496 | 7369 | 0,228472222 | 0,324305556 | 1 | 182 22 00 | 25591 | 291800 | 4292 |
| X | 9 | 1040 12 00 | 502300 | 0,228472222 | 035 | 1 | 118 65 | 68 42 00 | 1037 | 280800 |
| X | 47 | 89110700 | 344900 | 0,228472222 | 035 | 1 | 18592 | 220 41 00 | 851 | 15 56 |
| X | 29 | 45423500 | 29 53 00 | 0,228472222 | 035 | 1 | 9085 | 1203800 | 9 01 | 15 02 |
| X | 28 | 43762500 | 24 52 00 | 0,228472222 | 035 | 1 | 107 96 | 1324800 | 449 | 707 |
| X | 46 | 8865 15 00 | 0.94375 | 0,228472222 | 035 | 1 | 97 01 00 | 119 09 00 | 502 | 1223 |
| X | 26 | 40894600 | 0,436805556 | 0,228472222 | 035 | 1 | 46 13 00 | 56 01 00 | 326 | 427 |
| X | 25 | 4053 27 00 | 7 58 | 0,228472222 | 0 35 | 1 | 47 39 00 | 54 96 | 4 06 | 1 07 |
| X | 6 | 97465 | 0,224305556 | 0,228472222 | 0,324305556 | 1 | 14 33 | 905 | 257 | 0.128472222 |
| X | 1 | 852 09 00 | 2 09 | 0.228472222 | 0 35 | 1 | 0,608333333 | 11 06 | 0,058333333 | 0,068055556 |
| X | 21 | 210371 | 2 12 | 0,228472222 | 035 | 1 | 804 | 10 16 | 044 | 1 03 |
| X | 2 | 871 14 00 | 0,060416667 | 0,228472222 | 035 | 1 | 307 | 457 | 043 | 022 |
| X | 5 | 96062 | 0,214583333 | 034 | 0,324305556 | 1 | 0,729861111 | 1222 | 0,146527778 | 249 |
| X | 43 | 8681 04 00 | 2966 | 0,2375 | 0,324305556 | 1 | 14289 | 1725500 | 9 17 | 20 19 |
| X | 50 | 9281 87 | 20 39 | 0.257638889 | 0,324305556 | 1 | 475 69 | 496 08 00 | 0,469444444 | 14 34 |
| X | 45 | 8804 42 00 | 2006 | 0,263194444 | 0.324305556 | 1 | 16891 | 189 51 00 | 4 17 | 15 02 |
| X | 3 | 899 15 00 | 0,051388889 | 0.3 | 0 35 | 1 | 11 35 | 1209 | 0 59 | 0 02 |
| X | 30 | 456589 | 26 51 00 | 0,304166667 | 0,502083333 | 1 | 157 61 | 184 12 00 | 1008 | 1.015277778 |
| X | 10 | 1203 03 00 | 0,210416667 | 0,304166667 | 0,324305556 | 1 | 2036 | 0,675694444 | 246 | 423 |
| X | 55 | 992785 | 222 | 0,304166667 | 0,502083333 | 1 | 23 46 | 2124 | 0,065972222 | 206 |
| X | 4 | 921 04 00 | 0,066666667 | 0,304166667 | 0 35 | 1 | 7 56 | 8 51 | 0,058333333 | 0 01 |
| X | 22 | 3215 05 00 | 343 | 045 | 0,324305556 | 1 | 14 06 | 18 03 | 0,086805556 | 3 32 |
| X | 32 | 506366 | 4 15 | 0,313888889 | 0,591666667 | 1 | 63 06 00 | 67 75 | 0 38 | 4 06 |
| X | 23 | 3313 87 | 0,508333333 | 0,3222222222 | 0,591666667 | 1 | 81 37 00 | 93 08 00 | 302 | 1214 |
| X | 36 | 642987 | 1312 | 006 | 0,502083333 | 1 | 318 41 00 | 3052900 | 759 | 1717 |
| X | 12 | 147075 | 1 38 | 006 | 0,591666667 | 1 | 8 12 | 905 | 0,099305556 | 045 |
| X | 27 | 42081900 | 18 17 | 0,434722222 | 0,502083333 | 1 | 38261 | 40078 | 15 18 | 250400 |
| X | 33 | 5750 21 00 | 3 16 | 0,434722222 | 0.591666667 | 1 | 58 61 | 61 77 | 1 38 | 0.21875 |
| X | 20 | 20522400 | 247 | 0,434722222 | 0.502083333 | 1 | 12 46 | 0,647916667 | 3 17 | 2 45 |
| X | 56 | 1172203 | 0.1 | 0,450694444 | 0.591666667 | 1 | 30 19 00 | 320300 | 207 | 259 |
| X | 54 | 9704 36 00 | 443 | 0,488194444 | 0.502083333 | 1 | 87 01 00 | 82 58 00 | 4 46 | 705 |
| X | 53 | 966274 | 1 27 | 0,492361111 | 0.591666667 | 1 | 32 21 00 | 30 95 | 1 02 | 2 32 |
| X | 24 | 3950 16 00 | 0,133333333 | 0,051388889 | 0,591666667 | 1 | 340400 | 37 11 00 | 351 | 5 |
| X | 38 | 662768 | 4575 | 0,553472222 | 1 | 1 | 1421 11 00 | 1375 36 00 | 4467 | 104 92 |
| X | 31 | 47060700 | 0,30625 | 0,553472222 | 0,591666667 | 1 | 174 78 | 181 59 00 | 3 15 | 0,733333333 |
| X | 40 | 766668 | 3 | 0,553472222 | 0,591666667 | 1 | 33 89 | 3089 | 0,30625 | 0,220833333 |
| X | 52 | 9415 34 00 | 250300 | 0,565277778 | 0,591666667 | 1 | 976 16 00 | 950 86 | 4673 | 5968 |
| X | 19 | 2021 45 00 | 0,218055556 | 0,5777777778 | 0,502083333 | 1 | 106 36 00 | 111 09 00 | 1201 | 10 15 |
| X | 37 | 6525 48 00 | 403 | 0,577777778 | 0,591666667 | 1 | 100 16 00 | 104 02 00 | 0,545833333 | 2 24 |
| X | 35 | 5901 84 | 5 46 | 0,059722222 | 0,591666667 | 1 | 672700 | 61 81 | 0,151388889 | 0,850694444 |
| X | 18 | 1735 68 | 036 | 0,059722222 | 1 | 1 | 1055 | 10 19 | 1 28 | 055 |
| X | 13 | 1515 13 00 | 0,052083333 | 0,656944444 | 1 | 1 | 13 17 | 1243 | 0.174305556 | 1 09 |
| X | 49 | 912663 | 8 36 | 0,676388889 | 0,591666667 | 1 | 1361 05 00 | 1369 41 00 | 67 54 00 | 8978 |
| X | 39 | 677386 | 007 | 0,676388889 | 1 | 1 | 390600 | 3889 | 0,261111111 | 0,110416667 |
| X | 15 | 160293 | 039 | 0.676388889 | 1 | 1 | 10 15 | 1054 | 3 13 | 009 |
| X | 16 | 1646 06 00 | 0 37 | 0,676388889 | 0,591666667 | 1 | 0,438888889 | 1029 | 2 43 | 0 42 |
| X | 14 | 1558 92 | 022 | 0.676388889 | 1 | 1 | 0,442361111 | 0,427083333 | 2 42 | 1 19 |
| X | 17 | 1691 66 | 014 | 0.676388889 | 1 | 1 | 949 | 935 | 1 55 | 0,050694444 |
| X | 34 | 586281 | 021 | 0,682638889 | 0,591666667 | 1 | 28 97 | 28 76 | 0,090972222 | 0,186111111 |
| X | 41 | 7761 24 00 | 0 15 | 0,682638889 | 1 | 1 | 600600 | 6075 | 204 | 0,145138889 |
| X | 11 | 142678 | 002 | 1 | 1 | 1 | 902 | 9 18 | 249 | 0,065277778 |
| X | 51 | 93760700 | 001 | 1 | 1 | 1 | 510 08 00 | 510 08 00 | 18 17 7 | 27 06 00 |

**Table 5D**

| | | | | | | | | | | |
|---|---|---|---|---|---|---|---|---|---|---|
| **ClinProt Peak Statistic** | | | | | | | | | | |
| ClinProTools Version 20 build 365 | | | | | | | | | | |
| Number of peaks 61 | | | | | | | | | | |
| Sort Mode p value tta | | | | | | | | | | |

| S | Index | Mass | DAve | PTTA | PWKW | PAD | Ave1 | Ave2 | StdDev1 | StdDev2 |
|---|---|---|---|---|---|---|---|---|---|---|
| X | 58 | 56922500 | 11385 | 0.259722222 | 0.413194444 | 1 | 370 67 | 256 82 | 0.671527778 | 107 64 |
| X | 59 | 6631 42 00 | 69 09 00 | 0.259722222 | 0.385416667 | 1 | 114 08 00 | 44 09 00 | 37 66 | 36 02 00 |
| X | 4 | 10132600 | 680700 | 0.259722222 | 0.352777778 | 1 | 142 52 00 | 73 81 | 11 06 | 4995 |
| X | 5 | 10375700 | 59 | 0.259722222 | 0.352777778 | 1 | 117 39 00 | 583900 | 0.472916667 | 3877 |
| X | 3 | 98785 | 54 09 00 | 0.259722222 | 0.385416667 | 1 | 98 04 00 | 43 95 | 18 13 | 46 92 |
| X | 34 | 316574 | 532800 | 0.259722222 | 0.352777778 | 1 | 4524300 | 50571 | 1808 | 31 93 |
| X | 46 | 39524700 | 4379 | 0.259722222 | 0.385416667 | 1 | 329 | 372 79 | 0.263194444 | 39 77 |
| X | 43 | 3453 99 | 4378 | 0.259722222 | 0.385416667 | 1 | 39367 | 4374500 | 0.474305556 | 3099 |
| X | 55 | 50670800 | 3861 | 0.259722222 | 0.385416667 | 1 | 154 12 00 | 115 51 00 | 2 44 | 31 51 00 |
| X | 57 | 5635 94 | 33 44 00 | 0.259722222 | 0.413194444 | 1 | 90 07 00 | 57 26 00 | 0.359027778 | 32 57 00 |
| X | 49 | 4091 82 | 250300 | 0.259722222 | 0.413194444 | 1 | 334 15 00 | 308 85 | 6 23 | 23 52 |
| X | 35 | 32640200 | 2331 | 0.259722222 | 0.413194444 | 1 | 875700 | 11088 | 211 | 21 26 |
| X | 42 | 3439 75 | 1652 | 0.259722222 | 0.502777778 | 1 | 113 71 | 130 24 00 | 0.1 | 16 57 |
| X | 28 | 29030400 | 1602 | 0.259722222 | 0.352777778 | 1 | 223 59 00 | 23961 | 951 | 353 |
| X | 20 | 207394 | 0.678472222 | 0.259722222 | 0.352777778 | 1 | 157 08 00 | 172 85 | 0.045138889 | 9 07 |
| X | 41 | 341368 | 0.668055556 | 0.259722222 | 0.502777778 | 1 | 150 16 00 | 165 78 | 2 58 | 16 01 |
| X | 17 | 1725 49 00 | 0.564583333 | 0.259722222 | 0.352777778 | 1 | 29 67 | 42 59 00 | 0.063194444 | 9 33 |
| X | 30 | 3002 18 00 | 1209 | 0.259722222 | 0.502777778 | 1 | 403700 | 532700 | 0.063194444 | 1304 |
| X | 6 | 1060 35 00 | 0.554861111 | 0.259722222 | 0.413194444 | 1 | 31 09 00 | 19 11 | 2 34 | 10 25 |
| X | 29 | 2949 93 | 0.549305556 | 0.259722222 | 0.413194444 | 1 | 44 65 | 57 36 00 | 0.133333333 | 11 26 |
| X | 56 | 553674 | 1236 | 0.259722222 | 0.413194444 | 1 | 28 17 00 | 0.68125 | 2 05 | 11 31 |
| X | 54 | 4964 78 | 0.523611111 | 0.259722222 | 0.385416667 | 1 | 39 09 00 | 51 84 | 0.090972222 | 10 42 |
| X | 9 | 128874 | 0.514583333 | 0.259722222 | 0.352777778 | 1 | 31 79 | 43 61 | 0.214583333 | 6 09 |
| X | 45 | 3743 25 00 | 11 49 | 0.259722222 | 0.413194444 | 1 | 39 87 | 51 37 00 | 2 33 | 11 44 |
| X | 31 | 3056 24 00 | 0.475694444 | 0.259722222 | 0.385416667 | 1 | 1824 | 29 01 00 | 4 09 | 9 07 |
| X | 48 | 3993 26 00 | 0.420833333 | 0.259722222 | 0.385416667 | 1 | 152 65 | 142 99 | 5 22 | 5 14 |
| X | 16 | 158273 | 919 | 0.259722222 | 0.502777778 | 1 | 22 18 | 31 37 00 | 1 26 | 9 16 |
| X | 12 | 1398 13 00 | 8 09 | 0.259722222 | 0.352777778 | 1 | 0.720138889 | 25 67 | 0.085416667 | 0.272222222 |
| X | 44 | 3606 54 00 | 0.391666667 | 0.259722222 | 0.413194444 | 1 | 24 07 00 | 33 54 00 | 0.147222222 | 8 34 |
| X | 18 | 197568 | 8 52 | 0.259722222 | 0.352777778 | 1 | 16 45 | 24 97 | 0 19 | 6 06 |
| X | 38 | 335862 | 844 | 0.259722222 | 0.413194444 | 1 | 49 66 | 58 01 00 | 2 23 | 0.342361111 |
| X | 24 | 276987 | 809 | 0.259722222 | 0.413194444 | 1 | 74 35 00 | 82 44 00 | 5 01 | 8 02 |
| X | 21 | 237971 | 8 03 | 0.259722222 | 0.385416667 | 1 | 59 62 | 67 65 | 0.225694444 | 5 09 |
| X | 50 | 4111 71 | 0.34375 | 0.259722222 | 0.352777778 | 1 | 111 32 00 | 103 58 00 | 1 23 | 3 02 |
| X | 39 | 338099 | 507 | 0.259722222 | 0.385416667 | 1 | 25 03 00 | 31 | 1 23 | 5 22 |
| X | 2 | 85963 | 5 14 | 0.259722222 | 0.352777778 | 1 | 17 18 | 22 31 | 0 34 | 3 57 |
| X | 36 | 331768 | 0.209722222 | 0.259722222 | 0.352777778 | 1 | 33 59 00 | 28 97 | 4 14 | 0 59 |
| X | 33 | 3123 92 | 0.184027778 | 0.259722222 | 0.413194444 | 1 | 26 | 29 84 | 1 09 | 0.189583333 |
| X | 61 | 7997 94 | 0.546527778 | 0.265972222 | 0.413194444 | 1 | 26 | 13 33 | 5 07 | 0.5875 |
| X | 22 | 2496 21 00 | 11 28 | 0.265972222 | 0.502777778 | 1 | 27 48 00 | 38 75 | 2 23 | 12 03 |
| X | 51 | 41482500 | 3287 | 0.288194444 | 0.413194444 | 1 | 936 98 | 969 85 | 37 87 | 0.938888889 |
| X | 7 | 1064 29 00 | 11 34 | 0.288888889 | 0.502777778 | 1 | 48 37 00 | 37 03 00 | 1 58 | 0.605555556 |
| X | 60 | 7941 68 | 4 07 | 0.288888889 | 0.413194444 | 1 | 9 27 | 4 57 | 2 22 | 0.25625 |
| X | 15 | 1561 91 | 1925 | 0.301388889 | 0.502777778 | 1 | 113 11 00 | 132 36 00 | 0.345138889 | 25 |
| X | 11 | 1389 88 | 6 17 | 0.307638889 | 0.502777778 | 1 | 20 07 | 26 87 | 0.100694444 | 8 31 |
| X | 14 | 1518 43 00 | 0.215972222 | 0.313888889 | 0.413194444 | 1 | 24 07 00 | 28 77 | 3 21 | 6 43 |
| X | 25 | 279785 | 1308 | 0.321527778 | 0.502777778 | 1 | 303 85 | 317 65 | 18 08 | 14 59 |
| X | 47 | 3971 08 00 | 5 59 | 0.321527778 | 0.502777778 | 1 | 66 26 00 | 71 85 | 0.129166667 | 8 21 |
| X | 27 | 2846 42 00 | 12 55 | 0.322916667 | 0.502777778 | 1 | 185 83 | 173 29 00 | 0.433333333 | 0.803472222 |
| X | 53 | 43000200 | 0.466666667 | 0.322916667 | 0.502777778 | 1 | 69 57 00 | 5885 | 0.71875 | 713 |
| X | 26 | 2813 24 00 | 0.313888889 | 0.322916667 | 0.502777778 | 1 | 128 59 00 | 135 51 00 | 0.473611111 | 0.096527778 |
| X | 32 | 3109 23 00 | 0.095138889 | 0.322916667 | 0.413194444 | 1 | 65 93 | 67 69 | 2 56 | 1 09 |
| X | 13 | 1465 38 00 | 0.484722222 | 0.371527778 | 0.413194444 | 1 | 155 61 | 166 59 00 | 0.586111111 | 0.843055556 |
| X | 23 | 2741 99 | 10 16 | 0.371527778 | 0.413194444 | 1 | 98 69 | 88 53 00 | 0.641666667 | 16 08 |
| X | 1 | 8534300 | 229 | 0.371527778 | 0.502777778 | 1 | 1846 | 0.885416667 | 025 | 0.211111111 |
| X | 19 | 20450700 | 0.09375 | 0.386111111 | 0.502777778 | 1 | 31 03 00 | 29 55 00 | 2 07 | 3 22 |
| X | 40 | 3397 04 00 | 0.126388889 | 0.390277778 | 0.502777778 | 1 | 105 73 | 108 34 00 | 2 04 | 0.264583333 |
| X | 37 | 3334 25 00 | 1 02 | 0.429861111 | 1 | 1 | 25 66 | 26 69 | 2 29 | 0.106944444 |
| X | 52 | 4232 59 00 | 0.098611111 | 0.613888889 | 1 | 1 | 6081 | 5898 | 841 | 0.584722222 |
| X | 8 | 120563 | 1 24 | 0.657638889 | 0.502777778 | 1 | 600500 | 61 29 00 | 0.693055556 | 17 05 |
| X | 10 | 13505700 | 0 22 | 0.670833333 | 0.502777778 | 1 | 23 54 | 1.011111111 | 5 09 | 6 32 |

**Table 5E**

| | | | | | | | | | | |
|---|---|---|---|---|---|---|---|---|---|---|
| **ClinProt Peak Statistic** | | | | | | | | | | |
| ClinProTools Version 2 0 build 365 | | | | | | | | | | |
| Number of peaks 51 | | | | | | | | | | |
| Sort Mode p value tea | | | | | | | | | | |

| S | Index | Mass | DAve | PTTA | PWKW | PAD | Ave1 | Ave2 | StdDev1 | StdDev2 |
|---|---|---|---|---|---|---|---|---|---|---|
| X | 5 | 1865 31 00 | 1125300 | 0.60625 | 0.64375 | 1 | 1032200 | 215 75 | 1 08 | 31 59 00 |
| X | 37 | 5903 01 00 | 57 96 | 0.60625 | 1 | 1 | 728 62 | 670 66 | 43 03 00 | 144 66 |
| X | 33 | 464299 | 430200 | 0.60625 | 1 | 1 | 8362500 | 8794600 | 167 52 00 | 340300 |
| X | 49 | 92823400 | 3669 | 0.60625 | 1 | 1 | 122371 | 1187 02 00 | 8874 | 55 15 00 |
| X | 38 | 5918 23 00 | 272800 | 0.60625 | 0.64375 | 1 | 34361 | 3163300 | 3679 | 0.813194444 |
| X | 6 | 1897 39 00 | 2225 | 0.60625 | 0.64375 | 1 | 3967 | 61 92 | 649 | 28 51 00 |
| X | 40 | 6087 15 00 | 2202 | 0.60625 | 0.64375 | 1 | 9984 | 77 82 | 14 04 | 21 19 |
| X | 39 | 5961 1800 | 2143 | 0.60625 | 0.64375 | 1 | 90 11 00 | 6868 | 31 55 00 | 0.389583333 |
| X | 29 | 3813 37 00 | 21 17 | 0.60625 | 0.64375 | 1 | 123 34 00 | 102 17 00 | 13 11 | 847 |
| X | 42 | 77622900 | 0.811111111 | 0.60625 | 1 | 1 | 134 37 00 | 153 26 00 | 0.552777778 | 484600 |
| X | 10 | 2209 66 | 11 12 | 0.60625 | 0.64375 | 1 | 30 25 00 | 41 37 00 | 3 02 | 0.271527778 |
| X | 22 | 3191 87 | 1039 | 0.60625 | 1 | 1 | 1520300 | 141 91 | 0.761805556 | 281800 |
| X | 30 | 3881 46 00 | 9 52 | 0.60625 | 1 | 1 | 520900 | 61 62 | 545 | 21 52 |
| X | 21 | 3158 58 00 | 902 | 0.60625 | 0.64375 | 1 | 117 44 00 | 108 43 00 | 11 13 | 0.919444444 |
| X | 3 | 1740 35 00 | 0.376388889 | 0.60625 | 0.64375 | 1 | 99 18 00 | 90 56 00 | 5 27 | 0.461805556 |
| X | 48 | 9171 27 00 | 8 53 | 0.60625 | 0.64375 | 1 | 35 45 00 | 43 98 | 13 29 | 0.054166667 |
| X | 25 | 3261 68 | 0.349305556 | 0.60625 | 1 | 1 | 2563600 | 2485300 | 1246 | 2661 |
| X | 7 | 1944 21 00 | 638 | 0.60625 | 1 | 1 | 16411 00 | 157 73 | 1003 | 0.758333333 |
| X | 4 | 177831 00 | 637 | 0.60625 | 1 | 1 | 463800 | 5275 | 0.045833333 | 749 |
| X | 27 | 352499 | 0.272916667 | 0.60625 | 0.64375 | 1 | 470800 | 5373 | 0.218055556 | 9 36 |
| X | 19 | 309479 | 0.252777778 | 0.60625 | 1 | 1 | 61 23 00 | 6687 | 2032 | 0.252777778 |
| X | 31 | 39560600 | 0.252083333 | 0.60625 | 1 | 1 | 570800 | 514500 | 272100 | 0.513194444 |
| X | 11 | 236597 | 526 | 0.60625 | 0.64375 | 1 | 363200 | 31 06 00 | 0.127083333 | 246 |
| X | 15 | 29320500 | 524 | 0.60625 | 0.64375 | 1 | 620600 | 5681 | 0.106944444 | 601 |
| X | 36 | 5335 89 | 509 | 0.60625 | 1 | 1 | 69 | 6391 | 939 | 17 16 |
| X | 18 | 302995 | 0.23125 | 0.60625 | 0.64375 | 1 | 6221 00 | 572800 | 0.231944444 | 6 11 |
| X | 8 | 2021 63 | 0.210416667 | 0.60625 | 0.64375 | 1 | 672300 | 6261 | 1235 | 903 |
| X | 17 | 2990 44 00 | 4 06 | 0.60625 | 0.64375 | 1 | 50 19 00 | 45 06 00 | 0.296527778 | 0.302083333 |
| X | 26 | 3277 04 00 | 409 | 0.60625 | 1 | 1 | 131 17 00 | 1270900 | 0.600694444 | 0.693055556 |
| X | 47 | 9055 41 00 | 401 | 0.60625 | 1 | 1 | 354300 | 394400 | 0.394444444 | 0.145138889 |
| X | 13 | 266078 | 0.186805556 | 0.60625 | 0.64375 | 1 | 500700 | 4681 | 521 | 0.175694444 |
| X | 2 | 16272400 | 0.176388889 | 0.60625 | 0.64375 | 1 | 642900 | 605500 | 0.263194444 | 551 |
| X | 35 | 4732 95 | 0.172222222 | 0.60625 | 1 | 1 | 93 17 00 | 89 05 00 | 11 36 | 6 28 |
| X | 32 | 42824600 | 359 | 0.60625 | 1 | 1 | 5787 | 542900 | 1208 | 0.293055556 |
| X | 28 | 36823200 | 325 | 0.60625 | 0.64375 | 1 | 3278 | 29 53 00 | 0.146527778 | 554 |
| X | 1 | 8594900 | 3 13 | 0.60625 | 1 | 1 | 18 34 | 2147 | 0.397916667 | 737 |
| X | 50 | 942794 | 0.148611111 | 0.60625 | 0.64375 | 1 | 5473 | 51 08 00 | 259 | 328 |
| X | 12 | 2583 51 00 | 0.130555556 | 0.60625 | 0.64375 | 1 | 62 33 00 | 59 65 | 1 07 | 0.126388889 |
| X | 9 | 2081 54 00 | 245 | 0.60625 | 1 | 1 | 10577 | 1082200 | 0.213888889 | 0.460416667 |
| X | 43 | 7825 04 00 | 0.102083333 | 0.60625 | 1 | 1 | 0.959722222 | 24 49 00 | 6 31 | 0.338194444 |
| X | 41 | 746487 | 0.086111111 | 0.60625 | 0.64375 | 1 | 1626 | 17 09 | 2 | 1 54 |
| X | 45 | 8138 09 00 | 144 | 0.60625 | 1 | 1 | 2039 | 0.932638889 | 0.098611111 | 0.269444444 |
| X | 20 | 31165400 | 1 42 | 0.60625 | 1 | 1 | 36 14 00 | 3472 | 0.100694444 | 331 |
| X | 14 | 28834200 | 1 38 | 0.60625 | 1 | 1 | 590900 | 61 29 00 | 503 | 0.129166667 |
| X | 44 | 792088 | 1 15 | 0.60625 | 1 | 1 | 0.674305556 | 0.725694444 | 2 32 | 5 07 |
| X | 16 | 2952 | 0.136805556 | 0.660416667 | 1 | 1 | 284 15 00 | 286 91 | 25 01 00 | 49 08 00 |
| X | 24 | 324074 | 203 | 0.660416667 | 1 | 1 | 157 06 00 | 155 57 00 | 0.765277778 | 240800 |
| X | 23 | 3207 46 00 | 1 02 | 0.660416667 | 1 | 1 | 790400 | 780200 | 13 04 | 0.646527778 |
| X | 51 | 1025539 | 1 06 | 0.660416667 | 1 | 1 | 3897 | 3792 | 0.255555556 | 1004 |
| X | 34 | 4717 27 00 | 002 | 0.660416667 | 1 | 1 | 31 94 | 32 14 00 | 4 17 | 302 |
| X | 46 | 88563900 | 009 | 0.660416667 | 1 | 1 | 21 08 | 0.936805556 | 1 57 | 147 |

### Reproducibility for serum proteome profiling and LMW recovery using NSPP approach comparing to Dynal Peptide Profiler RPC18 and Profiling Kit MB-HIC-8 magnetic bead-based MALDI- TOF MS

The reproducibility of three analytical approaches was evaluated by repeating 30 times the analysis of 30 different aliquots of the same serum sample for each approach.

As demonstrated in Fig. 1, Profiling Kit MB-HIC-8 approach resulted in the identification of 61 peaks with an average CV of 16.87% (Fig. 1A) compared to 64 peaks with an average CV of 7.99% for NSPP (MB-HIC8) approach (Fig. 1B). Using Profiling Kit MB-HIC-8, 3.28% of peaks had a CV> 50% and 47.50% of peaks had a CV< 10% (Fig. 1A), instead using NSPP, only 1.56% of peaks had a CV greater than 50% and 75% of peaks had a CV< 10% (Fig. 1B). Dynal Peptide Profiler RPC18 approach resulted in the identification of 56 peaks with an average CV of 9.12% (Fig. 1C) compared to 62 peaks with an average CV of 3.32% for NSPP approach (Fig. 1D). Using Dynal Peptide Profiler RPC18, 0% of peaks had a CV> 50% and 58.93% of peaks had a CV< 10% (Fig. 1C) instead using NSPP, 0% of peaks had a CV greater than 50% and 99.39% of peaks had a CV< 10% (Fig. 1D).

Comparing MALDI mass spectra profiling obtained from NSPP approach to those obtained from Profiling Kit MB-HIC-8 and Dynal Peptide Profiler RPC18 approaches, the authors showed a consistent statistical differences (via the tailed *t*-test): comparing the MALDI MS proteome profiles of serum prepared using NSPP approach to Profiling Kit MB-HIC- 8 MALDI MS profiles, 67.42% of peaks were found to have *p* < 0.01 (via the tailed *t*-test) (Fig. 2A); instead comparing NSPP profiles to Dynal Peptide Profiler RPC18 profiles 66.70% of peaks were found to have *p* < 0.01 (via the tailed *t*-test) (Fig. 2B). The use of NSPP in conjunction with MB-HIC-8 magnetic beads resulted in a gain of LMW peaks recovered in a mass range from 4,500 to 10,000 Da (Fig. 2C); while the use of NSPP approach with RPC 18 magnetic beads yielded an increased detection of LMW peaks within 2,850-10,700 Da mass range (Fig. 2D).

These data suggest that the best reproducibility and the best recovery for LMW proteome profiling was by pre-treatment of the serum using the newly described NSPP method followed by commercial magnetic bead enrichment.

### Selective efficiency of NSPP procedure for LMW proteins recovery

To evaluate the selective efficiency of LMW proteins recovery of NSPP, the authors compared tricine gel electrophoresis purification of NSPP sample to Profiling Kit MB-HIC- 8 and Dynal Peptide Profiler RPC18 sample. As shown in figure 3, pre-treatment of the sera utilizing the NSPP approach allowed a less contaminated and a better separation of LMW proteins and peptides from highly-abundant serum proteins, in particular from Albumin, Transferrin and Immunoglobulins.

The following table summarizes all the MS information (Swiss Prot Accession Number, protein description, protein score, protein mass, matched peptides, peptides expected m/z, peptides expected mr, peptides delta error, peptides miss cleavage, peptides score, peptides sequences..) of each single protein identified and reported in Figure 3.

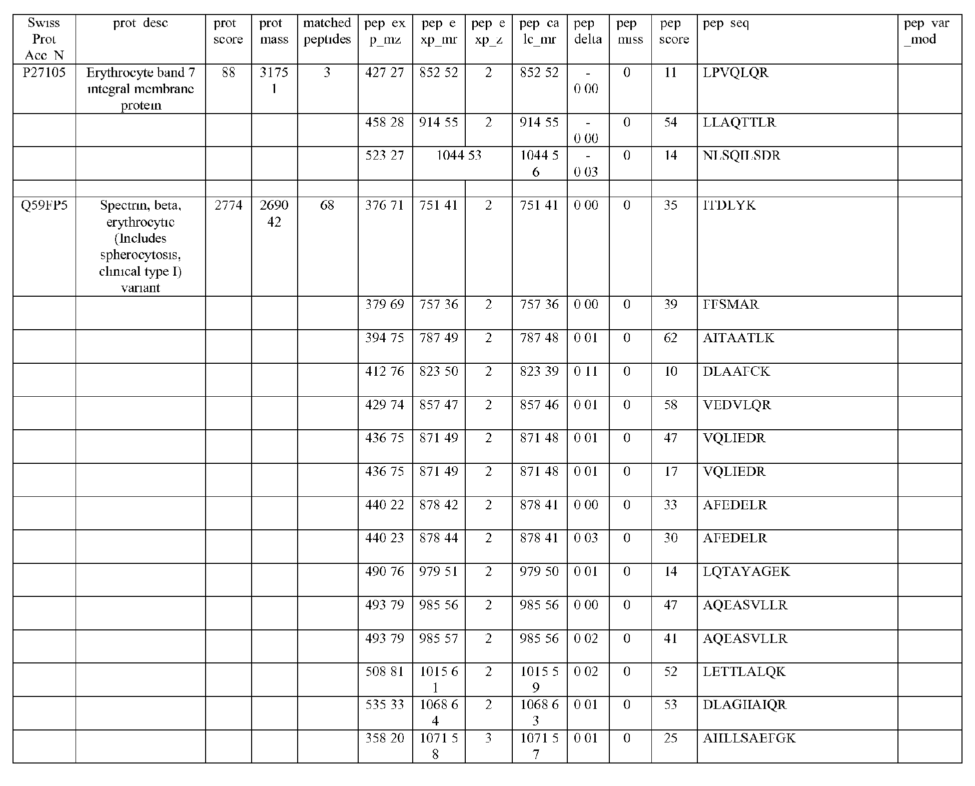

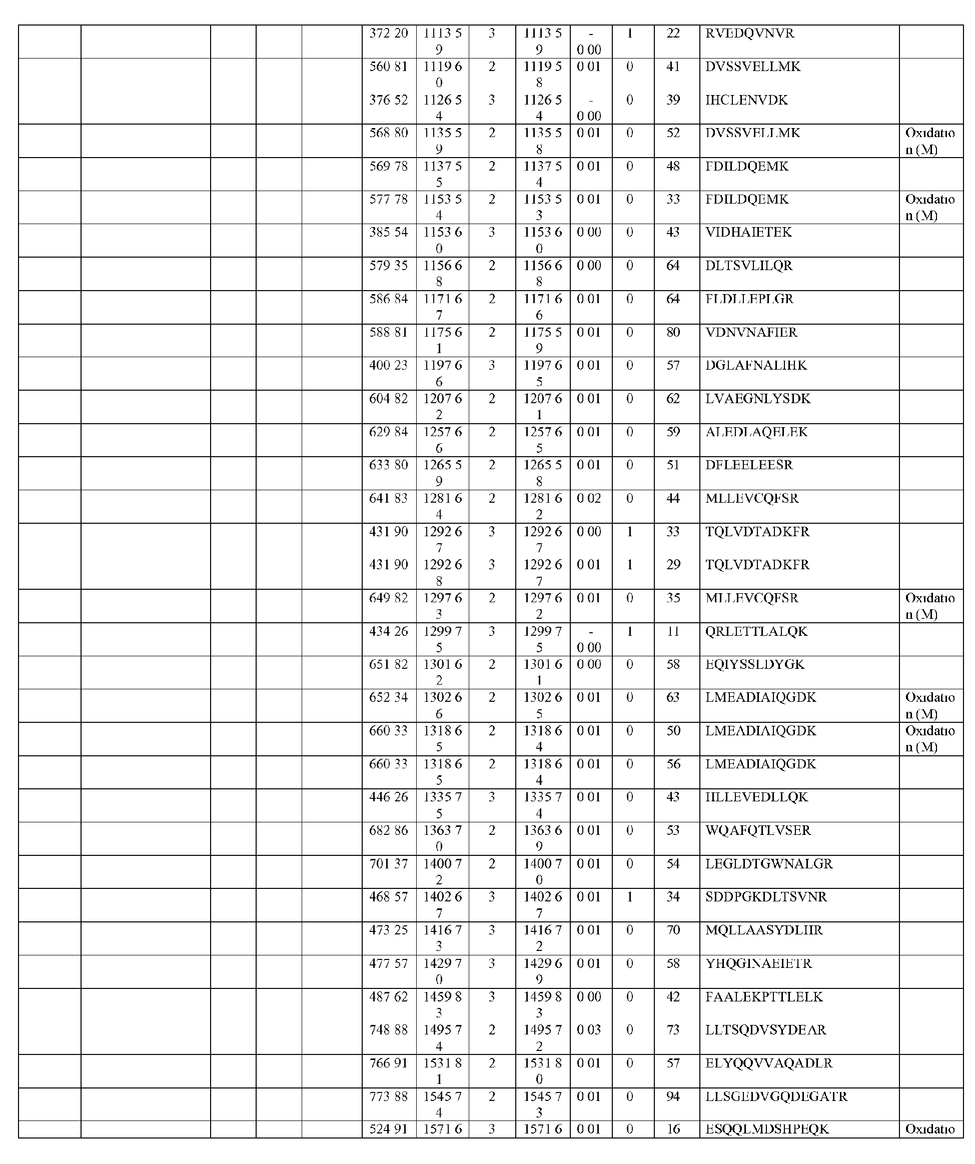

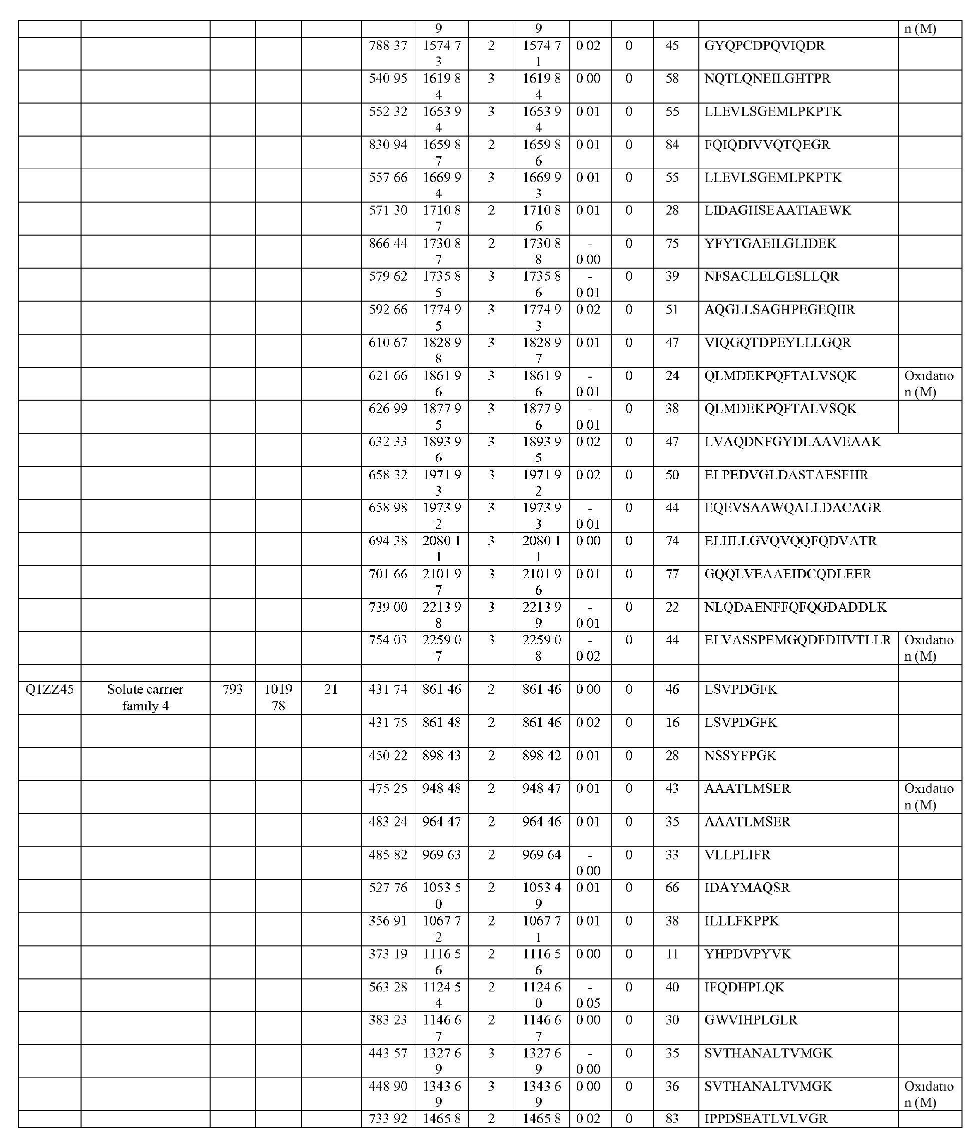

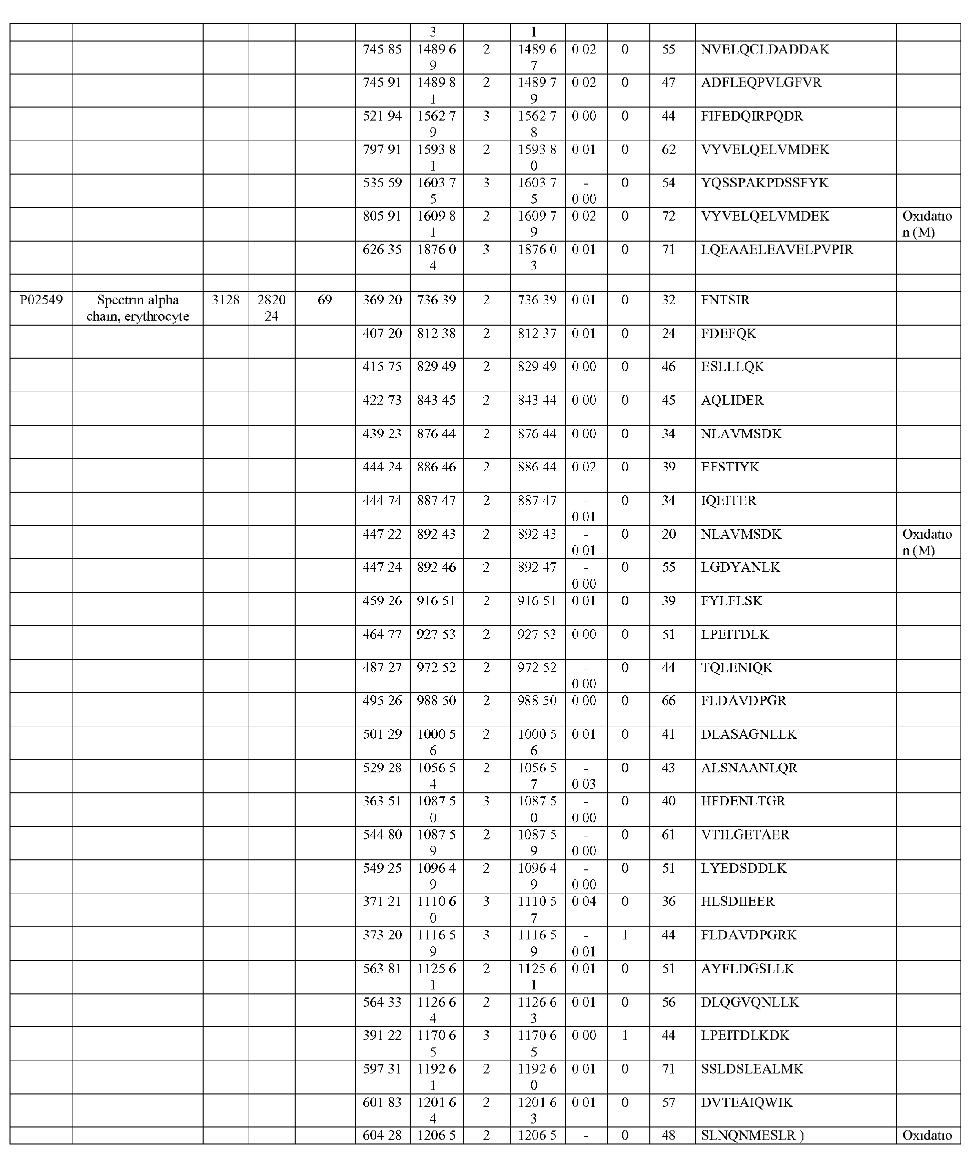

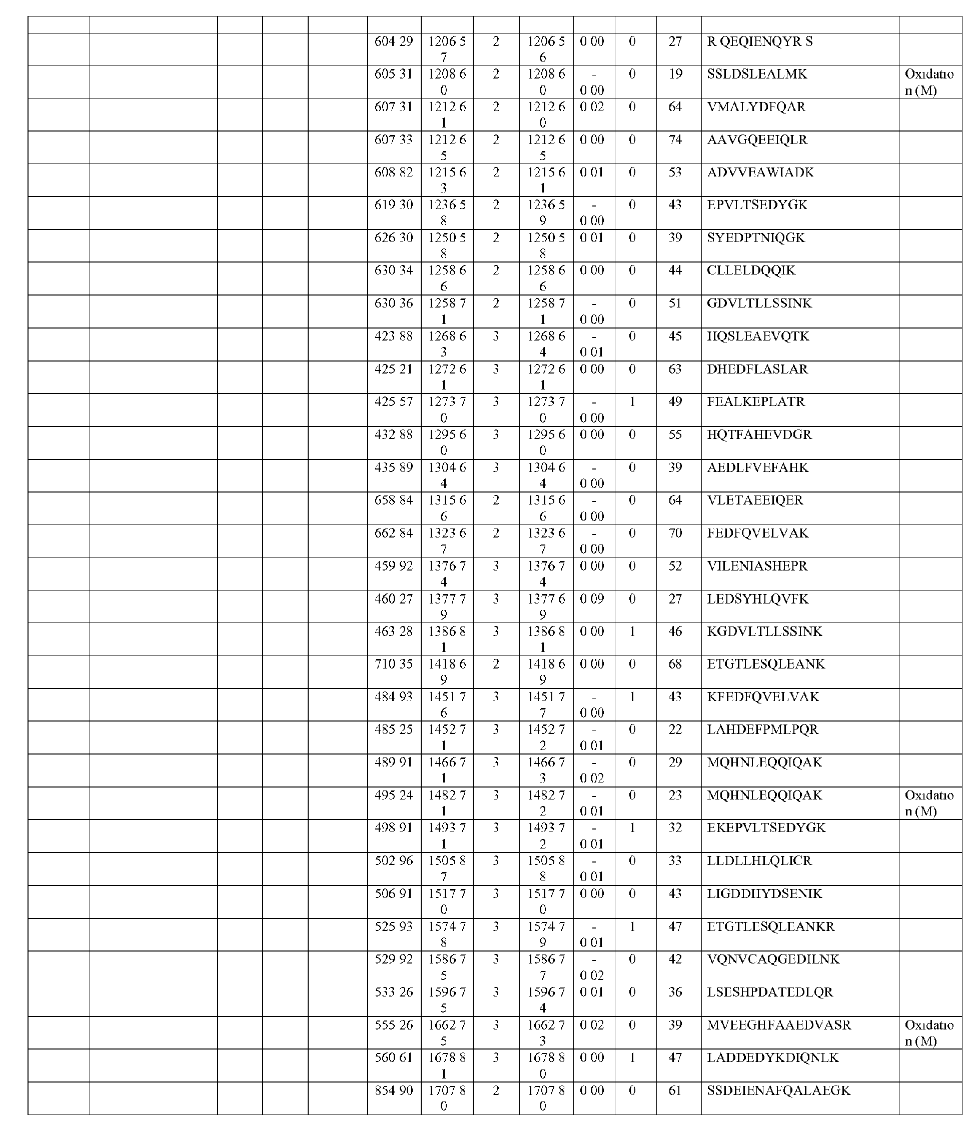

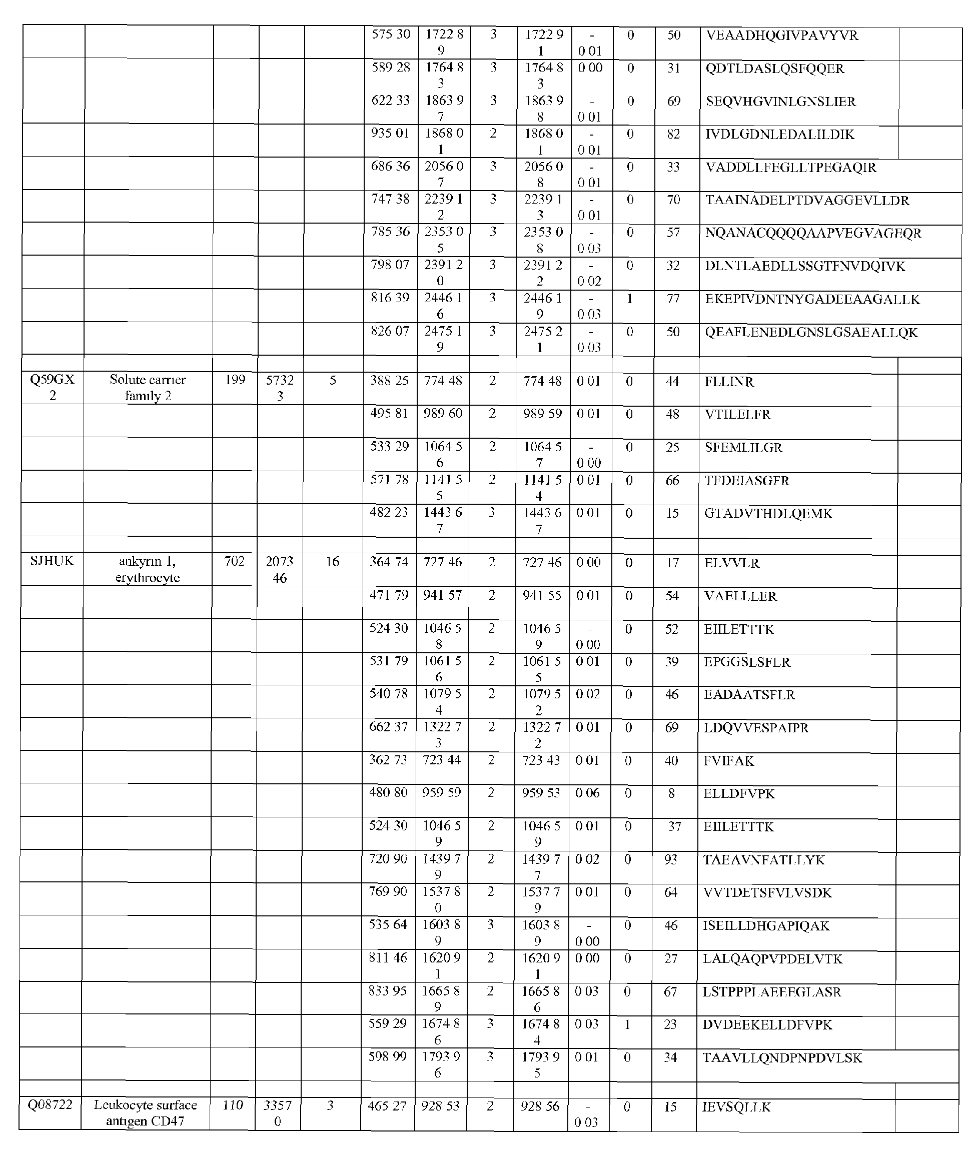

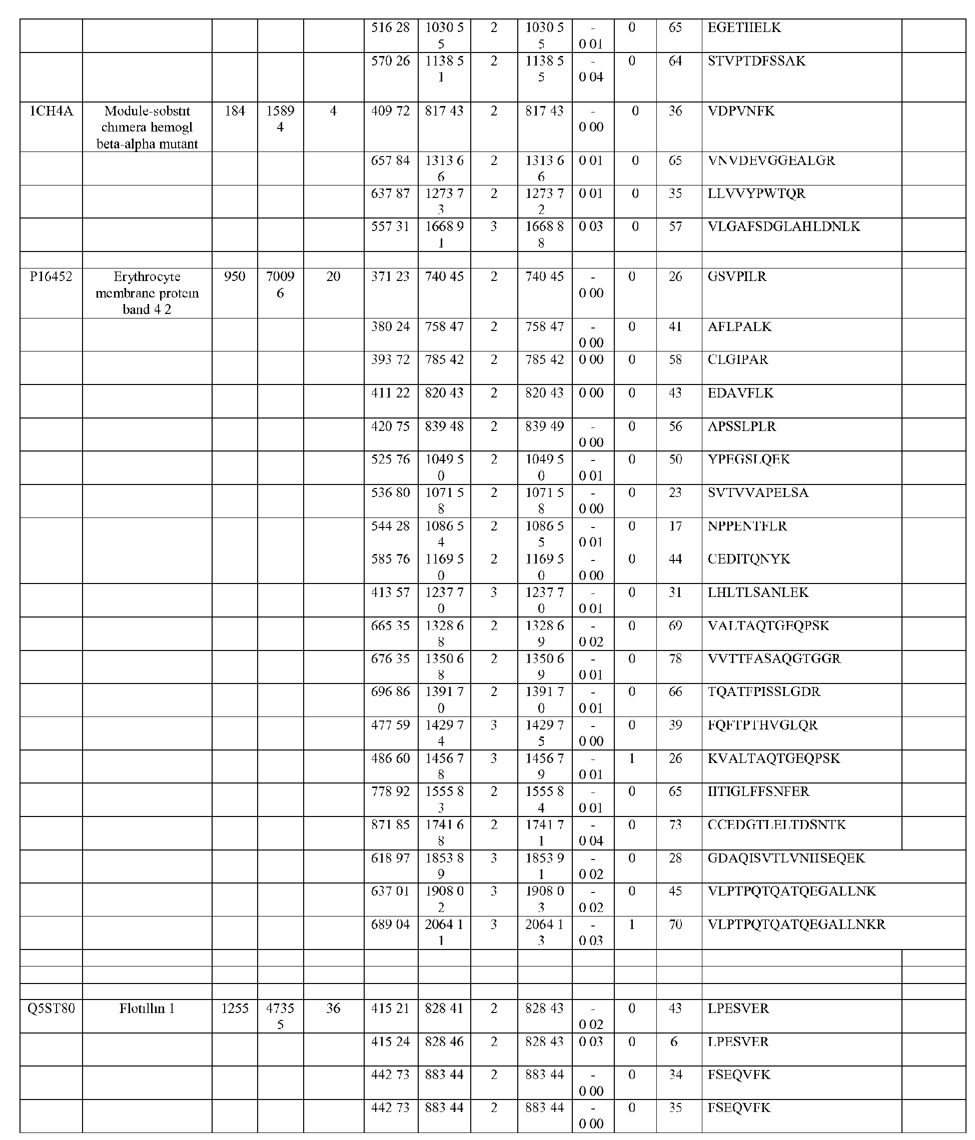

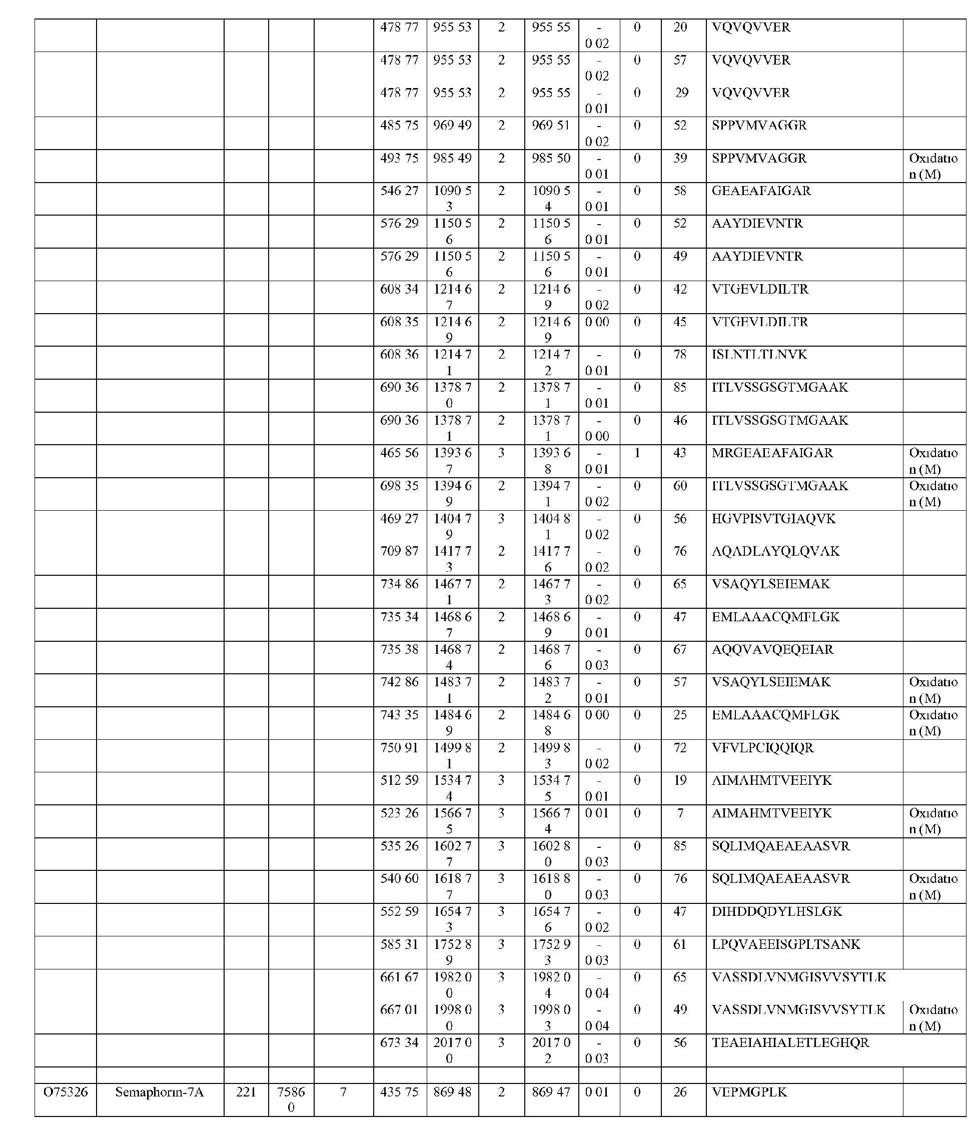

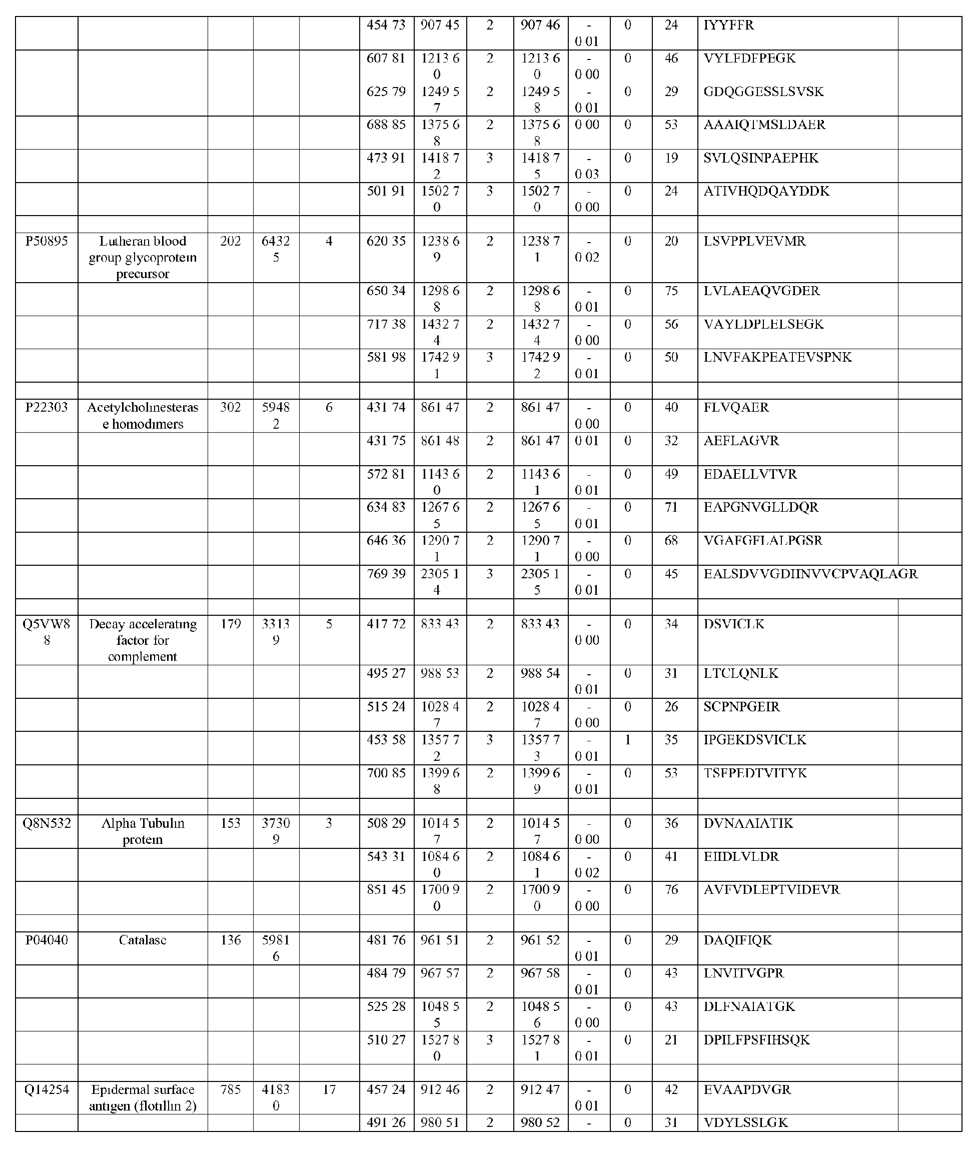

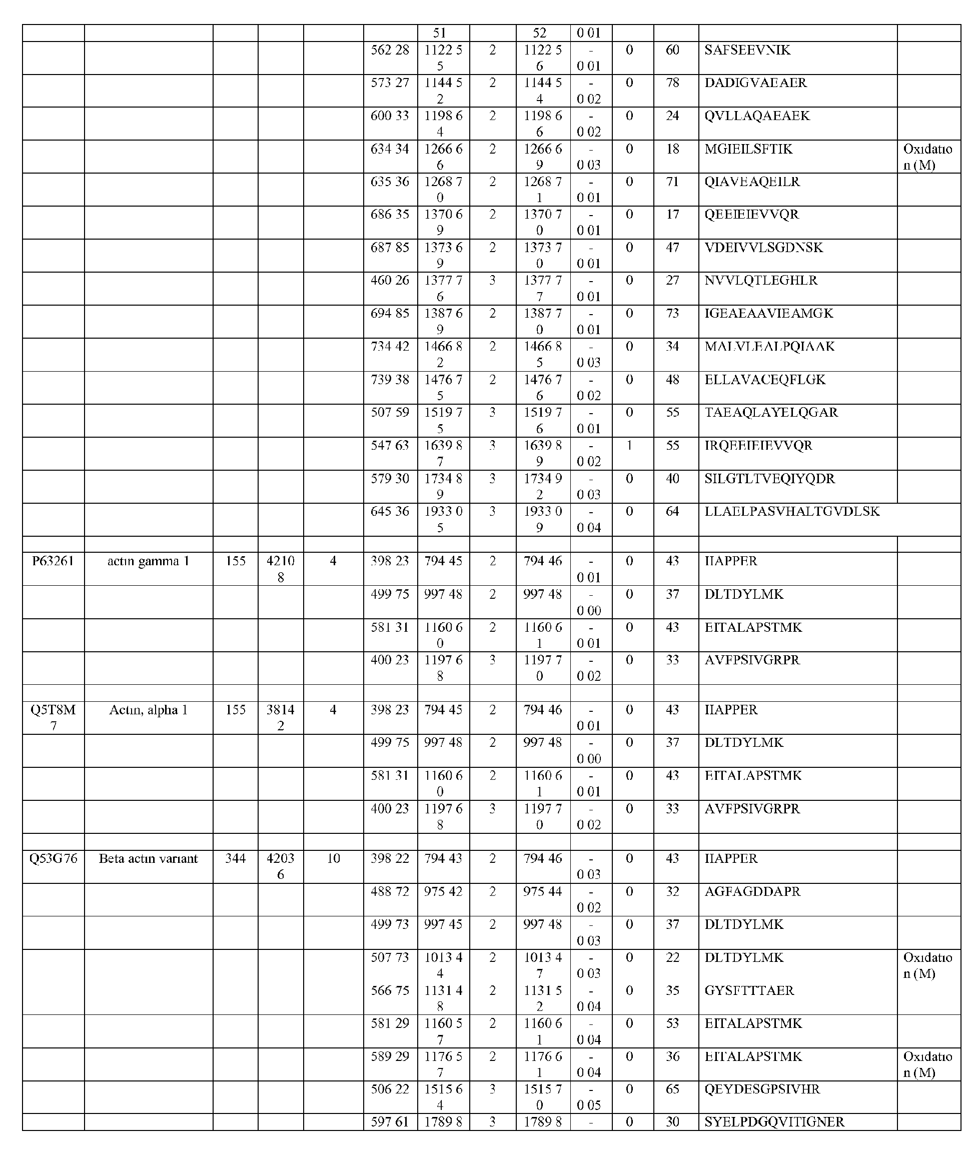

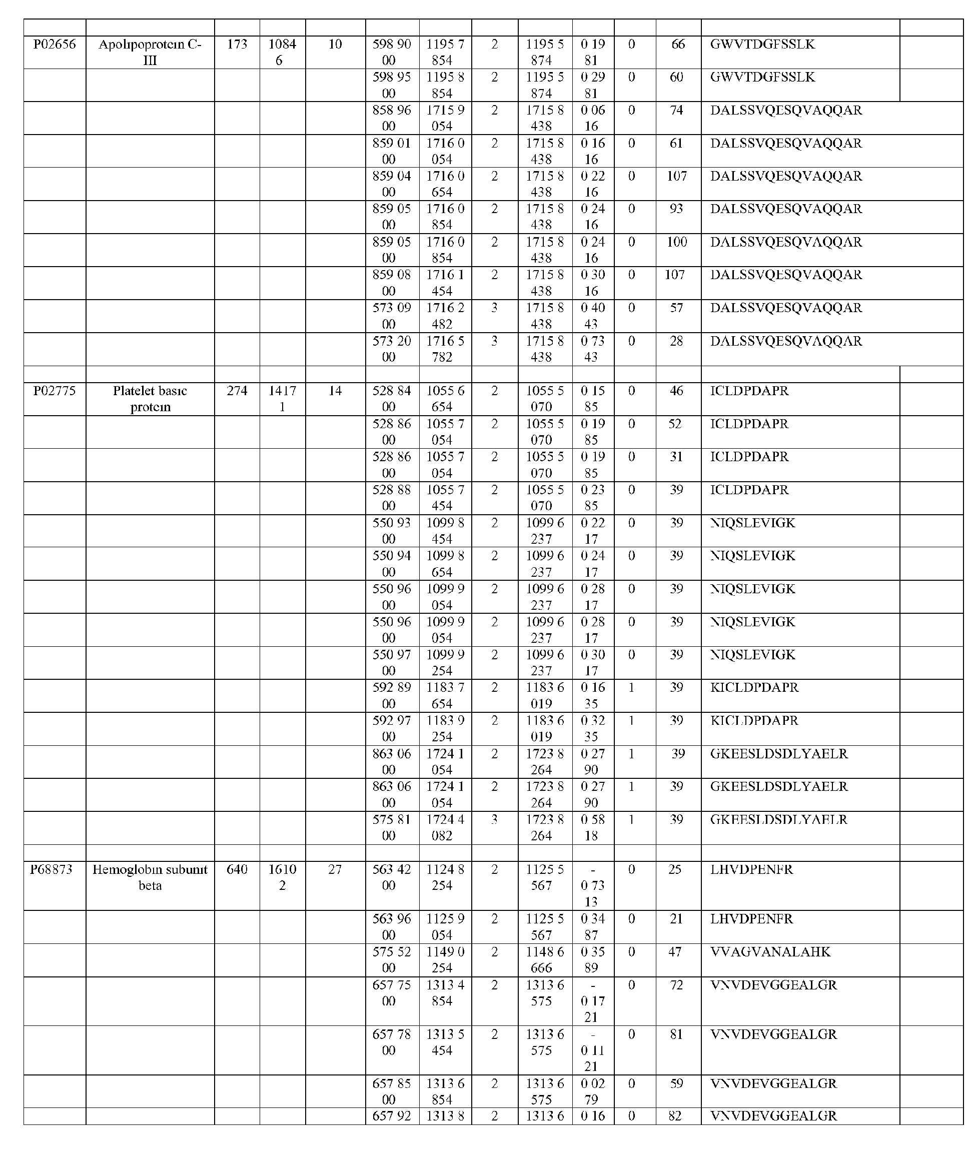

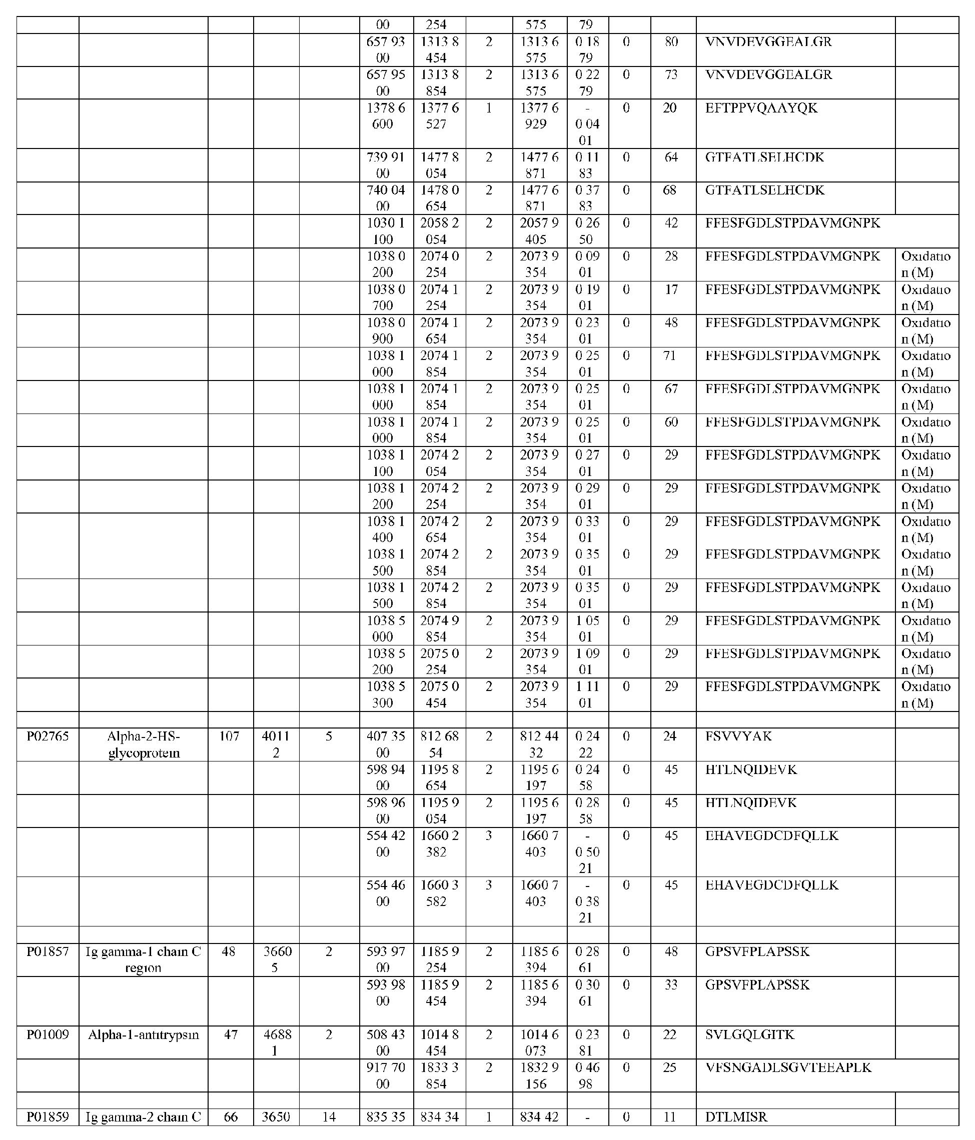

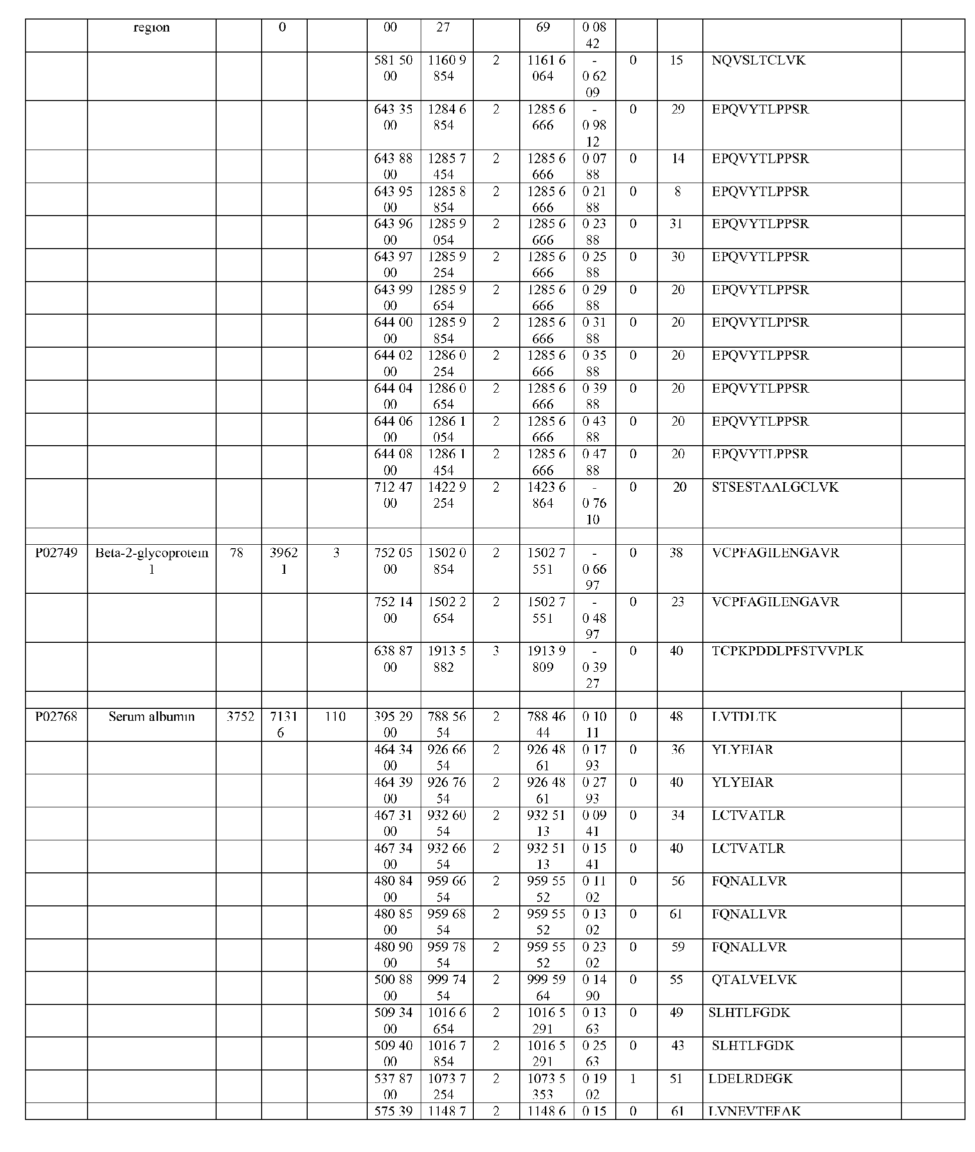

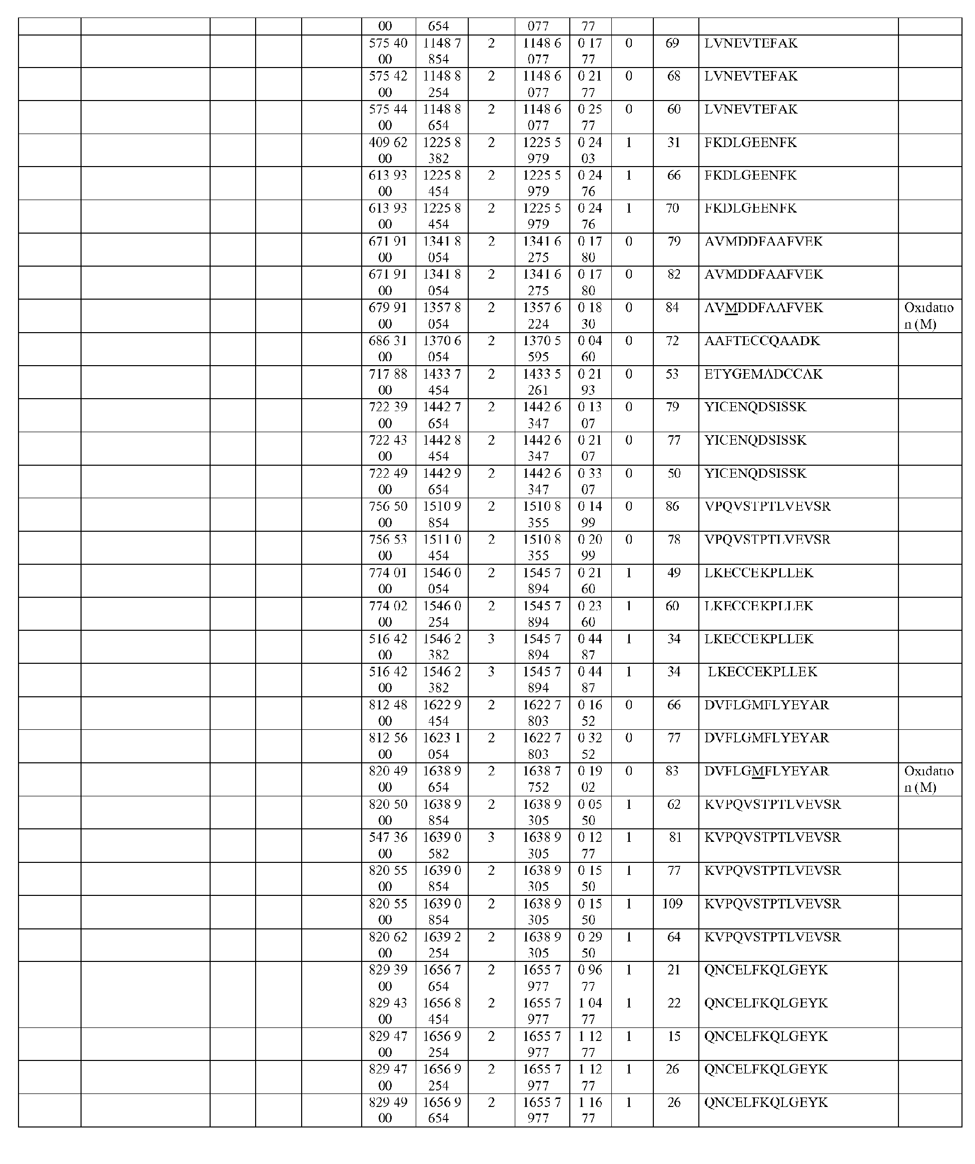

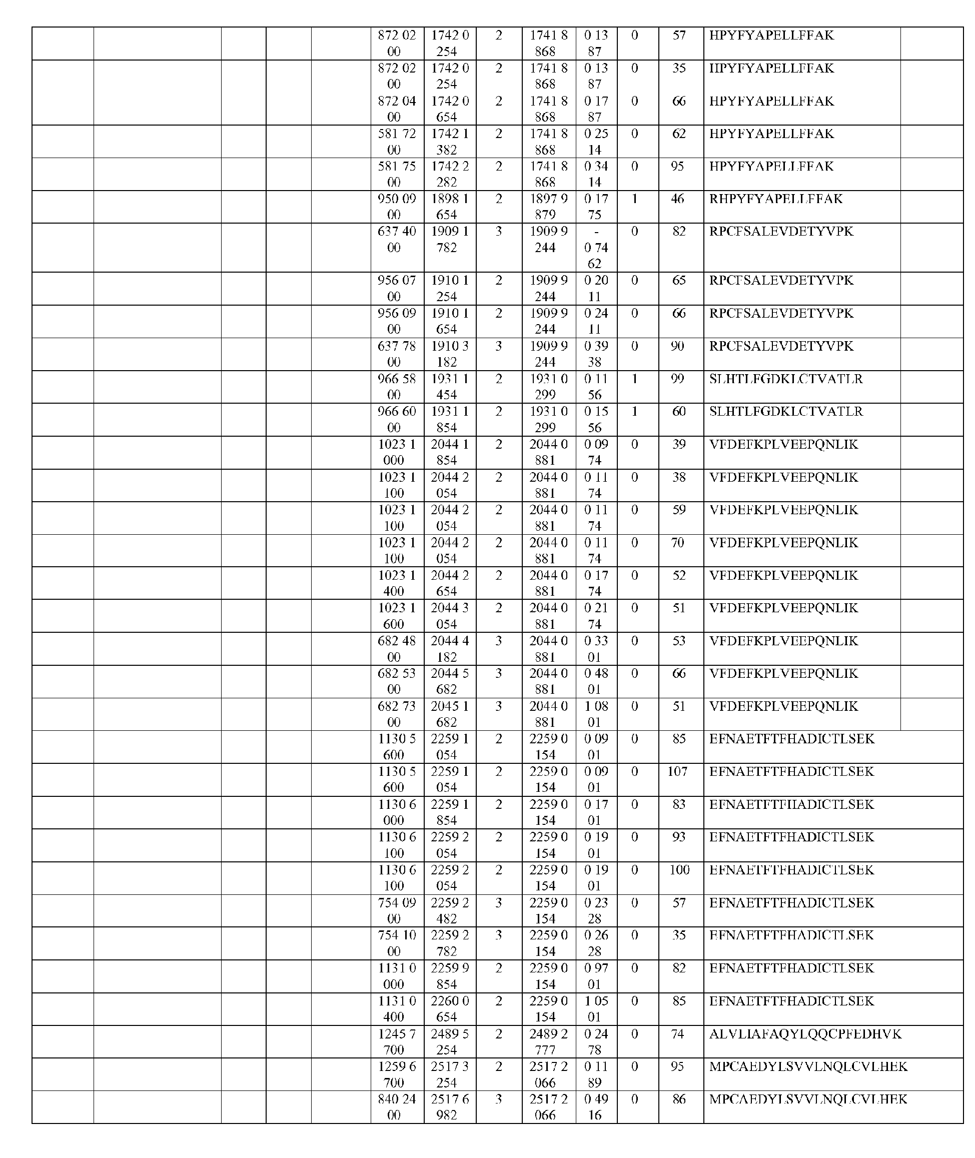

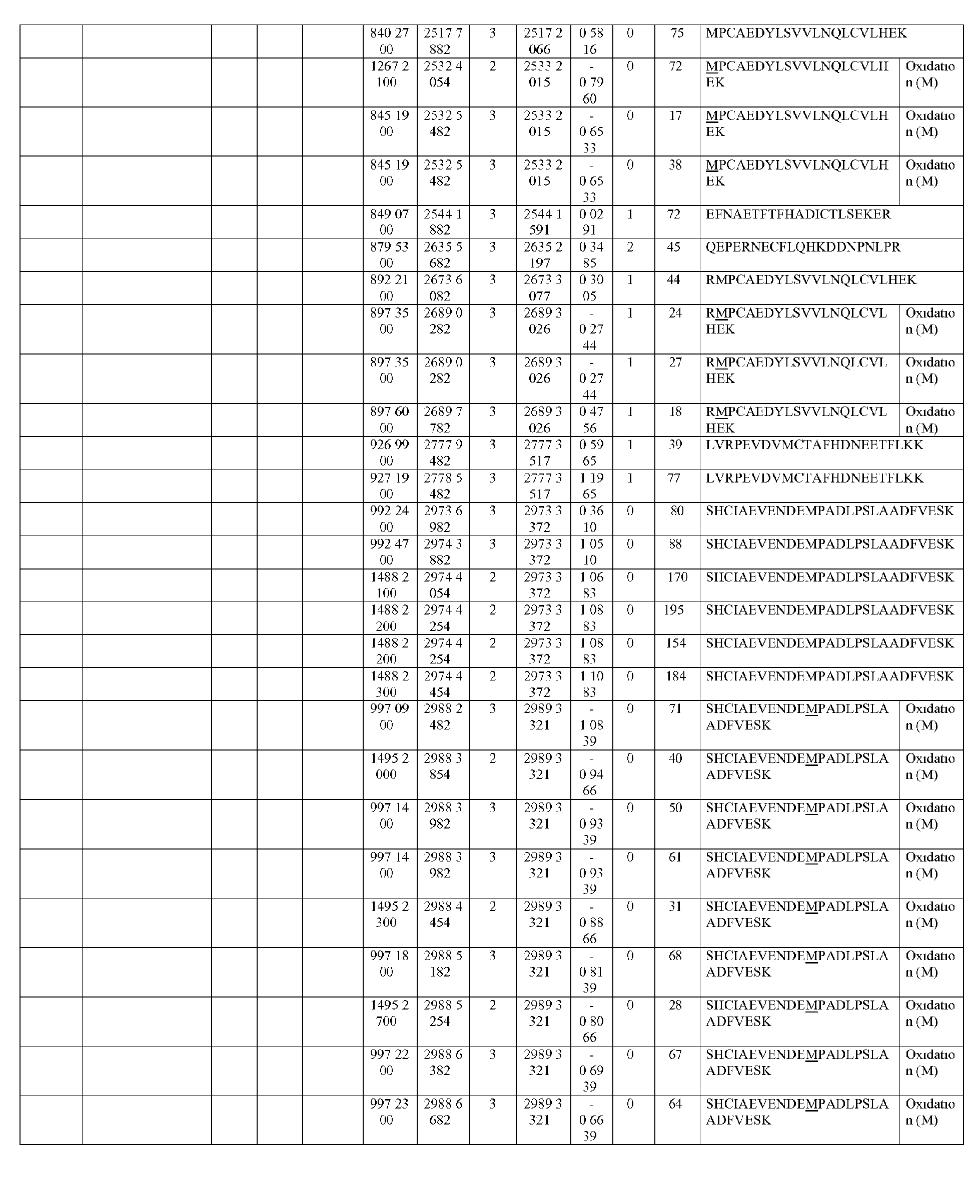

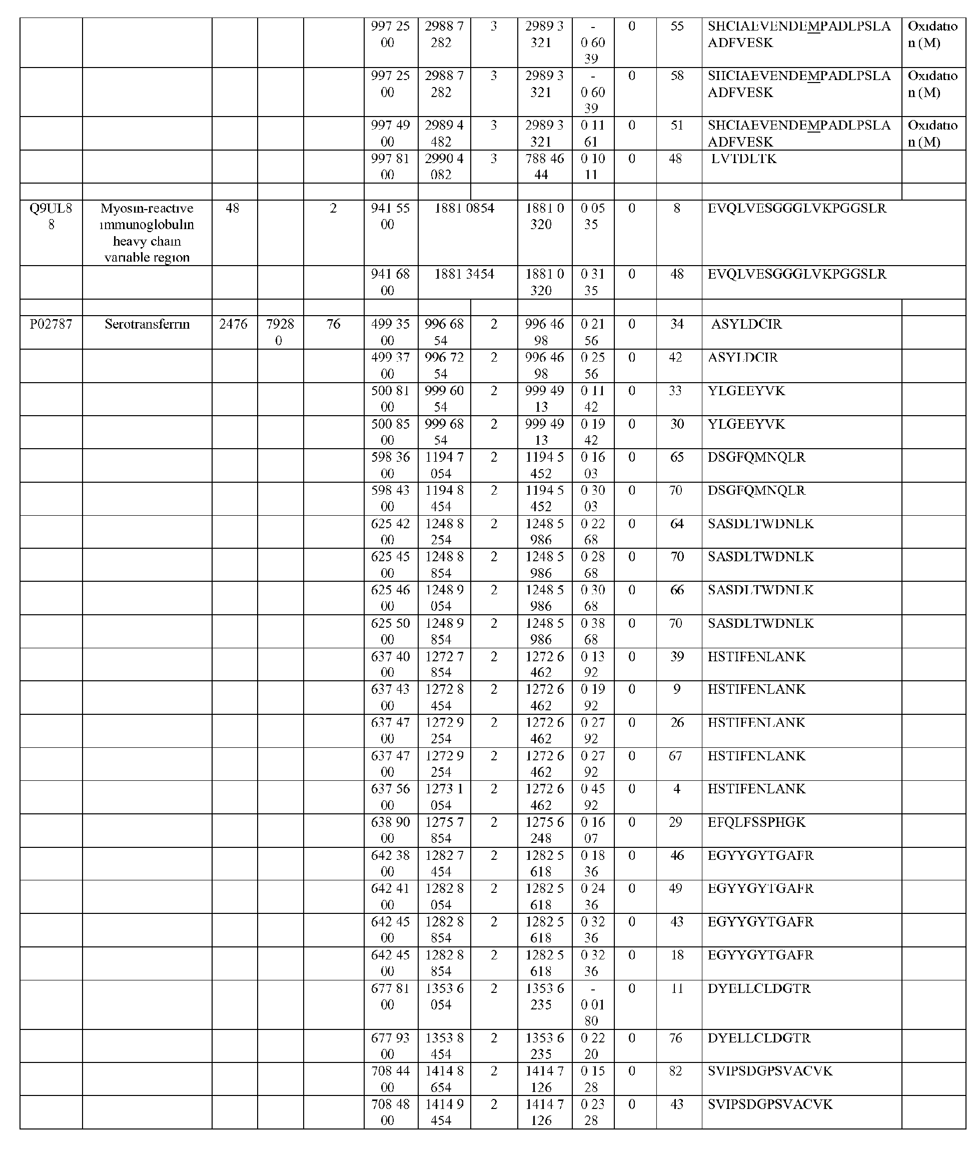

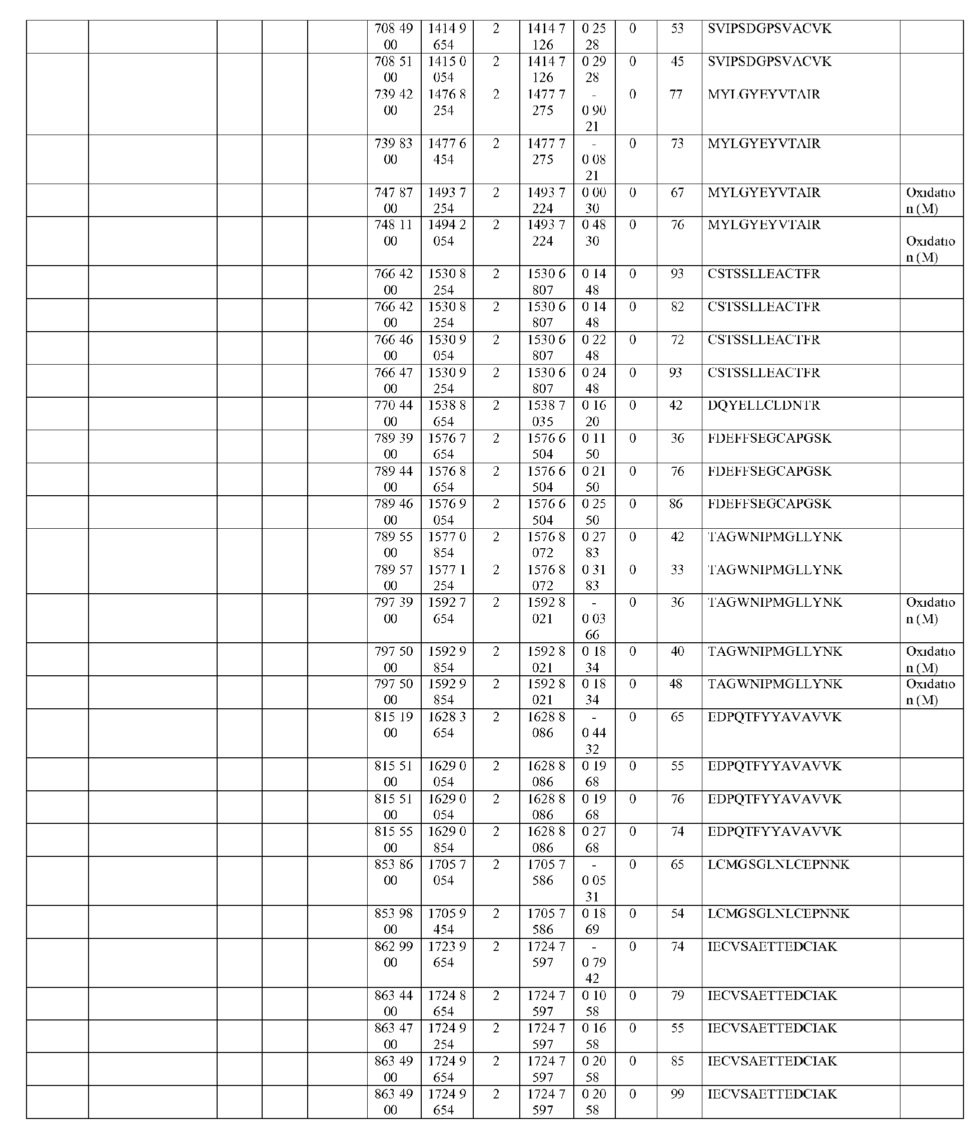

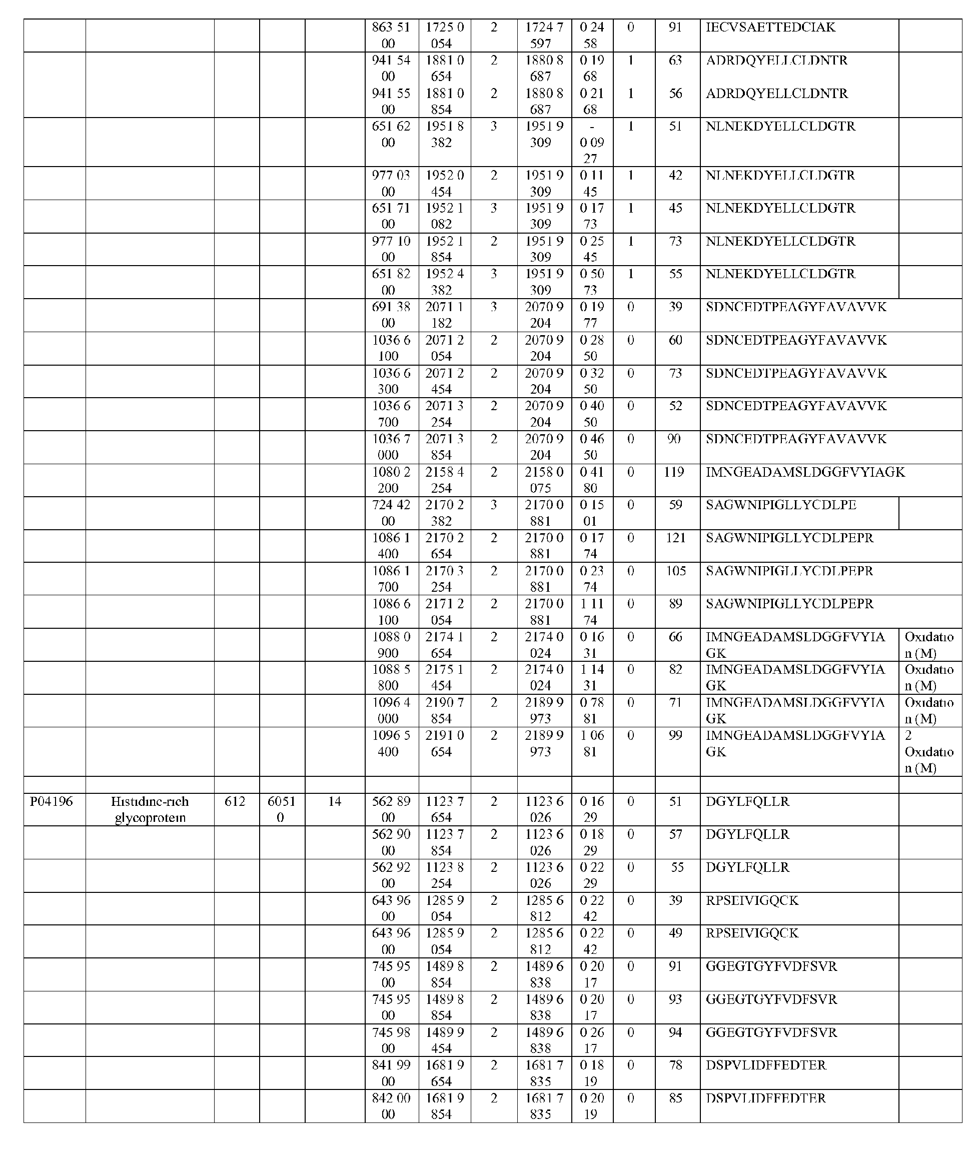

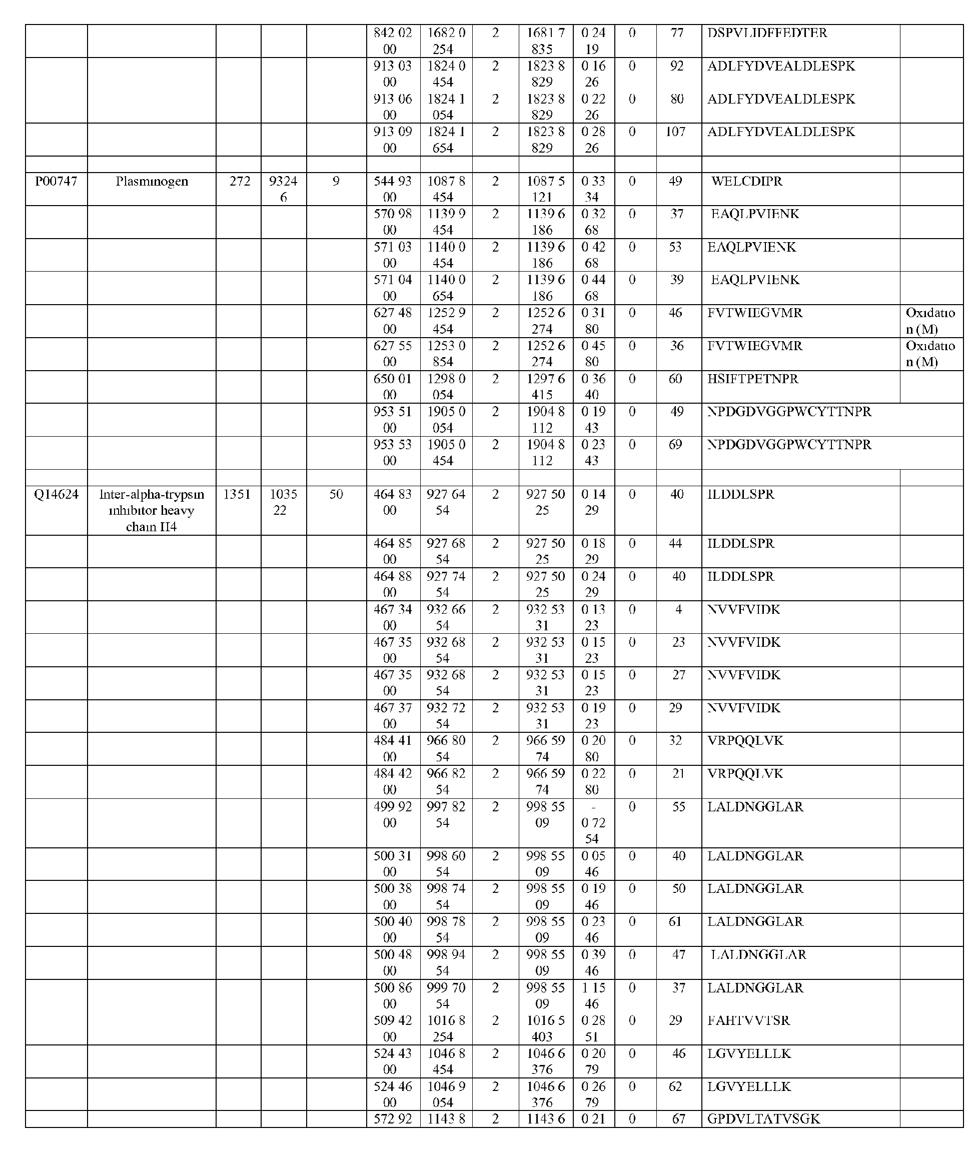

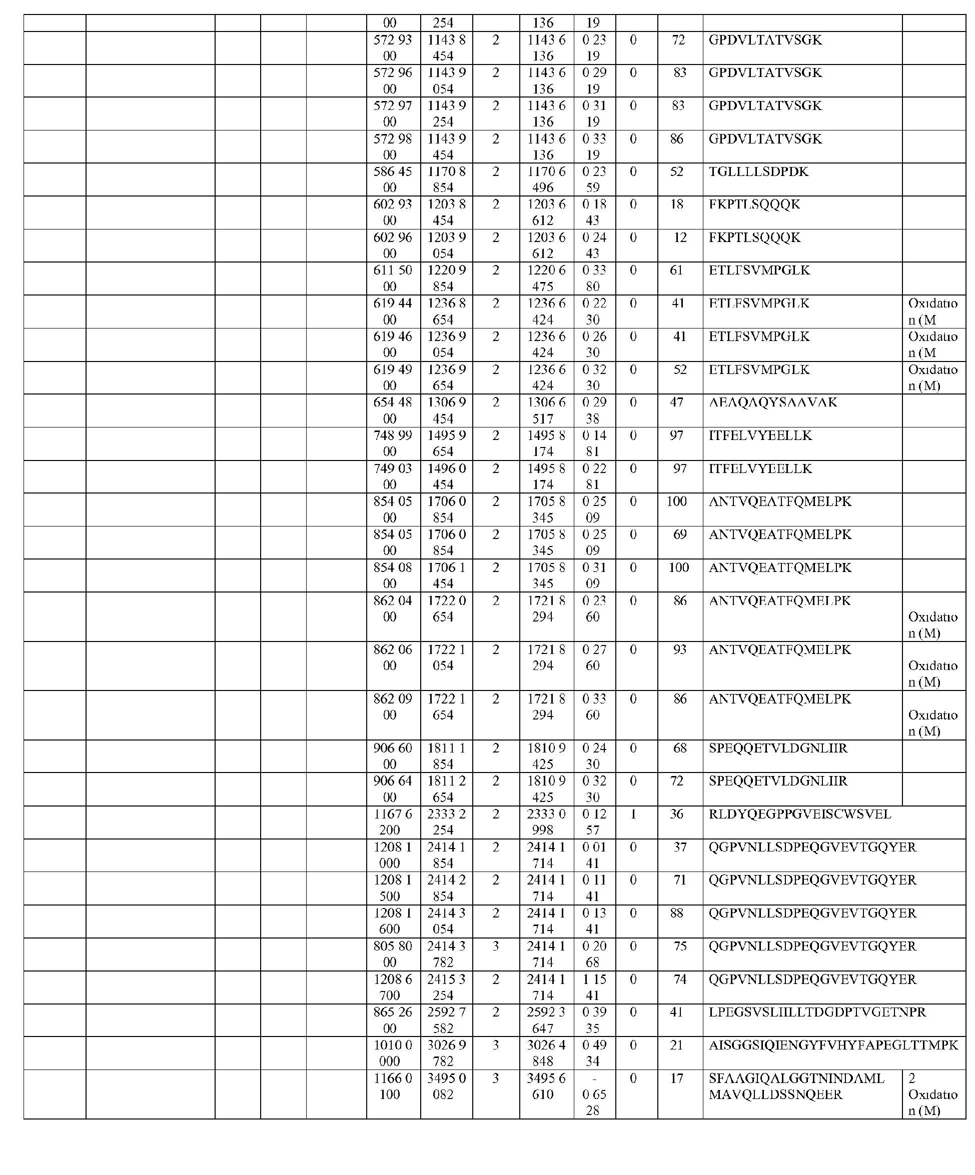

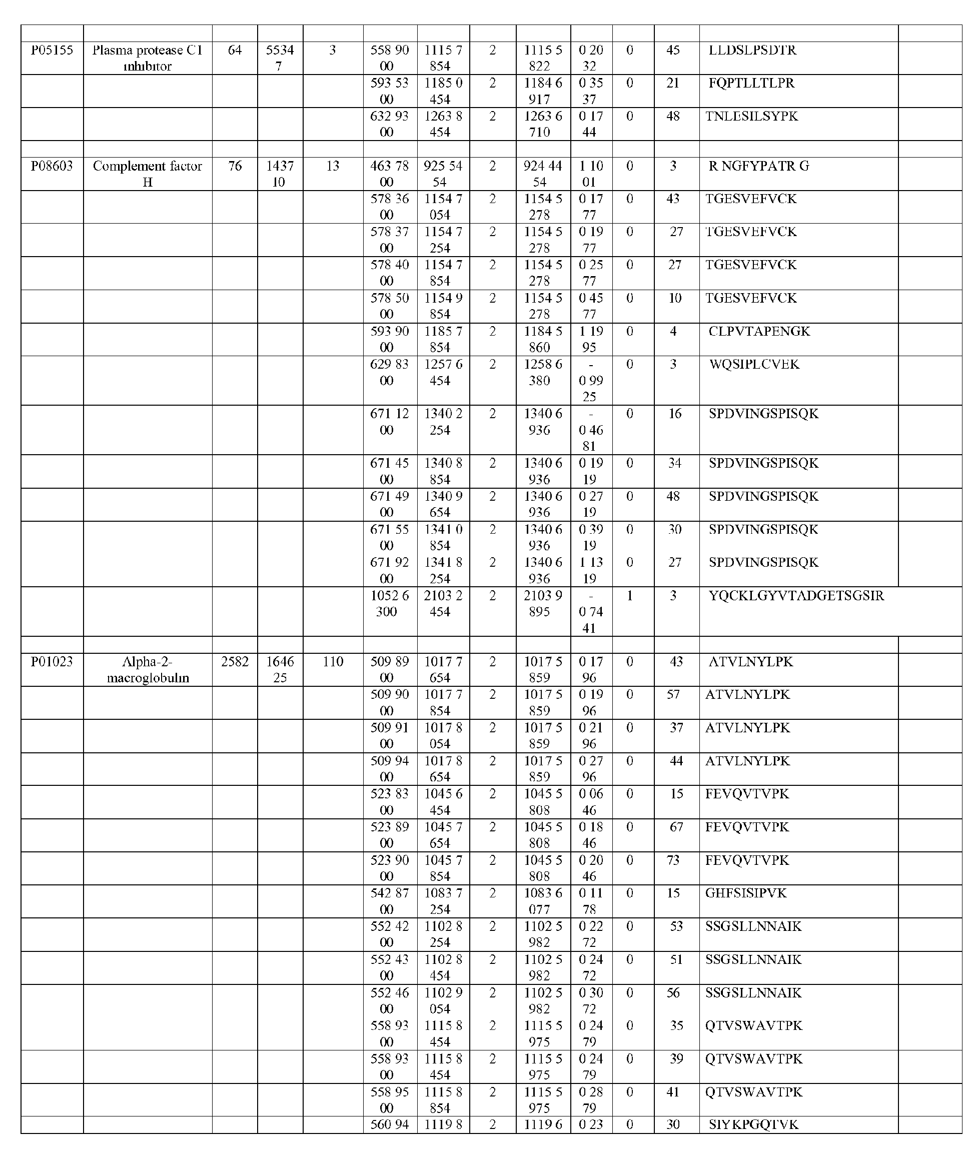

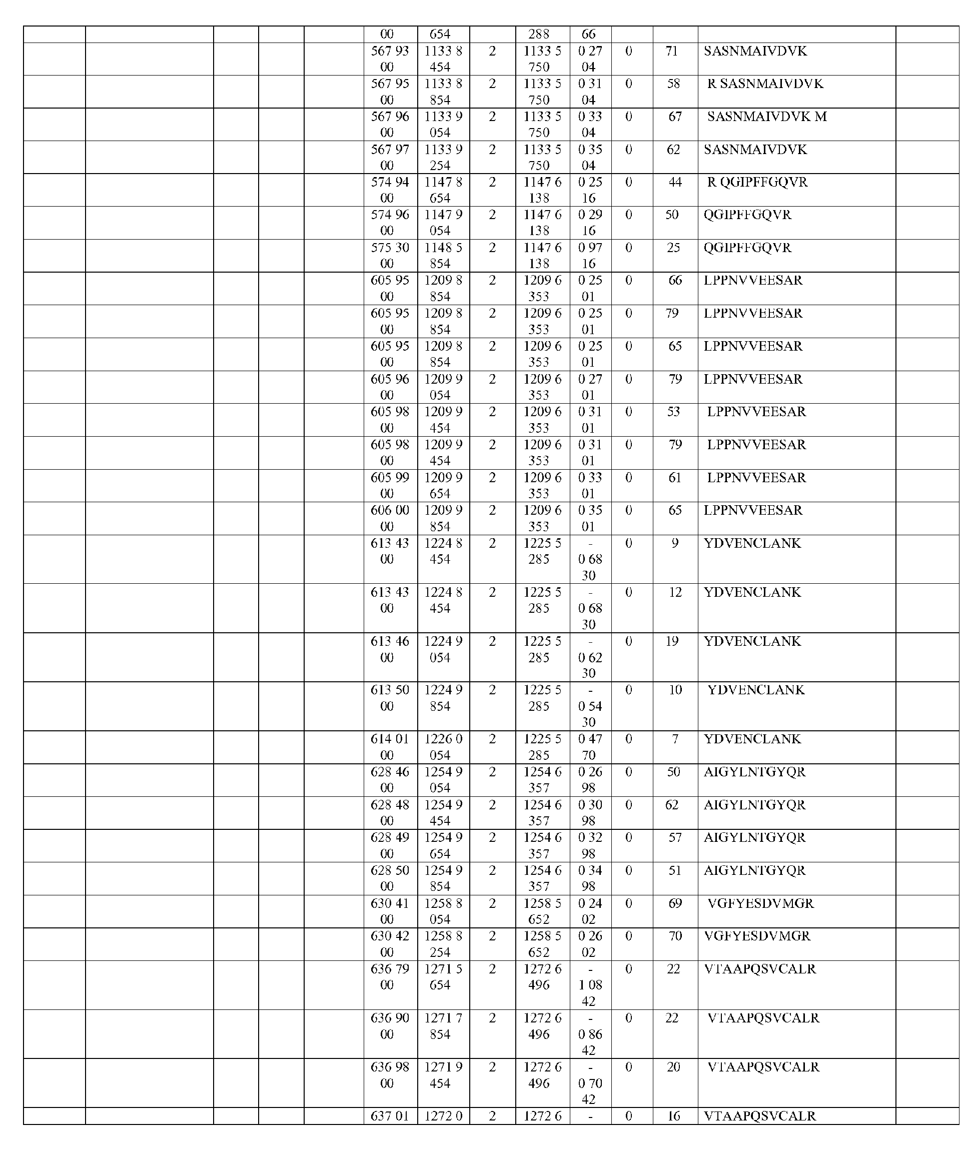

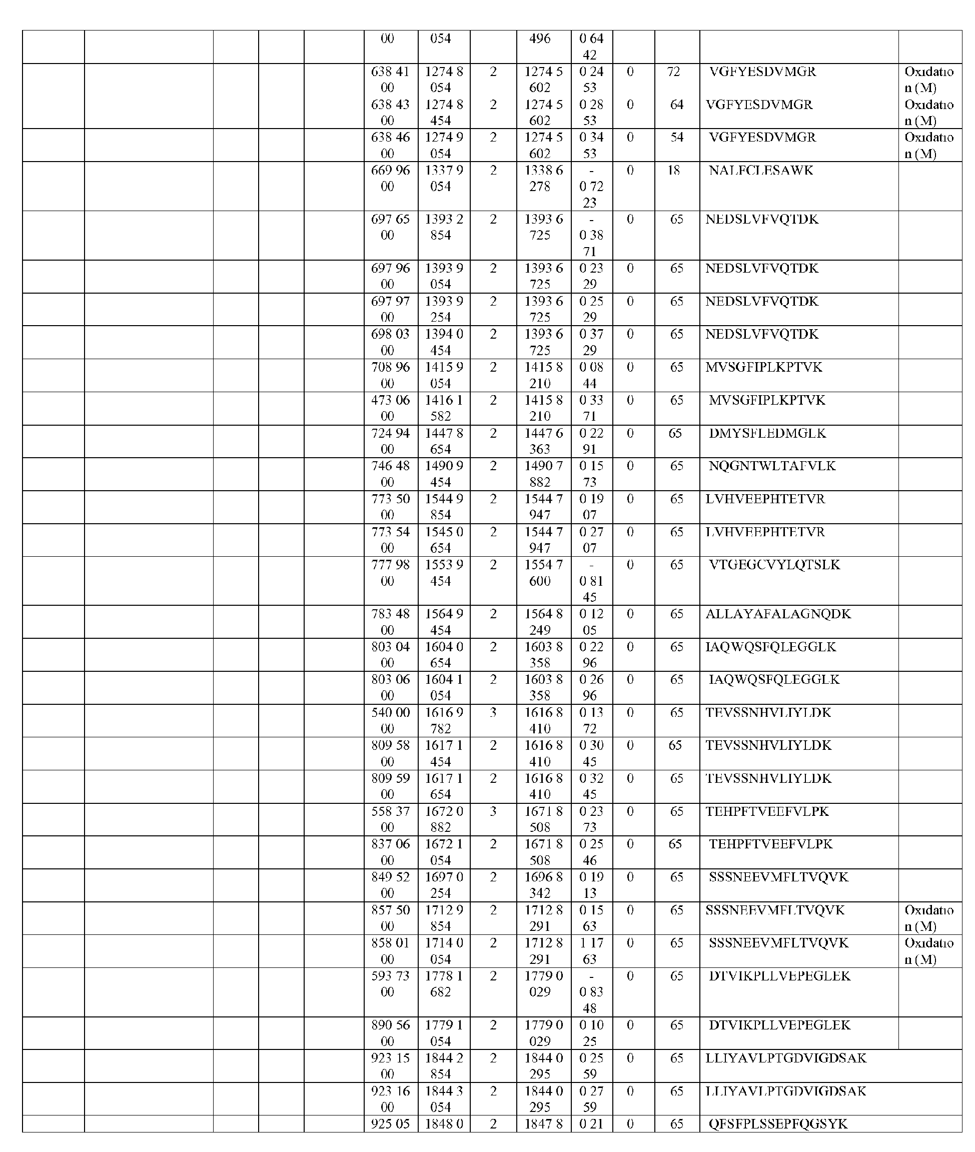

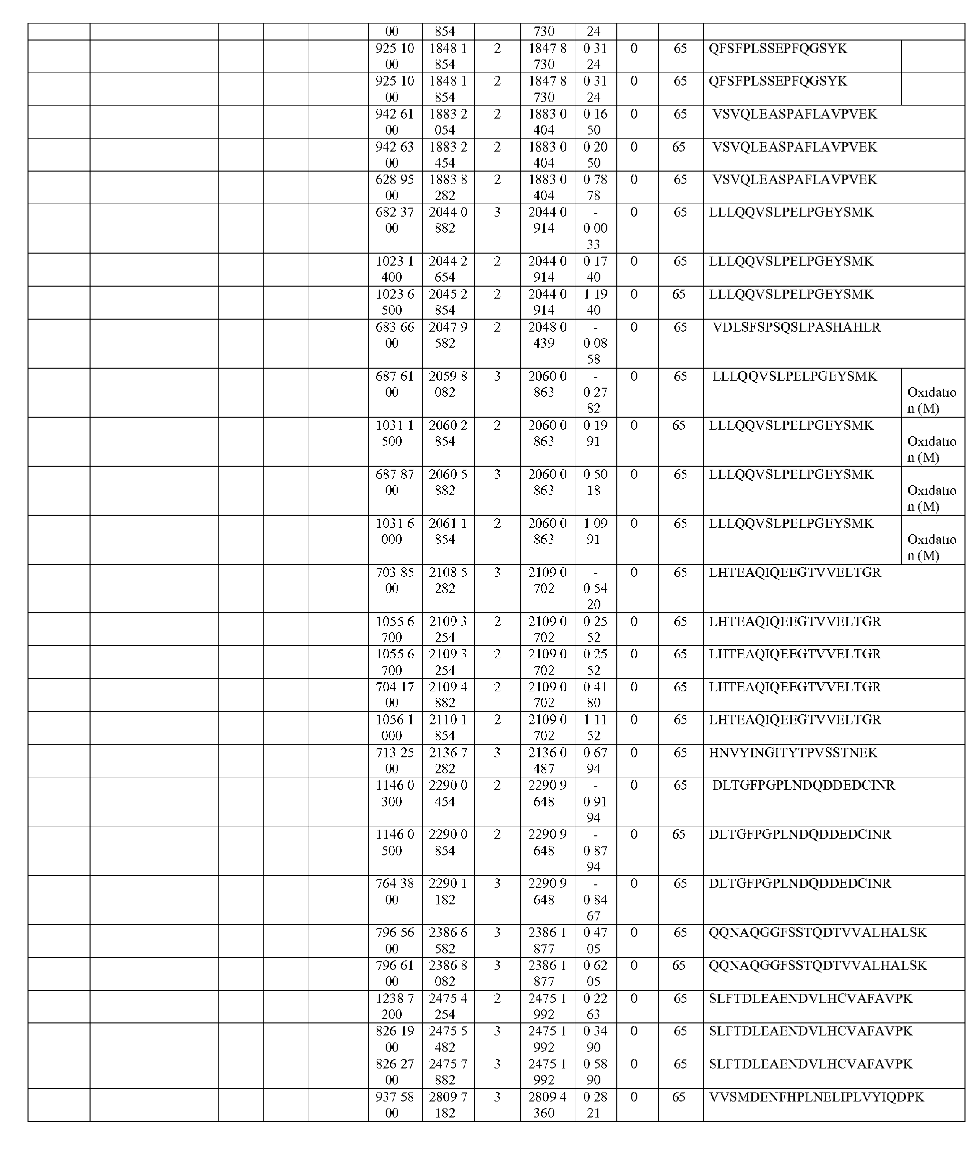

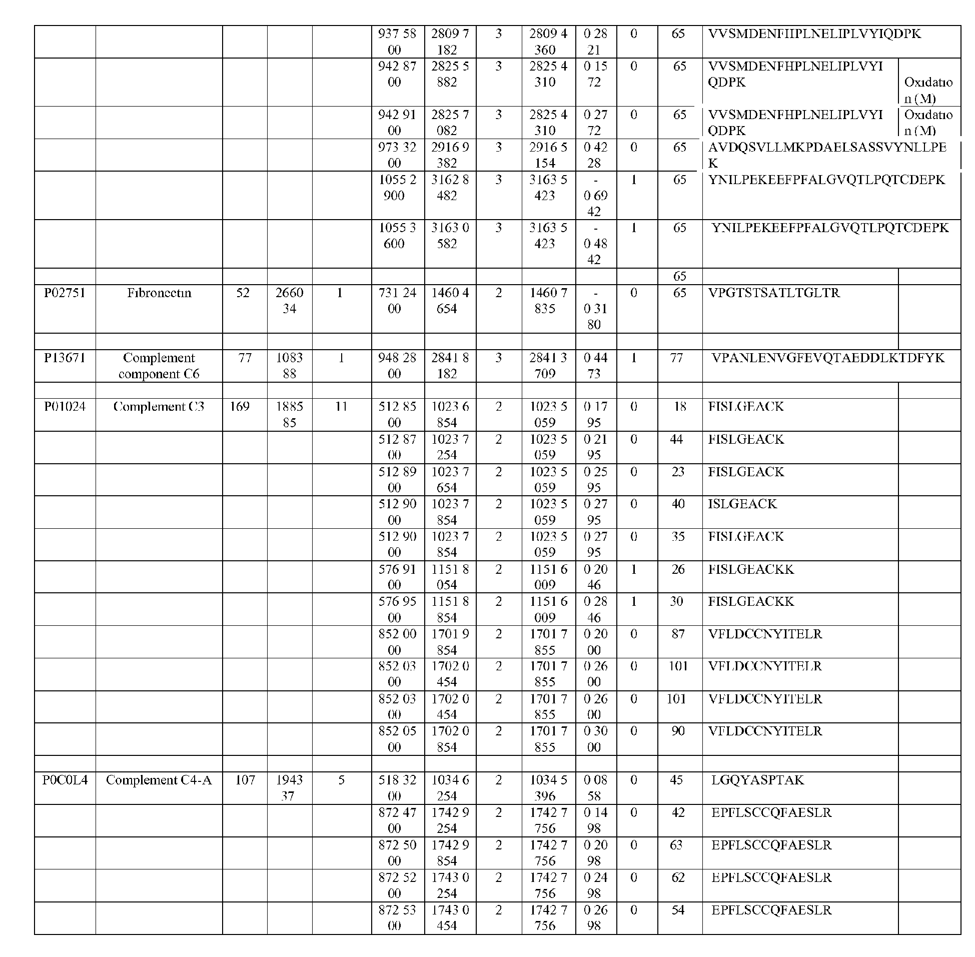

### LMW proteins profiling of CRC patients sera using NSPP, Profiling Kit MB-HIC- 8 and Dynal Peptide Profiler RPC18 approaches

To evaluate whether a more sensitive procedure could be of potential use in the biomarker discovery field, two sets of sera (normal donors *versus* colorectal cancer patients) have been analyzed. Sera from 15 CRC patients and 15 CTRL were analyzed using NSPP, Profiling Kit MB-HIC- 8 and Dynal Peptide Profiler RPC18 methods. A comparative analysis among the three analytical approaches was performed to evaluate whether these technologies could detect LMW protein profiling discriminating between CRC patients and CTRL.

Figure 4 shows the results of a comparison between MALDI LMW protein profiling of CRC patients and CRTL groups obtained by NSPP (MB-HIC8) performed using ClinProTools™ 2.0 statistical software. A statistically significant different expression of 12 peptide masses was observed between the two groups of sera studied. In contrast, only 6 peptide masses were detected when applying Profiling Kit MB-HIC- 8 (Fig. 4). Similar results were obtained when the NSPP (RPC 18) approach was compared to the Dynal Peptide Profiler RPC18. In fact, while 3 peptide masses were detected using the NSPP, only 1 discriminatory peptide was observed using the Dynal Peptide Profiler RPC18 purification (Fig. 5).

To test the peptidic nature of the discriminatory peaks, a nano ESI QToF MS/MS analysis was performed on the same samples. A confirmatory detection of MW= 2010 Da, 3157 Da and 4279 Da peaks was obtained (Fig. 6), demonstrating that the peaks detected by the present new approach were of peptidic nature.

### DISCUSSION

The NSPP approach was developed to optimize the recovery and reproducibility of serum LMW protein profiling by MALDI-TOF mass spectrometry. This method of sample preparation was far superior than using the serum Profiling Kit MB-HIC-8 and Dynal Peptide Profiler RPC18 alone. The NSPP uses acetonitrile and TFA to denature high MW carrier proteins and allows carrier protein removal. This improves the detection of the small, low-abundance proteins and peptides normally bound to these carrier proteins. [22]. NSPP improved the recovery of non-associated and associated (to high abundant carrier proteins) LMW proteins and also improved their detection using reverse phase magnetic beads while making them available for detection by mass spectrometry [23, 24]. In contrast, Profiling Kit MB-HIC8 and Dynal Peptide Profiler RPC18 approaches using raw serum samples allows to recovery only free LMW proteins (non-linked to high abundant carrier proteins).

NSPP, Profiling Kit MB-HIC8 and Dynal Peptide Profiler RPC18 sample preparations were analyzed by mass spectrometry MALDI-TOF using a sample crystallization technique on MALDI target, to obtain a clean spectra which were easily comparable to each other. However, a higher number of LMW proteins were recovered by NSPP when compared to the low number of LMW proteins recovered by Profiling Kit MB-HIC8 and Dynal Peptide Profiler RPC18 (Figure 1). Moreover, as shown in figure 3, NSPP allowed selective recovery of LMW proteins thus preventing beads sites from saturation by high abundant serum proteins.

The statistical analysis performed using ClinProTools software demonstrated a better reproducibility of serum LMW protein profiles using NSPP compared to protein profiles obtained with the Profiling Kit MB-HIC8 and Dynal Peptide Profiler RPC18 (p<0.01) (Fig. 1); furthermore, as reported in Fig. 2, a significant improved recovery was obtained when using NSPP in comparison to Profiling Kit MB-HIC8 and to Dynal Peptide Profiler RPC18.

To evaluate whether a more sensitive procedure could be of potential usefulness in the biomarker discovery field, two sets of sera (normal donors *versus* colorectal cancer patients) have been analyzed. The results demonstrated the recovery of a significant higher number of discriminatory peptides using NSPP compared to the kits alone (p<0.01) and suggested the possibility of detecting a discriminatory serum pattern profile only when using NSPP (Figures 4 and 5), thus strengthening the technical value of this new approach. The data obtained in this regards must be considered preliminary, and obviously a study on a greater number of cases should be specifically designed, nevertheless, taking into account the ability to improve recovery and reproducibility of LMW protein pattern profiles of this new approach, they are suggestive for its potential usefulness in the field of biomarker discovery. As suggested by other De Noo et al. [25]: "The discrepancies in discriminating protein profiles, found by different research groups, lead to serious concerns regarding biological variations and technological reproducibility issues.". Therefore, the availability of an automated technology platform and a new highly sensitive and reproducible serum processing procedure might be of great help in partially solving the technological issue.

In conclusion, the results obtained in the present study demonstrate that the NSPP pre-treatment of serum samples improves the performance of two commercial available kits today considered the most sensitive and reproducible (Profiling Kit MB-HIC8 and Dynal Peptide Profiler RPC18) and are suggestive for a potential evolution of the use of this new approach in the biomarkers discovery field.

### REFERENCES

[1] Etzioni R, Urban N, Ramsey S, McIntosh M, Schwartz S, Reid B, Radich J, Anderson G, Hartwell L. The case for early detection. Nat Rev Cancer. 2003: 4, 243-52.
[2] Stoughton RB, Friend SH. How molecular profiling could revolutionize drug discovery. Nat Rev Drug Discov. 2005:4, 345-50.
[3] Eleftherios P. Diamandis, How Are We Going to Discover New Cancer Biomarkers? A Proteomic Approach for Bladder Cancer, Clinical Chemistry 2004: 5, 50
[4] Schrohl AS, Würtz S, Kohn E, Banks RE, Nielsen HJ, Sweep FC, Brünner N. Banking of biological fluids for studies of disease-associated protein biomarkers. Mol Cell Proteomics.2008: 2061-6.
[5] Lathrop JT, Hayes TK, Carrick K Hammond JD. Rarity gives a charm: evaluation of trace proteins in plasma and serum Expert Review of Proteomics Jun2005: 2, 393-406 .
[6] Petricoin EE, Paweletz CP, Liotta LA. Clinical applications of proteomics: proteomic pattern diagnostics. J mammary gland biol neoplasia 2002: 7, 433-440
[7] Joshua N. Adkins, Susan M. Varnum, Kenneth J. Auberry, Ronald J. Moore, Nicolas H. Angell, Richard D. Smith, David L. Springer, and Joel G. Pounds Toward a Human Blood Serum Proteome analysis by multidimensional separation coupled with mass spectrometry Molecular & Cellular Proteomics 2002: 12, 947-955
[7a] Lion N, Tissot JD Application of proteomics to haematology: the revolution is starting Expert Rev Proteomics 2008: 5, 375-379
[8] Anderson L, Anderson NG. The Human Plasma Proteome: History, Character, and Diagnostic Prospects 2002: 845-867
[8b] Adkins, J.N., Varnum, S.M., Auberry, K.J., Moore, R.J. et al., Toward a Human Blood Serum Proteome. Mol. Cell. Proteomics 2002, 1, 947- 955.
[9] Villanueva J, Shaffer DR, Philip J, Chaparro CA, Bromage HE, Olshen AB, Fleisher M, Lilja H, Brogi E, Boyd J, Carbayo MS, Holland EC, Cardo CC, Scher HI, Tempst P. Differential exoprotease activities confer tumor-specific serum peptidome patterns The Journal of Clinical Investigation 2006 116
[9b] Adkins, J.N., Varnum, S.M., Auberry, K.J., Moore, R.J. et al., Pounds Toward a Human Blood Serum Proteome analysis by multidimensional separation coupled with mass spectrometry. Mol. Cell. Proteomics 2002, 12, 947-955.
[10] Albrethsen J. Reproducibility in protein profiling by MALDI-TOF mass spectrometry. Clin Chem. 2007:5, 852-8. Review.
[10b] Anderson, N.L., Polanski, Pieper, Gatlin, M., R. et al., The human plasma proteome: a non redundant list developed by combination of four separate sources. Mol. Cell. Proteomics 2004, 3, 311-326.
[11] Merrell K, Southwick K, Steven W. Graves,a M. Sean Esplin,c Nathan E. Lewis,a and Craig D. Thulina,b, Analysis of Low-Abundance, Low-Molecular-Weight Serum Proteins Using Mass Spectrometry Journal of biomolecular techniques 2004: 15
[11b]Chertov, O., Simpson, J.T., Biragyn, A., Conrads, T.P. et al., Enrichment of low-molecular-weight proteins from biofluids for biomarker discovery. Expert Rev. Proteomics 2005, 1, 139-145.
[12] Chertov O, Biragyn A, Kwak LW, Simpson JT, Boronina T, Hoang VM, Prieto DA, Conrads TP, Veenstra TD, Fisher RJ. Organic solvent extraction of proteins and peptides from serum as an effective sample preparation for detection and identification of biomarkers by mass spectrometry. Proteomics 2004: 4, 1195-203.
[12b] Aresta, A., Calvano, C.D., Palmisano, F., Zambonina, C.G. et al, Impact of sample preparation in peptide/protein profiling in human serum by MALDI-TOF mass spectrometry. J. Pharm. Biomed. Anal. 2008, 46, 157-164.
[13] Ketterlinus R, Hsieh SY, Teng SH, Lee H, and Pusch W Fishing for biomarkers: analyzing mass spectrometry data with the new ClinProTools™ software BioTechniques. 2005: 37-40
[13b] Xiao, Z., Prieto, DR., Conrads, TP., Veenstra, TD., Issaq JH., Proteomic patterns: their potential for disease diagnosis. Molecular and Cellular Endocrinology. 2005, 230, 95-106.
[14] Christensen, R., Madsen, J.S., Vach, W. et al., Reproducibility of serum protein profiling by systematic assessment using solid-phase extraction and matrix-assisted laser desorption/ionization mass spectrometry. Callesen, A.K., Rapid Commun. Mass Spectrom. 2008, 22, 291-300.
[15] Villanueva, J., Lawlor, K., Toledo-Crow, R., Tempst, P., Automated serum peptide profiling. Nat. Protoc. 2006, 1, 880-91.
[16] Villanueva, J., Philip, J., Entenberg, D., Chaparro, C.A. et al., Serum peptide profiling by magnetic particle-assisted, automated sample processing and MALDI-TOF mass spectrometry. Anal. Chem. 2004, 15, 1560-1570.
[16b] Lopez FM, Mikulskis A, Kuzdzal S, Bennett DA, Kelly J, Golenko E, DiCesare J, Denoyer E, Patton WF, Ediger R, Sapp L, Ziegert T, Lynch C, Kramer S, Whiteley GR, Wall MR, Mannion DP, della Cioppa G, Rakitan JS Wolf GM. High-Resolution Serum Proteomic Profiling of Alzheimer Disease Samples Reveals Disease-Specific, Carrier-Protein-Bound Mass Signatures. Clinical Chemistry 2005: 51, 1946-1954
[17] Baumann, S., Ceglarek, U., Fiedler, G.M., Lembcke, J. et al., Standardized approach to proteome profiling of human serum based on magnetic bead separation and matrix-assisted laser desorption/ionization time-of-flight mass spectrometry. Clin. Chest. 2005, 51, 973-80.
[18] Jimenez C.R., Filali, Z.E.L. , Knol J.C., Hoekman K. et al., Automated serum peptide profiling using novel magnetic C18 beads off-line coupled to MALDI-TOF-MS. Proteomics Clin. Appl. 2007, 1, 598-604.
[19] Di Girolamo, F., Alessandroni, J., Somma, P., Guadagni, F., Pre-analytical operating procedures for serum Low Molecular Weight protein profiling. J Proteomics 2010, 3, 667-677.
[20] Ketterlinus, R., Hsieh, S.Y., Teng, S.H., Lee, H., Pusch, W., Fishing for biomarkers: analyzing mass spectrometry data with the new ClinProTools software. Biotechniques 2005, 38, S37-40.
[21] Hsieh, S.Y., Chen, R.K., Pan, Y.H., Lee, H.L., Systematical evaluation of the effects of sample collection procedures on low-molecular-weight serum/plasma proteome profiling. Proteomics 2006, 6, 3189-98.
[22] Chertov, O., Biragyn, A., Kwak, L.W., Simpson, J.T. et al., Organic solvent extraction of proteins and peptides from serum as an effective sample preparation for detection and identification of biomarkers by mass spectrometry. Proteomics 2004, 4, 1195-203.
[23] Albrethsen, J., Reproducibility in protein profiling by MALDI-TOF mass spectrometry. Clin. Chest. 2007, 5, 852-8.
[24] Merrell, K., Southwick, K., Steven, W., Graves, A.M. et al., Analysis of Low-Abundance, Low-Molecular-Weight Serum Proteins Using Mass Spectrometry. J. Biomol. Tech. 2004, 15, 238-248.
[25] De Noo, M.E., Mertens, B.J., Ozalp, A., Bladergroen, M.R. et al., Detection of colorectal cancer using MALDI-ToF serum protein profiling. Eur. J. Cancer 2006, 8, 1068-1076.

## Claims

1. Method for the detection of low molecular weight (LMW) protein profile from a serum sample comprising the step of:
a) precipitating high abundant serum proteins and collecting supernatant;
b) purifying said collected supernatant with magnetic beads with reverse phase functional surfaces;
c) crystallizing on Matrix-Assisted Laser Desorption Ionization (MALDI) mass spectrometer plate the products of step b), using a MALDI crystallization technique, in order to obtain four crystallized homogeneous spots;
d) analyzing LMW protein profiles of all crystallized spots by means of Matrix-Assisted Laser Desorption Ionization/Time-of-Flight mass spectrometer (MALDI/ToF ).

2. Method for the detection of LMW protein profile from a serum sample according to claim 1 wherein the precipitation step of step a) is performed by means of acetonitrile/TFA precipitation.

3. Method for the detection of LMW protein profile from a serum sample according any of previous claims wherein the purification step of step b) is performed by means of magnetic beads with C18 or C8 reverse phase functional surfaces.

4. Kit to work the method for the detection of LMW protein profile according to any of previous claims comprising:
- Acetonitrile;
- Trifluoroacetic acid (TFA) ;
- Deionized water;
- α-cyano-4-hydroxycinnamic acid (CHCA) ;
- Magnetic beads with C18 or C8 reverse phase functional surfaces.
